# EUROPÄISCHE PATENTSCHRIFT

(11) **EP 1 392 680 B1**
(45) Veröffentlichungstag und Bekanntmachung des Hinweises auf die Patenterteilung: **22.07.2009**
(21) Anmeldenummer: 02735333.3
(22) Anmeldetag: 03.05.2002
(51) Int. Cl.: C07D 401/14, C07D 401/12, C07D 407/12, C07D 409/14, A61K 31/47, A61K 31/44, A61P 29/00

(54) **SELEKTIVE ANTHRANYLAMIDPYRIDINAMIDE ALS VEGFR-2 UND VEGFR-3 INHIBITOREN**
SELECTIVE ANTHRANILAMIDE PYRIDINE AMIDES AS INHIBITORS OF VEGFR-2 AND VEGFR-3
ANTHRANYLAMIDES PYRIDINE AMIDES SELECTIVES EN TANT QU'INHIBATEURS VEGFR-2 ET VEGFR-3

(30) Priorität: 08.05.2001 DE 10123574; 15.05.2001 DE 10125294; 21.12.2001 DE 10164590
(43) Veröffentlichungstag der Anmeldung: 03.03.2004
(73) Patentinhaber: Bayer Schering Pharma Aktiengesellschaft, 13353 Berlin (DE)
(72) Erfinder: ERNST, Alexander, 4052 Basel (CH); HUTH, Andreas, 12437 Berlin (DE); KRÜGER, Martin, 13465 Berlin (DE); THIERAUCH, Karl-Heinz, 14469 Berlin (DE); MENRAD, Andreas, 16515 Oranienburg (DE); HABEREY, Martin, 12169 Berlin (DE)
(86) Internationale Anmeldenummer: PCT/EP2002/004924
(87) Internationale Veröffentlichungsnummer: WO 2002/090352

(56) Entgegenhaltungen:
- EP-A- 0 686 625
- WO-A-00/27819
- WO-A-00/27820
- WO-A-01/55114
- WO-A-01/85715

## Beschreibung

Die Erfindung betrifft selektive Anthranylamidpyridinamide als VEGFR-2 und VEGFR-3 Inhibitoren, deren Herstellung und Verwendung als Arzneimittel zur Behandlung von Erkrankungen, die durch persistente Angiogenese ausgelöst werden.

Persistente Angiogenese kann die Ursache für verschiedene Erkrankungen wie Psoriasis, Arthritis, wie rheumatoide Arthritis, Hämangioma, Endometriose, Angiofribroma, Augenerkrankungen, wie diabetische Retinopathie, Neovaskulares Glaukom, Nierenerkrankungen, wie Glomerulonephritis, diabetische Nephropathie, maligne Nephrosklerose, thrombotische mikroangiopatische Syndrome, Transplantationsabstoßungen und Glomerulopathie, fibrotische Erkrankungen, wie Leberzirrhose, mesangialzellproliferative Erkrankungen und Artheriosklerose sein oder zu einer Verschlimmerung dieser Erkrankungen führen.
Die persistente Angiogenese wird durch den Faktor VEGF über seinen Rezeptor induziert. Damit VEGF diese Wirkung entfalten kann ist es nötig, daß VEGF am Rezeptor bindet und eine Tyrosinphosphorylierung hervorgerufen wird.

Eine direkte oder indirekte Inhibition des VEGF-Rezeptors (VEGF = vaskulärer endothelialer Wachstumsfaktor) kann zur Behandlung derartiger Erkrankungen und anderer VEGF-induzierter pathologischer Angiogenese und vaskularer permeabiler Bedingungen, wie Tumor-Vaskularisierung, verwendet werden. Beispielsweise ist bekannt, daß durch lösliche Rezeptoren und Antikörper gegen VEGF das Wachstum von Tumoren gehemmt werden kann.

Aus der WO 00/27819 sind Anthranylsäureamide bekannt, die als Arzneimittel zur Behandlung von Psoriasis, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen, Artheriosklerose, Verletzungen des Nervengewebes und zur Hemmung der Reocclusion von Gefäßen nach Ballonkatheterbehandlung, bei der Gefäßprothetik oder nach dem Einsetzen von mechanischen Vorrichtungen zum Offenhalten von Gefäßen, wie z. B. Stents, zum Einsatz kommen.
Aus der WO 0027820A sind N-Aryl(thio)anthranilsäureamide bekannt, als Inhibitoren des VEGF-Rezeptors, aber die anmeldungsgemäßen Verbindungen (I) in der vorliegenden Erfindung unterscheiden sich von den meisten in den Beispielen gemäß WO 0027820A hergestellten Verbindungen durch die Definition des Substituenten E. Eine starke Angiogenese ist Voraussetzung der Wucherung von extrauterinem Endometrium bei der Endometriose. Die Angiogenesehemmung läßt sich daher auch zur Therapie dieser Krankheitsform verwenden, die schmerzhafte Zustände verursacht und häufig zur Unfruchtbarkeit führt.
Die bekannten Verbindungen sind in den angegebenen Indikationen zwar allgemein wirksam, aber ihre Wirksamkeit geht in der Regel einher mit einer Toxizität und einer schlechteren Verträglichkeit des Medikaments.
Es besteht daher ein Wunsch nach einerseits wirksameren und andererseits toxikologisch unbedenklicheren Verbindungen, die darüberhinaus auch noch besser verträglich sein sollten.
Es wurde nun gefunden, daß Verbindungen der allgemeinen Formel I 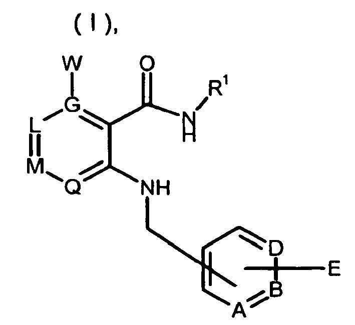 in der
- A, B und D: unabhängig voneinander für ein Stickstoff- oder Kohlenstoff-Atom stehen,
- E: für gegebenenfalls ein- oder mehrfach, gleich oder verschieden mit Halogen, Cyano, C₁-C₆-Alkyl, C₁-C₆-Alkoxy, Halo-C₁-C₆-Alkyl oder mit der Gruppe -OR⁵, -SR⁴, -SOR⁴ oder -SO₂R⁴ substituiertes Aryl oder Hetaryl steht, oder für die Gruppe -COOR⁸, -CONR²R³,-SR⁴,
-SOR⁴, -SO₂R⁴, -SCN, -PO(OR⁶)(OR⁷), -CH=CH-COR⁹ oder -C ≡ C-R⁹ steht,
- G: für die Gruppe -C-X steht,
- L: für die Gruppe -C-X steht,
- M: für die Gruppe -C-X steht,
- Q: für die Gruppe -C-X steht,
- W: für Wasserstoff oder Halogen steht,
- X: für Wasserstoff, Halogen oder für unsubstituiertes oder gegebenenfalls ein- oder mehrfach mit Halogen substituiertes C₁-C₆-Alkyl, C₁-C₆-Alkyloxy oder C₁-C₆-Carboxyalkyl steht,
- R¹: für gegebenenfalls ein- oder mehrfach, gleich oder verschieden mit Halogen, Hydroxy, C₁-C₆-Alkyloxy, Aralkyloxy, C₁-C₆-Alkyl und/oder mit der Gruppe -NR²R³ substituiertes verzweigtes oder unverzweigtes C₁-C₁₂-Alkyl oder C₂-C₁₂-Alkenyl steht; oder für gegebenenfalls ein- oder mehrfach, gleich oder verschieden mit Halogen, Hydroxy, C₁-C₆-Alkyloxy, C₁-C₆-Alkyl und/ oder mit der Gruppe -NR²R³ substituiertes C₃-C₁₀-Cycloalkyl oder C₃-C₁₀-Cycloalkenyl steht; oder für gegebenenfalls ein- oder mehrfach, gleich oder verschieden mit Halogen, Cyano, Hydroxy, C₁-C₆-Alkyloxy, C₂-C₆-Alkenyl, Aryl-C₁-C₆-alkyloxy, Aralkyloxy, C₁-C₆-Alkyl, Halo-C₁-C₆-alkyl oder mit der Gruppe =O, -SO₂R⁴, OR⁵, -R⁵ oder -PO(OR⁶)(OR⁷) substituiertes Aryl oder Hetaryl steht,
- R² und R³: unabhängig voneinander für Wasserstoff oder für gegebenenfalls ein- oder mehrfach, gleich oder verschieden mit Halogen, Cyano, C₁-C₆-Alkyl, Phenyl, Hydroxy-C₁-C₆-Alkyl, Halo-C₁-C₆-Alkyl oder mit der Gruppe -NR⁶R⁷, -OR⁵, C₁-C₆-Alkyl-OR⁵, -SR⁴, -SOR⁴ oder -SO₂R⁴ substituiertes C₁-C₆-Alkyl, C₃-C₆-Cycloalkyl, C₃-C₆-Cycloalkenyl, Aryl oder Hetaryl steht, oder
- R² und R³: gemeinsam mit dem Stickstoff-Atom einen C₃-C₈-Ring bilden, der gegebenenfalls ein weiteres Stickstoff-, Schwefel- oder Sauerstoffatom im Ring enthalten kann, oder die Gruppe -N(R¹⁰) enthalten kann, und der gegebenenfalls ein oder mehrfach, gleich oder verschieden mit Halogen, Cyano, C₁-C₆-Alkyl, Halo-C₁-C₆-Alkyl, Aryl oder mit der Gruppe -OR⁵, -SR⁴, -SOR⁴ oder -SO₂R⁴ substituiert sein kann,
- R⁴: für Hydroxy, C₁-C₆-Alkyl, Aryl, Hetaroaryl oder für die Gruppe -NR²R³ steht,
- R⁵: für Wasserstoff, C₁-C₁₂-Alkyl, Halo-C₁-C₆-Alkyl, C₃-C₆-Cycloalkyl oder Halo-C₃-C₆-Cycloalkyl steht, oder für C₁-C₁₂-Alkyl steht, welches ein- oder mehrfach mit Sauerstoff unterbrochen ist, oder für die Gruppe -(CH₂)₂NR²R³, -CH₂CN , oder -CH₂CF₃ steht,
- R⁶ und R⁷: unabhängig voneinander für Wasserstoff oder C₁-C₆-Alkyl stehen, oder
- R⁶ und R⁷: gemeinsam einen 5 bis 7 gliedrigen Ring bilden, der ein Sauerstoff- oder Schwefel-Atom oder die Gruppe -N(R¹⁰)-enthalten kann,
- R⁸: für Wasserstoff oder für gegebenenfalls mit Halogen ein- oder mehrfach substituiertes C₁-C₆-Alkyl, C₁-C₆-Alkoxy, Benzyl, Aryl oder Hetaryl steht,
- R⁹: für Wasserstoff, C₁-C₆-Alkyl, Tri-C₁₋₆-alkylsilyl, Aryl, Hetaryl oder für die Gruppe -NR²R³ oder -COR¹¹ steht,
- R¹⁰: für Wasserstoff, C₁-C₆-Alkyl oder Aryl steht,
- R¹¹: für Wasserstoff, C₁-C₆-Alkyl oder für die Gruppe -NR²R³ steht,
bedeuten, sowie deren Isomeren und Salze, die oben aufgeführten Nachteile überwinden.

Die erfindungsgemäßen Verbindungen verhindern eine Tyrosinphosphorylierung bzw. stoppen die persistente Angiogenese und damit das Wachstum und ein Ausbreiten von Tumoren, wobei sie sich insbesondere durch eine geringere Inhibition von Isoformen des Cytochroms P 450 (2C9 und 2C19) auszeichnen. Viele Arzneimittel werden über diese Isoformen abgebaut. Bei einer Inhibierung dieser Isoformen steigt der Plasmaspiegel dieser Arzneimittel an, was zu unerwünschten Nebenwirkungen führen kann.

Unter Alkyl ist jeweils ein geradkettiger oder verzweigter Alkylrest, wie beispielsweise Methyl, Ethyl, Propyl, Isopropyl, Butyl, Isobutyl, sek. Butyl, Pentyl, Isopentyl oder Hexyl, Heptyl, Octyl, Nonyl, Decyl, Undecyl, Dodecyl zu verstehen.

Unter Alkoxy ist jeweils ein geradkettiger oder verzweigter Alkoxyrest, wie beispielsweise Methyloxy, Ethyloxy, Propyloxy, Isopropyloxy, Butyloxy, Isobutyloxy, sek. Butyloxy, Pentyloxy, Isopentyloxy, Hexyloxy, Heptyloxy, Octyloxy, Nonyloxy, Decyloxy, Undecyloxy oder Dodecyloxy zu verstehen.

Unter Cycloalkyl sind monocyclische Alkylringe wie Cyclopropyl, Cyclobutyl, Cyclopentyl, Cyclohexyl oder Cycloheptyl, Cyclooctyl, Cyclononyl oder Cyclodecyl, aber auch bicyclische Ringe oder tricyclische Ringe wie zum Beispiel Adamantanyl zu verstehen.

Unter Cycloalkenyl ist jeweils Cyclobutenyl, Cyclopentenyl, Cyclohexenyl, Cycloheptenyl, Cyclooctenyl, Cyclononenyl oder Cyclodecenyl zu verstehen, wobei die Anknüpfung sowohl an der Doppelbindung wie auch an den Einfachbindungen erfolgen kann.

Unter Halogen ist jeweils Fluor, Chlor, Brom oder Jod zu verstehen.

Unter Alkenyl ist jeweils ein geradkettiger oder verzweigter Alkenyl-Rest zu verstehen, der 2 - 6, bevorzugt 2 - 4 C-Atome enthält. Beispielsweise seien die folgenden Reste genannt: Vinyl, Propen-1-yl, Propen-2-yl, But-1-en-1-yl, But-1-en-2-yl, But-2-en-1-yl, But-2-en-2-yl, 2-Methyl-prop-2-en-1-yl, 2-Methyl-prop-1-en-1-yl, But-1-en-3-yl, But-3-en-1-yl, Allyl.

Der Arylrest hat jeweils 6 - 12 Kohlenstoffatome wie beispielsweise Naphthyl, Biphenyl und insbesondere Phenyl.

Der Heteroarylrest umfaßt jeweils 3 - 16 Ringatome und kann anstelle des Kohlenstoffs ein- oder mehrere, gleiche oder verschiedene Heteroatome, wie Sauerstoff, Stickstoff oder Schwefel im Ring enthalten, und kann mono-, bi- oder tricyclisch sein, und kann zusätzlich jeweils benzokondensiert sein.

### Beispielsweise seien genannt:

Thienyl, Furanyl, Pyrrolyl, Oxazolyl, Thiazolyl, Imidazolyl, Pyrazolyl, Isoxazolyl, Isothiazolyl, Oxadiazolyl, Triazolyl, Thiadiazolyl, etc. und Benzoderivate davon, wie z. B. Benzofuranyl, Benzothienyl, Benzoxazolyl, Benzimidazolyl, Indazolyl, Indolyl, Isoindolyl, etc.; oder Pyridyl, Pyridazinyl, Pyrimidinyl, Pyrazinyl, Triazinyl, etc. und Benzoderivate davon, wie z. B. Chinolyl, Isochinolyl, etc.; oder
Oxepinyl,
Azocinyl, Indolizinyl, Indolyl, Isoindolyl, Indazolyl, Benzimidazolyl, Purinyl, etc. und Benzoderivate davon; oder Chinolinyl, Isochinolinyl, Cinnolinyl, Phthalazinyl, Chinazolinyl, Chinoxalinyl, Naphthyridinyl, Pteridinyl, Carbazolyl, Acridinyl, Phenazinyl, Phenothiazinyl, Phenoxazinyl, Xanthenyl, etc.

Der Aryl- und der Heteroarylrest kann jeweils 1-, 2- oder 3-fach gleich oder verschieden substitutiert sein mit Hydroxy, Halogen, C₁₋₄-Alkoxy, mit C₁₋₄-Alkyl, ein oder mehrfach mit Halogen substituiertes C₁₋₄-Alkyl.

Ist eine saure Funktion enthalten, sind als Salze die physiologisch verträglichen Salze organischer und anorganischer Basen geeignet wie beispielsweise die gut löslichen Alkali- und Erdalkalisalze sowie N-Methyl-glukamin, Dimethylglukamin, Ethyl-glukamin, Lysin, 1,6-hexandiamin, Ethanolamin, Glukosamin, Sarkosin, Serinol, Tris-hydroxy-methyl-amino-methan, Aminopropandiol, Sovak-Base, 1-Amino-2,3,4-butantriol.

Ist eine basische Funktion enthalten sind die physiologisch verträglichen Salze organischer und anorganischer Säuren geeignet wie Salzsäure, Schwefelsäure, Phosphorsäure, Zitronensäure, Weinsäure, Fumarsäue u.a.

Bei den erfindungsgemäßen Verbindungen der allgemeinen Formel I beinhaltet der Ausdruck "Isomere" die möglichen tautomeren Formen und umfasst die E- oder Z-Isomeren oder, falls ein chirales Zentrum vorhanden ist, auch die Racemate und Enantiomeren.

Als besonders wirksam haben sich solche Verbindungen der allgemeinen Formel I erwiesen, in der
- A, B und D: unabhängig voneinander für ein Stickstoff- oder Kohlenstoff-Atom stehen,
- E: für gegebenenfalls ein- oder mehrfach, gleich oder verschieden mit Halogen, Cyano, C₁-C₆-Alkyl, C₁-C₆-Alkoxy, Halo-C₁-C₆-Alkyl oder mit der Gruppe -OR⁵, -SR⁴, -SOR⁴ oder -SO₂R⁴ substituiertes Aryl oder Hetaryl steht, oder für die Gruppe -COOR⁸, -CONR²R³, -SR⁴, -SOR⁴, -SO₂R⁴, -SCN, -PO(OR⁶)(OR⁷), -CH=CH-COR⁹ oder -C ≡ C-R⁹ steht,
- G: für die Gruppe -C-X steht,
- L: für die Gruppe -C-X steht,
- M: für die Gruppe -C-X steht,
- Q: für die Gruppe -C-X steht,
- W: für Wasserstoff oder Halogen steht,
- X: für Wasserstoff, Halogen oder für unsubstituiertes oder gegebenenfalls ein- oder mehrfach mit Halogen substituiertes C₁-C₆-Alkyl, C₁-C₆-Alkyloxy oder C₁-C₆-Carboxyalkyl steht,
- R¹: für gegebenenfalls ein- oder mehrfach, gleich oder verschieden mit Halogen, Cyano, Hydroxy, C₁-C₆-Alkyloxy, C₂-C₆-Alkenyl, Aryl-C₁-C₆-alkyloxy, Aralkyloxy, C₁-C₆-Alkyl, Halo-C₁-C₆-alkyl oder mit der Gruppe =O, -SO₂R⁴, OR⁵, -R⁵ oder -PO(OR⁶)(OR⁷) substituiertes Aryl oder Hetaryl steht,
- R² und R³: unabhängig voneinander für Wasserstoff oder für gegebenenfalls ein- oder mehrfach, gleich oder verschieden mit Halogen, Cyano, C₁-C₆-Alkyl, Phenyl, Hydroxy-C₁-C₆-Alkyl, Halo-C₁-C₆-Alkyl oder mit der Gruppe -NR⁶R⁷, -OR⁵, C₁-C₆-Alkyl-OR⁵, -SR⁴, -SOR⁴ oder -SO₂R⁴ substituiertes C₁-C₆-Alkyl, C₃-C₆-Cycloalkyl, C₃-C₆-Cycloalkenyl, Aryl oder Hetaryl steht, oder
- R² und R³: gemeinsam mit dem Stickstoff-Atom einen C₃-C₈-Ring bilden, der gegebenenfalls ein weiteres Stickstoff-, Schwefel- oder Sauerstoffatom im Ring enthalten kann, oder die Gruppe -N(R¹⁰) enthalten kann, und der gegebenenfalls ein oder mehrfach, gleich oder verschieden mit Halogen, Cyano, C₁-C₆-Alkyl, Halo-C₁-C₆-Alkyl, Aryl oder mit der Gruppe -OR⁵, -SR⁴, -SOR⁴ oder -SO₂R⁴ substituiert sein kann,
- R⁴: für Hydroxy, C₁-C₆-Alkyl, Aryl, Hetaroaryl oder für die Gruppe -NR²R³ steht,
- R⁵: für Wasserstoff, C₁-C₁₂-Alkyl, Halo-C₁-C₆-Alkyl, C₃-C₆-Cycloalkyl oder Halo-C₃-C₆-Cycloalkyl steht, oder für C₁-C₁₂-Alkyl steht, welches ein- oder mehrfach mit Sauerstoff unterbrochen ist, oder für die Gruppe -(CH₂)₂NR²R³, -CH₂CN, oder -CH₂CF₃ steht,
- R⁶ und R⁷: unabhängig voneinander für Wasserstoff oder C₁-C₆-Alkyl stehen, oder
- R⁶ und R⁷: gemeinsam einen 5 bis 7 gliedrigen Ring bilden, der ein Sauerstoff- oder Schwefel-Atom oder die Gruppe -N(R¹⁰)-enthalten kann,
- R⁸: für Wasserstoff oder für gegebenenfalls mit Halogen ein- oder mehrfach substituiertes C₁-C₆-Alkyl, C₁-C₆-Alkoxy, Benzyl, Aryl oder Hetaryl steht,
- R⁹: für Wasserstoff, C₁-C₆-Alkyl, Tri-C₁-C₆-alkylsilyl, Aryl, Hetaryl oder für die Gruppe -NR²R³ oder -COR¹¹ steht,
- R¹⁰: für Wasserstoff, C₁-C₆-Alkyl oder Aryl steht,
- R¹¹: für Wasserstoff, C₁-C₆-Alkyl oder für die Gruppe -NR²R³ steht,
bedeuten, sowie deren Isomeren und Salze.

Insbesondere wirksam sind solche Verbindungen der allgemeinen Formel I, in der
- A, B und D: unabhängig voneinander für ein Stickstoff- oder Kohlenstoff-Atom stehen,
- E: für gegebenenfalls ein- oder mehrfach, gleich oder verschieden mit Halogen, Cyano, C₁-C₆Alkyl, C₁-C₆Alkoxy, Halo- C₁-C₆Alkyl oder mit der Gruppe -OR⁵, -SR⁴, -SOR⁴ oder -SO₂R⁴ substituiertes Aryl oder Hetaryl steht, oder für die Gruppe -COOR⁸, -CONR²R³, -SR⁴, -SOR⁴, -SO₂R⁴, -SCN, -PO(OR⁶)(OR⁷), -CH=CH-COR⁹ oder -C ≡ C-R⁹ steht,
- G: für die Gruppe -C-X steht,
- L: für die Gruppe -C-X steht,
- M: für die Gruppe -C-X steht,
- Q: für die Gruppe -C-X steht,
- W: für Wasserstoff oder Fluor steht,
- X: für Wasserstoff oder Halogen steht,
- R¹: für gegebenenfalls ein- oder mehrfach, gleich oder verschieden mit Halogen, Hydroxy, C₁-C₆-Alkyloxy, Aralkyloxy, C₁-C₆-Alkyl, Halo-C₁-C₆-alkyl oder mit der Gruppe -SO₂R⁴, OR⁵, -R⁵ oder -PO(OR⁶)(OR⁷) substituiertes Aryl oder Hetaryl steht,
- R² und R³: unabhängig voneinander für Wasserstoff oder für gegebenenfalls ein- oder mehrfach, gleich oder verschieden mit Halogen, Cyano, C₁-C₆-Alkyl, Phenyl, Hydroxy-C₁-C₆-Alkyl, Halo-C₁-C₆-Alkyl oder mit der Gruppe -NR⁶R⁷, -OR⁵, C₁-C₆-Alkyl-OR⁵, -SR⁴, -SOR⁴ oder -SO₂R⁴ substituiertes C₁-C₆-Alkyl, C₃-C₆-Cycloalkyl, C₃-C₆-Cycloalkenyl, Aryl oder Hetaryl steht, oder
- R² und R³: gemeinsam mit dem Stickstoff-Atom einen C₃-C₈-Ring bilden, der gegebenenfalls ein weiteres Stickstoff-, Schwefel- oder Sauerstoffatom im Ring enthalten kann, oder die Gruppe -N(R¹⁰) enthalten kann, und der gegebenenfalls ein oder mehrfach, gleich oder verschieden mit Halogen, Cyano, C₁-C₆-Alkyl, Halo-C₁-C₆-Alkyl, Aryl oder mit der Gruppe -OR⁵, -SR⁴, -SOR⁴ oder -SO₂R⁴ substituiert sein kann,
- R⁴: für Hydroxy oder für die Gruppe -NR²R³ steht,
- R⁵: für Wasserstoff, C₁-C₁₂-Alkyl oder für C₁-C₁₂-Alkyl steht, welches ein- oder mehrfach mit Sauerstoff unterbrochen ist, oder für die Gruppe -(CH₂)₂NR²R³, -CH₂CN, oder -CH₂CF₃ steht,
- R⁶ und R⁷: unabhängig voneinander für Wasserstoff oder C₁-C₆-Alkyl stehen, oder
- R⁶ und R⁷: gemeinsam einen 5 bis 7 gliedrigen Ring bilden, der ein Sauerstoff- oder Schwefel-Atom oder die Gruppe -N(R¹⁰)-enthalten kann,
- R⁸: für Wasserstoff oder für gegebenenfalls mit Halogen ein- oder mehrfach substituiertes C₁-C₆-Alkyl, C₁-C₆-Alkoxy, Benzyl, Aryl oder Hetaryl steht,
- R⁹: für Wasserstoff, C₁-C₆-Alkyl, Tri-C₁-C₆-alkylsilyl, Aryl, Hetaryl oder für die Gruppe -NR²R³ oder -COR¹¹ steht,
- R¹⁰: für Wasserstoff, C₁-C₆-Alkyl oder Aryl steht,
- R¹¹: für Wasserstoff, C₁-C₆-Alkyl oder für die Gruppe -NR²R³ steht,
bedeuten, sowie deren Isomeren und Salze.

Gute Eigenschaften haben solche Verbindungen der allgemeinen Formel I, in der
- A, B und D: für ein Stickstoff- oder Kohlenstoff-Atom stehen,
- E: für gegebenenfalls ein- oder mehrfach, gleich oder verschieden mit Halogen, Cyano, C₁₋₆Alkyl, C₁-C₆-Alkoxy, Halo-C₁-C₆-Alkyl oder mit der Gruppe -OR⁵, -SR⁴, -SOR⁴ oder -SO₂R⁴ substituiertes Hetaryl steht, oder für die Gruppe -COOR⁸, -CONR²R³,
-SR⁴, -SOR⁴, -SO₂R⁴, -SCN, -PO(OR⁶)(OR⁷), -CH=CH-COR⁹ oder -C ≡ C-R⁹ steht,
- G: für die Gruppe -C-X steht,
- L: für die Gruppe -C-X steht,
- M: für die Gruppe -C-X steht,
- Q: für die Gruppe -C-X steht,
- W: für Wasserstoff oder Fluor steht,
- X: für Wasserstoff oder Halogen steht,
- R¹: für gegebenenfalls ein- oder mehrfach, gleich oder verschieden mit Halogen, Hydroxy, C₁-C₆-Alkyloxy, Aralkyloxy, C₁-C₆-Alkyl, Halo-C₁-C₆-alkyl oder mit der Gruppe -SO₂R⁴, OR⁵, -R⁵ oder-PO(OR⁶)(OR⁷) substituiertes Phenyl, Thiophen, Furan, Oxazol, Thiazol, Imidazol, Pyrazol, Pyridin, Pyrimidin, Triazin, Chinolin, Isochinolin oder substituiert an der Gruppe 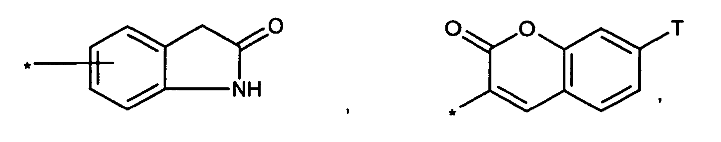 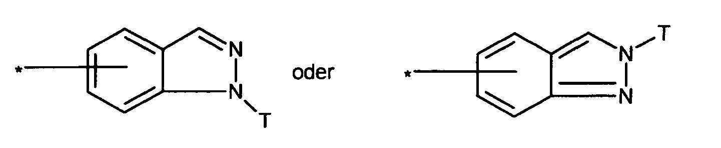 steht, in der T für Wasserstoff, C₁-C₆-Alkyl oder C₁-C₆-Alkoxy steht,
- R² und R³: unabhängig voneinander für Wasserstoff oder für gegebenenfalls ein- oder mehrfach, gleich oder verschieden mit Halogen, Cyano, C₁-C₆-Alkyl, Phenyl, Hydroxy-C₁-C₆-Alkyl, Halo-C₁-C₆-Alkyl oder mit der Gruppe -NR⁶R⁷, -OR⁵, C₁-C₆-Alkyl-OR⁵, -SR⁴, -SOR⁴ oder -SO₂R⁴ substituiertes C₁-C₆-Alkyl, C₃-C₆-Cycloalkyl, C₃-C₆-Cycloalkenyl, Aryl oder Hetaryl steht, oder
- R² und R³: gemeinsam mit dem Stickstoff-Atom einen C₃-C₈-Ring bilden, der gegebenenfalls ein weiteres Stickstoff-, Schwefel- oder Sauerstoffatom im Ring enthalten kann, oder die Gruppe -N(R¹⁰) enthalten kann, und der gegebenenfalls ein oder mehrfach, gleich oder verschieden mit Halogen, Cyano, C₁-C₆-Alkyl, Halo-C₁-C₆-Alkyl, Aryl oder mit der Gruppe -OR⁵, -SR⁴, -SOR⁴ oder -SO₂R⁴ substituiert sein kann,
- R⁴: für Hydroxy oder für die Gruppe -NR²R³ steht,
- R⁵: für Wasserstoff, C₁-C₁₂-Alkyl oder für C₁-C₁₂-Alkyl steht, welches ein- oder mehrfach mit Sauerstoff unterbrochen ist, oder für die Gruppe -(CH₂)₂NR²R³, -CH₂CN, oder -CH₂CF₃ steht,
- R⁶ und R⁷: unabhängig voneinander für Wasserstoff oder C₁-C₆-Alkyl stehen,
oder
- R⁶ und R⁷: gemeinsam einen 5 bis 7 gliedrigen Ring bilden, der ein Sauerstoff- oder Schwefel-Atom enthalten kann,
- R⁸: für Wasserstoff oder für gegebenenfalls mit Halogen ein- oder mehrfach substituiertes C₁-C₆-Alkyl, C₁-C₆-Alkoxy, Benzyl, Aryl oder Hetaryl steht, und
- R⁹: für Wasserstoff, C₁-C₆-Alkyl oder Tri-C₁-C₆-alkylsilyl steht,
bedeuten, sowie deren Isomeren und Salze.

Hervorragende Eigenschaften haben solche Verbindungen der allgemeinen Formel I, in der
- A, B und D: unabhängig voneinander für ein Stickstoff- oder Kohlenstoff-Atom stehen,
- E: für Thienyl, Pyridyl oder für die Gruppe -COOR⁸, -CONR²R³ oder -C ≡ C-R⁹ steht,
- G: für die Gruppe -C-X steht,
- L: für die Gruppe -C-X steht,
- M: für die Gruppe -C-X steht,
- Q: für die Gruppe -C-X steht,
- W: für Wasserstoff oder Fluor steht,
- X: für Wasserstoff oder Halogen steht,
- R¹: für gegebenenfalls ein- oder mehrfach, gleich oder verschieden mit Halogen, Hydroxy, C₁-C₆-Alkyloxy, Aralkyloxy, C₁-C₆-Alkyl, Halo-C₁-C₆-alkyl oder mit der Gruppe -SO₂R⁴, OR⁵, -R⁵ oder-PO(OR⁶)(OR⁷) substituiertes Phenyl, Thiophen, Furan, Oxazol, Thiazol, Imidazol, Pyrazol, Pyridin, Pyrimidin, Triazin, Chinolin oder Isochinolin oder substituiert an der Gruppe 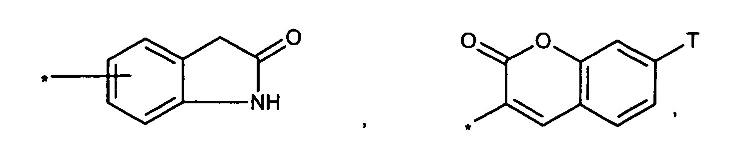 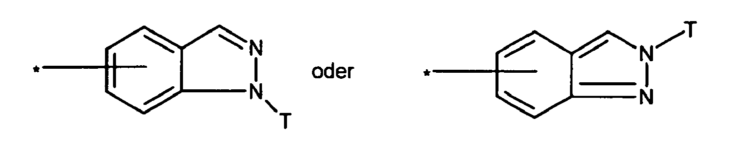 steht, in der T für Wasserstoff, C₁-C₆-Alkyl oder C₁-C₆-Alkoxy steht,
- R² und R³: unabhängig voneinander für Wasserstoff oder für gegebenenfalls ein- oder mehrfach, gleich oder verschieden mit Halogen, C₁-C₆-Alkyl, Phenyl oder mit der Gruppe -NR⁶R⁷, -OR⁵ oder C₁-C₆-Alkyl-OR⁵, substituiertes C₁-C₆-Alkyl, C₃-C₆-Cycloalkyl, Phenyl oder Pyridyl steht, oder
- R² und R³: gemeinsam mit dem Stickstoff-Atom einen C₃-C₈-Ring bilden, der gegebenenfalls ein weiteres Stickstoff- oder Sauerstoffatom im Ring enthalten kann, und der gegebenenfalls ein oder mehrfach, gleich oder verschieden mit C₁-C₆-Alkyl substituiert sein kann,
- R⁴: für Hydroxy oder für die Gruppe -NR²R³ steht,
- R⁵, R⁶ und R⁷: unabhängig voneinander für Wasserstoff oder C₁-C₆-Alkyl stehen,
oder
- R⁶ und R⁷: gemeinsam einen 5 bis 7 gliedrigen Ring bilden, der ein Sauerstoff- oder Schwefel-Atom enthalten kann,
- R⁸: für Wasserstoff, C₁-C₆-Alkyl oder Benzyl steht, und
- R⁹: für Wasserstoff, C₁-C₆-Alkyl oder Tri-C₁-C₆-alkylsilyl steht,
bedeuten, sowie deren Isomeren und Salze.

Die erfindungsgemäßen Verbindungen sowie deren physiologisch verträglichen Salze verhindern eine Tyrosinphosphorylierung bzw. stoppen die persistente Angiogenese und damit das Wachstum und ein Ausbreiten von Tumoren, wobei sie sich insbesondere durch eine geringere Inhibition von Isoformen des Cytochroms P 450 (2C9 und 2C19) auszeichnen. Die Medikation mit den erfindungsgemäßen Verbindungen kann daher auch ohne Rücksicht auf begleitend verabreichte Arzneimittel, die über diese Isoformen abgebaut werden, risikolos erfolgen.

Die Verbindungen der Formel I sowie deren physiologisch verträglichen Salze sind auf Grund ihrer inhibitorischen Aktivität in Bezug auf Phosphorylierung des VEGF-Rezeptors als Arzneimittel verwendbar. Auf Grund ihres Wirkprofils eignen sich die erfindungsgemäßen Verbindungen zur Behandlung von Erkrankungen, die durch eine persistente Angiogenese hervorgerufen oder gefördert werden.

Da die Verbindungen der Formel I als Inhibitoren der Tyrosinkinasen VEGFR-1 und VEGFR-2 identifiziert werden, eignen sie sich insbesondere zur Behandlung von solchen Krankheiten, die durch die über den VEGF-Rezeptor ausgelöste persistente Angiogenese oder eine Erhöhung der Gefäßpermeabilität hervorgerufen oder gefördert werden.

Gegenstand der vorliegenden Erfindung sind somit auch Arzneimittel zur Behandlung von Tumoren bzw. deren Verwendung.

Die erfindungsgemäßen Verbindungen können entweder alleine oder in Formulierung als Arzneimittel zur Behandlung von Psoriasis, Kaposis Sarkom, Restenose, wie z. B. Stent-induzierte Restenose, Endometriose, Crohns disease, Hodgkins disease, Leukämie, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen, Artheriosklerose, Verletzungen des Nervengewebes und zur Hemmung der Reocclusion von Gefäßen nach Ballonkatheterbehandlung, bei der Gefäßprothetik oder nach dem Einsetzen von mechanischen Vorrichtungen zum Offenhalten von Gefäßen, wie z. B. Stents, als Immunsuppressiva, zur Unterstützung der narbenfreien Wundheilung, bei Altersflecken und bei Kontaktdermatitis zum Einsatz kommen.

Bei der Behandlung von Verletzungen des Nervengewebes kann mit den erfindungsgemäßen Verbindungen eine schnelle Narbenbildung an den Verletzungsstellen verhindert werden, d. h. es wird verhindert, daß die Narbenbildung eintritt, bevor die Axone wieder Verbindung miteinander aufnehmen. Damit würde eine Rekonstruktion der Nervenverbindungen erleichtert.

Ferner kann mit den erfindungsgemäßen Verbindungen die Ascites-Bildung bei Patienten unterdrückt werden. Ebenso lassen sich VEGF bedingte Ödeme unterdrücken.

Die Lymphangiogenese spielt eine wichtige Rolle bei der lymphogenen Metastasierung (Karpanen, T. et al., Cancere Res. 2001 Mar 1, 61(5): 1786-90, Veikkola T. et al., EMBO J. 2001, Mar 15; 20 (6): 1223-31).

Die erfindungsgemäßen Verbindungen zeigen nun ebenfalls hervorragende Wirkung als VEGFR Kinase 3 - Inhibitoren und eignen sich daher auch als wirksame Inhibitoren der Lymphangiogenese.
Durch eine Behandlung mit den erfindungsgemäßen Verbindungen wird nicht nur eine Reduzierung der Größenentwicklung von Metastasen, sondern auch eine Verringerung der Anzahl der Metastasen erreicht.
Die erfindungsgemäßen Verbindungen sind auch wirksam bei Erkrankungen, die mit übermäßiger Lymphangiogenese verbunden sind und daher zum Lymphangiohyper und -dysplasie-Syndrom gerechnet werden.

Derartige Arzneimittel, deren Formulierungen und Verwendungen sind ebenfalls Gegenstand der vorliegenden Erfindung.

Die Erfindung betrifft somit ferner die Verwendung der Verbindungen der allgemeinen Formel I, zur Herstellung eines Arzneimittels zur Verwendung als, bzw. zur Behandlung von Psoriasis, Kaposis Sarkom, Restenose, wie z. B. Stent-induzierte Restenose, Endometriose, Crohns disease, Hodgkins disease, Leukämie, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen, Artheriosklerose, Verletzungen des Nervengewebes und zur Hemmung der Reocclusion von Gefäßen nach Ballonkatheterbehandlung, bei der Gefäßprothetik oder nach dem Einsetzen von mechanischen Vorrichtungen zum Offenhalten von Gefäßen, wie z. B. Stents, als Immunsuppressiva, als Unterstützung bei der narbenfreien Wundheilung, bei Altersflecken und bei Kontaktdermatitis.

Ferner kann mit den erfindungsgemäßen Verbindungen die Ascites-Bildung bei Patienten unterdrückt werden. Ebenso lassen sich VEGF bedingte Ödeme unterdrücken.

Zur Verwendung der Verbindungen der Formel I als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe wie Konservierungs-, Stabilisierungs-, Netzmittel oder Emulgatoren, Salze zur Veränderung des osmotischen Drucks oder Puffer.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff oder bei Bedarf ein oder mehrere Geschmacksstoffe beigefügt ist.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5-1000 mg, vorzugsweise 50-200 mg, wobei die Dosis als einmal zu verabreichende Einzeldosis oder unterteilt in 2 oder mehreren Tagesdosen gegeben werden kann.
Die oben beschrieben Formulierungen und Darreichungsformen sind ebenfalls Gegenstand der vorliegenden Erfindung.

Die Herstellung der erfindungsgemäßen Verbindungen erfolgt nach an sich bekannten Methoden. Beispielsweise gelangt man zu Verbindungen der allgemeinen Formel I dadurch, daß man eine Verbindung der allgemeinen Formel II, 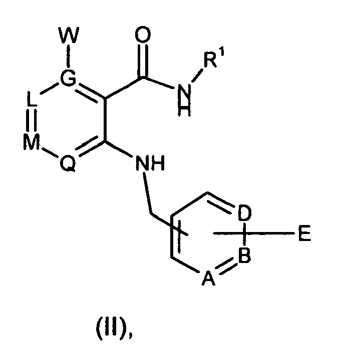 worin A, B, D, G, L, M, Q, W und R¹ die in der allgemeinen Formel I angegebenen Bedeutungen haben und E für eine Carbonsäure -COOH steht, in einem geeigneten Lösungsmittel und einer geeigneten organischen Base, mit einem Amin der allgemeinen Formel III

H-NR⁸R⁹ (III),

worin R⁸ und R⁹ die in der allgemeinen Formel I angegebenen Bedeutungen haben, nach an sich literaturbekannten Verfahren umsetzt oder wenn E eine Nitrilgruppe bedeutet, das Nitril zum Amid verseift, oder eine Verbindung der allgemeinen Formel IV 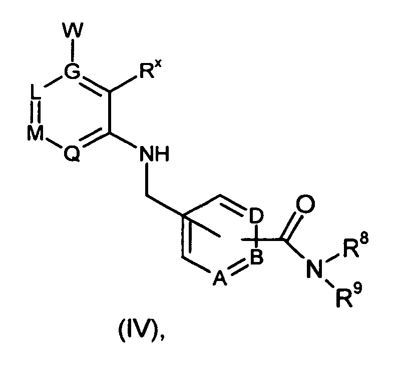 worin A, B, D, G, L, M, Q, W, R⁸ und R⁹ die in der allgemeinen Formel I angegebenen Bedeutungen haben und R^{x} eine Ester- oder Säuregruppe bedeutet, in das entsprechende Amid überführt.

Die Amidbildung erfolgt nach literaturbekannten Methoden.
Zur Amidbildung kann man von einem entsprechenden Ester ausgehen. Der Ester wird nach J. Org. Chem. 1995, 8414 mit Aluminiumtrimethyl und dem entsprechenden Amin in Lösungsmitteln wie Toluol bei Temperaturen von 0°C bis zum Siedepunkt des Lösungsmittels umgesetzt. Enthält das Molekül zwei Estergruppen, werden beide in das gleiche Amid überführt. Statt Aluminiumtrimethyl kann man auch Natriumhexamethyldisilazid verwenden.

Zur Amidbildung stehen aber auch alle aus der Peptidchemie bekannten Verfahren zur Verfügung. Beispielsweise kann die entsprechende Säure in aprotischen polaren Lösungsmitteln wie zum Beispiel Dimethylformamid über eine aktiviertes Säurederivat, zum Beispiel erhältlich mit Hydroxybenzotriazol und einem Carbodiimid wie zum Beispiel Diisopropylcarbodiimid oder auch mit vorgebildeten Reagenzien wie zum Beispiel HATU (Chem. Comm. 1994, 201), oder BTU, bei Temperaturen zwischen 0°C und dem Siedepunkt des Lösungsmittels vorzugsweise bei 80°C mit dem Amin umgesetzt werden. Für die Amidbildung kann auch das Verfahren über das gemischte Säureanhydrid, Imidazolid oder Azid eingesetzt werden.

Nitrile lassen sich nach literaturbekannten Verfahren zu Amiden verseifen. Sehr effektiv ist die Umsetzung mit Kaliumkarbonat und Wasserstoffperoxid in einem aprotischen polaren Lösungsmittel wie Dimethylsulfoxid vorzugsweise bei Raumtemperatur nach Synthesis, 1989, 949.

Weiterhin lassen sich die erfindungsgemäßen Verbindungen der allgemeinen Formel I dadurch herstellen, daß man eine Verbindung der Allgemeinen Formel Ila 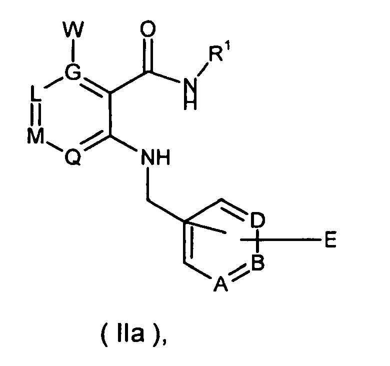 worin A, B, D, G, L, M, Q, W und R¹ die in der allgemeinen Formel I angegebenen Bedeutungen haben und E ein Halogen oder ein O-Sulfonat, wie z.B. ein Chlor-, Brom- oder lodatom, ein O-Trifluormethansulfonat oder O-Methylsulfonat bedeutet,
a. mit geeignet substituierten terminalen Alkenen in einer *Heck*-Reaktion (cf. "Palladium Reagents in Organic Syntheses", Academic Press 1985, New York, S. 179ff.) oder mit Vinylboronsäuren oder Vinylboronsäureestern in einer *Suzuki*-Reaktion (cf. Tetrahedron Lett. 1983, 39, 3271ff.) oder mit Vinylstannanen in einer *Stille*-Reaktion (cf. Pure & Appl. Chem. 1985, 57, 1771) umgesetzt wird, oder
b. mit beliebig substituierten terminalen Alkinen beispielsweise nach der Methode von *Stephens-Castro* (cf. J. Org. Chem. 1963, 28, 3313ff.) oder Palladium-katalysiert nach der Methode von *Sonogashira* (cf. "Comprehensive Organic Synthesis: Carbon-Carbon σ-Bond Formation", Pergamon Press 1991, Oxford UK, Volume 3, S. 551ff.) gekuppelt wird, oder
c. mit Aryl- und Hetarylborsäuren oder deren Estern in einer *Suzuki*-Reaktion (cf. Acc. Chem. Res. 1991, 63, 419ff. oder J. Am. Chem. Soc. 2000, 122, 4020ff.), oder mit Aryl- und Hetarylstannanen in einer *Stille*-Reaktion (cf. Angew. Chem. 1986, 98, 504ff. oder Angew. Chem. Int. Ed. 1999, 38, 2411ff.) oder mit Aryl- und Hetaryl-Grignardverbindungen oder den analogen Zinkorganischen Derivaten in einer *Negishi*-Reaktion (cf. "Metal-catalyzed Cross-coupling Reaction" Eds. Diederich/Stang, Wiley-VCH 1998, New York, Chapter 1 oder auch J. Am. Chem. Soc. 2001, 123, 2719ff.) gekuppelt wird, oder
d. in einer Palladium-katalysierten Carbonylierung unter 1 bis 20 bar Kohlenmonoxidatmosphäre in Dimethylformamid in Gegenwart des entsprechenden Alkohols (cf. "Palladium Reagents in Organic Syntheses", Academic Press 1985, New York, S. 352ff. oder Synth. Comm. 1997, 27, 515ff.) den entsprechenden Carbonsäureester überführt wird, oder
e. in einer Palladium-katalysierten Carbonylierung unter 1 bis 20 bar Kohlenmonoxidatmosphäre in Dimethylformamid-Wasser Gemischen in die entsprechende Carbonsäure überführt wird (cf. J. Org. Chem. 1981, 46, 4614ff.). Durch Verseifung der Carbonsäureester können ebenfalls die Carbonsäuren erhalten werden, oder
f. in einer Palladium-katalysierten Carbonylierung unter 1 bis 20 bar Kohlenmonoxidatmosphäre in Dimethylformamid in Gegenwart von Aminen die entsprechenden Carbonsäureamide hergestellt werden (cf. "Palladium Reagents in Organic Syntheses", Academic Press 1985, New York, S. 352ff., Tetrahedron Lett. 1982, 23, 3383ff.). Die Synthese der Carbonsäureamide kann ebenfalls aus Carbonsäureestern erfolgen; besonders bewährt hat sich hier die Methode nach *Weinreb* (cf. Tetrahedron Lett. 1977, 17, 4171ff., J. Org, Chem. 1995, 60, 8414ff.). Die Carbonsäureamide können ebenfalls aus den unter e) hergestellten Carbonsäuren synthetisiert werden; dazu stehen grundsätzlich alle aus der Peptidchemie bekannten Verfahren zur Verfügung (cf. Synthesis 1972, 453-63 oder "Comprehensive Organic Transformations", Wiley-VCH 1989, New York, 972-6). Beispielsweise kann die entsprechende Carbonsäure in aprotischen polaren Lösungsmitteln, wie zum Beispiel Dimethylformamid, über ein aktiviertes Carbonsäurederivat, beispielsweise erzeugt durch Zugabe von Carbonyldiimidazol, bei Temperaturen zwischen 0-120 °C, vorzugsweise bei Raumtemperatur, mit Aminen umgesetzt werden, oder
g. mit Thioalkylen, -arylen und -hetarylen direkt, in Gegenwart von Basen, wie beispielsweise Kaliumhydrid oder Kaliumtertiärbutanolat oder Übergangsmetallen, wie beispielsweise Kupferspänen, Kupferchlorid oder- bromid oder Palladiumdichlorid in aprotischen Lösungsmitteln, wie beispielsweise Dimethylformamid, N-Methylpyrrolidon, Dimethylsulfoxid oder Xylol bei Temperaturen zwischen 20-200 °C das entsprechende Sulfid überführt wird. Die Durchführung der Reaktion in einer Mikrowellenapperatur kann sich dabei als vorteilhaft erweisen (cf. Tetrahedron 1983, 39, 4153ff.). Die Herstellung von 2-Thiosubstituierten Pyridylderivaten kann auch leicht aus dem 2-Pyridonderivat nach Thionylierung mit Phosphorpentasulfid (cf. Bull. Soc. Chim. Fr.; 1953; 1001ff.) oder *Lawesson*-Reagenz (Tetrahedron 1984, 40, 2047ff.) und anschließender Alkylierung mit Alkylhalogeniden, vorzugsweise mit Alkyliodiden (cf. J. Org. Chem. 1999; 64, 7935-9) oder Alkylsulfonaten, vorzugsweise Alkyltrifluormethylsulfonaten, erfolgen.
h. Durch Oxidation der Sulfide mit gängigen Oxidationsmitteln wie beispielsweise Wasserstoffperoxid, Natriumperiodat, Teritiärbutoxyhypochlorit, Natriumchlorit, Metachlorperbenzoesäure, Trifluorperessigsäure, Dimethyldioxiran, Cerammoniumnitrat oder Salpetersäure (cf. "Oxidations in Organic Chemistry", ACS Washington 1990, S.252-63) in Lösungsmitteln, wie beispielweise Dichlormethan, Dichlorethan, Chloroform, Tetrahydrofuran, Acetonitril, Dimethylformamid, N-Methylpyrrolidinon, Dimethylsulfoxid, Dimethoxyethan, Diglym, Tetraglym oder Wasser, bei Temperaturen zwischen 20°C und dem Siedepunkt des Lösungsmittels können die entsprechenden Sulfoxide erhalten werden. Die so erhaltenen Sulfoxide können weiter zu den entsprechenden Sulfonen oxidiert werden; dieses wird beispielsweise durch Oxidationsmittel wie Wasserstoffperoxid, Kaliumpermanganat, Natriumperborat oder Kaliumhydrogenpersulfat (cf. Tetrahedron Lett. 1981, 22, 1287ff.) in Lösungsmitteln, wie beispielweise Dichlormethan, Dichlorethan, Chloroform, Tetrahydrofuran, Acetonitril, Dimethylformamid, N,N-Dimethylacetamid, Dimethylsulfoxid oder Wasser, bei Temperaturen zwischen 20°C und dem Siedepunkt des Lösungsmittels erreicht. Die Behandlung von Sulfiden mit einem Überschuß der oben aufgeführten Oxidationsmittel führt direkt zu den entsprechenden Sulfonen (cf. "The chemistry of sulphones and sulfoxides" in Patai, Wiley 1988, New York, S.165-231).
i. Durch Oxidation der unter g) erhaltenen Thiole können die Chlorsulfonate hergestellt werden; besonders bewährt haben sich hier die Oxidation mit Chlor in wäßriger Salzsäure (cf. J. Org. Chem. 1999; 64, 5896-903) oder Tetrachlorkohlenstoff (cf. J. Med. Chem. 2000, 43, 843-58) oder mit Natriumhypochlorit in Schwefelsäure (cf. Tetrahedron Asymm. 1997; 8; 3559-62).
j. Durch Umsetzung mit einem Gemisch aus Kupferrhodanit und Kaliumrhodanit in polar aprotischen Lösungsmitteln, wie beispielsweise Acetonitril, Dimethylformamid, N,N-Dimethylacetamid, Dimethylsulfoxid, Diglym, Tetraglym, N-Methylpyrrolidinon können die entsprechenden Thiocyanate erhalten werden (cf. J. Chem. Soc. Chem. Comm. 1989, 81ff). Aus diesen wiederum können durch Oxidation mit Hypochlorit die entsprechenden Sulfonsäurechloride erhalten werden.
k. Durch Umsetzung der unter i) aufgeführten Chlorsulfonate mit Aminen, in Lösungsmitteln, wie beispielweise Dichlormethan, Dichlorethan, Chloroform, Tetrahydrofuran, Ethylacetat, Acetonitril, Dimethylformamid, N-Methylpyrrolidinon, N,N-Dimethylacetamid, Dimethoxyethan oder Wasser, bei Temperaturen zwischen 0°C und dem Siedepunkt des Lösungsmittels, können die entsprechenden Sulfonamide erhalten werden (cf. Tetrahedron 2000, 56, 8253-62).
l. Durch Hydrolyse der unter i) erhaltenen Chlorsulfonate in Wasser oder wäßriger Lauge bei Temperaturen zwischen 5° und 100°C werden die entsprechenden Sulfonsäuren erhalten.
m. Durch Palladium-katalysierte Umsetzung mit O,O-Dialkylphosphonaten in aprotischen Lösungsmitteln wie beispielsweise Dimethylformamid, N-Methylpyrrolidinon, N,N-Dimethylacetamid, Dimethylsulfoxid oder Toluol in Gegenwart einer Base, wie beispielsweise Triethylamin oder Diisopropylethylamin, bei Temperaturen zwischen 0° und der Siedetemperatur des Lösungsmittels, bevorzugt bei 80°C, können die entsprechenden Phosphonate erhalten werden (cf. Bull. Chem. Soc. Jpn. 1982, 55, 909ff.).
n. Durch Metallierung, beispielsweise mit n-Butyllitium, sec-Butyllithium, tert.-Butyllithium, Methyllithium, Lithiumdiisopropyamid oder Ethylmagnesiumbromid, in aprotischen Lösungsmitteln wie beispielsweise Diethylether, Tetrahydrofuran oder Dioxan, bei Temperaturen zwischen -100°C und 0°C, vorzugsweise bei -78°C in Tetrahydrofuran und Reaktion mit Isocyanaten können die entsprechenden Carbonsäureamide erhalten werden.
o. Durch analoge Reaktionsführung wie unter n) beschrieben und Abfangen der metallierten Zwischenstufe mit Carbamoylchloriden können die entsprechenden Carbonsäureester erhalten werden.
p. Durch analoge Reaktionsführung wie unter n) beschrieben und Abfangen der metallierten Zwischenstufe mit Dimethylformamid, Ethylformiat oder N-Formylmorpholin können die entsprechenden Aldehyde erhalten werden.
q. Durch analoge Reaktionsführung wie unter n) beschrieben und Abfangen der metallierten Zwischenstufe mit Alkylhalogeniden oder Alkylsulfonaten, vorzugsweise Alkyliodide oder Alkyltrifluormethansulfonate, können die entsprechenden Pyridylalkylderivate ergeben.
r. Durch Reduktion mit Wasserstoff in Gegenwart katalytischer Mengen Palladium-, Nickel- oder Rhodiummetall oder Salze dieser Metalle, beispielsweise Palladium auf Aktivkohle in polar-protischen Lösungsmitteln oder Lösungsmittelgemischen, wie beispielsweise Methanol-Eisessig können die unter a) hergestellten Pyridylalkene und die unter b) hergestellten Pyridylalkine in die entsprechenden Pyridylalkane übergeführt werden.

Die Reihenfolge der Verfahrensschritte kann in allen Fällen auch vertauscht werden.

### Herstellung der erfindungsgemäßen Verbindungen

Die nachfolgenden Beispiele erläutern die Herstellung der erfindungsgemäßen Verbindungen, ohne den Umfang der beanspruchten Verbindungen auf diese Beispiele zu beschränken.

### Beispiel 1

### Herstellung von 5-{[2-(Isochinolin-3-ylcarbamoyl)-phenylamino]-methyl}-pyridin-2-carboxylic acid propylamid

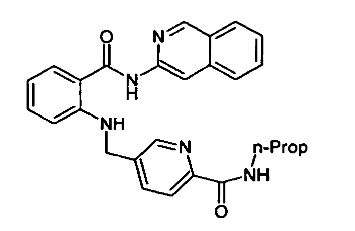

In 2,5 ml Dimethylformamid werden unter Argon und Feuchtigkeitsausschluss 50 mg (0,13 mMol) 5-{[2-(Isochinolin-3-ylcarbamoyl)-phenylamino]-methyl}-pyridin-2-carbonsäure und 42 mg (0,26 mMol) Carbonyldiimidazol eingetragen und 30min bei Raumtemperatur gerührt. Man fügt dann 15 mg (0,26 mMol) n-Propylamin zu dem Ansatz und rührt 12h bei Raumtemperatur weiter. Es wird dann mit Wasser auf ca. 30ml verdünnt und dreimal mit je 20 ml Essigester ausgeschüttelt. Die gesammelte organische Phase wird getrocknet, filtriert und eingeengt und der Rückstand über eine Flash Säule (5 g; Isolute flash silica, Fa. Separtis) mit einem Gradienten von 100% Hexan auf 50% Hexan und 50% Essigester chromatographiert. Man erhält 45 mg (79 % der Theorie) 5-{[2-(Isochinolin-3-ylcarbamoyl)-phenylamino]-methyl}-pyridin-2-carboxylic acid propylamid mit einem Molpeak im MS m/e= 439.

In analoger Verfahrensweise werden auch die nachfolgenden Beispiele hergestellt:

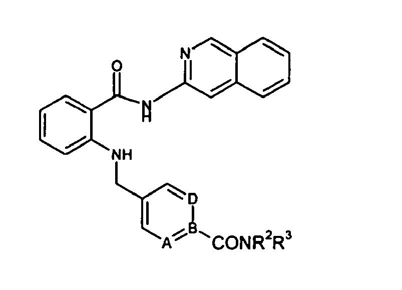

| **Beispiel Nr.** | **A** | **B** | **D** | **R²** | **R³** | **MW** | **Smp. [°C] / MS Molpeak (m/e)** |
|---|---|---|---|---|---|---|---|
| 1.1 | C | C | N | -CH₃ | -CH₃ | 425,49 | |
| 1.2 | C | C | N | -CH(CH₃)₂ | H | 439,52 | Harz/ 439 |
| 1.3 | C | C | N | 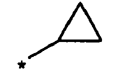 | H | 437,50 | Harz/ 437 |
| 1.4 | C | C | N | -CH₂CF₃ | H | | |
| 1.5 | C | C | N | -(CH₂)₂-OH | H | 441,49 | Harz/ 441 |
| 1.6 | C | C | N | -(CH₂)₃OH | H | 455,52 | Harz/ 455 |
| 1.7 | C | C | N | -(CH₂)₄OH | H | 469,54 | Harz/ 469 |
| 1.8 | C | C | N | 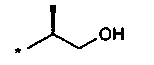 | H | 455, 52 | 155 |
| 1.9 | C | C | N | 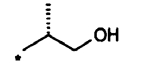 | H | 455,52 | Harz/455 |
| 1.10 | C | C | N | 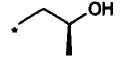 | H | 455, 52 | 109 |
| 1.11 | C | C | N | 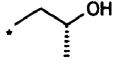 | H | 455, 52 | 82 |
| 1.12 | C | C | N | 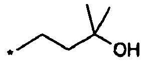 | | 483,87 | Harz/ 483 |
| 1.13 | C | C | N | -(CH₂)₂N(CH₃)₂ | H | 468, | /468 |
| 1.14 | C | C | N | -(CH₂)₃N(CH₃)₂ | H | 482,59 | /469 |

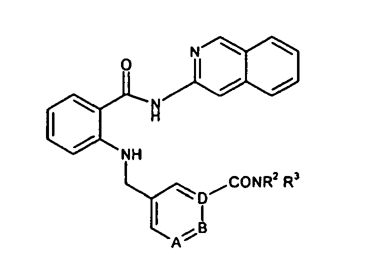

| **Beispiel Nr.** | **A** | **B** | **D** | **R²** | **R³** | **MW** | **Smp. [°C] / MS Molpeak (m/e)** |
|---|---|---|---|---|---|---|---|
| 1.15 | C | N | C | -CH₃ | -CH₃ | 425,49 | 106 |
| 1.16 | C | N | C | -CH₃ | H | 411,46 | 180 |
| 1.17 | C | N | C | -C₂H₅ | H | 425,49 | 165 |
| 1.18 | C | N | C | 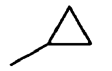 | H | 437,50 | 172 |
| 1.19 | C | N | C | -(CH₂)₂-OH | H | 441,49 | 136 |
| 1.20 | C | N | C | 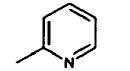 | H | 474,52 | 207 |
| 1.21 | C | N | C | 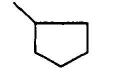 | H | 465,55 | 94 |
| 1.22 | C | N | C | 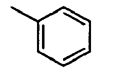 | H | 473,53 | 187 |
| 1.23 | C | N | C | -C₃H₇ | H | 439, 52 | 96 |
| 1.24 | C | N | C | -CH(CH₃)₂ | H | 439,52 | 174 |
| 1.25 | C | N | C | 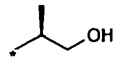 | H | 455,52 | 103 |
| 1.26 | C | N | C | 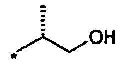 | H | 455,52 | 110 |
| 1.27 | C | N | C | 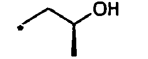 | H | 455,52 | 105 |
| 1.28 | C | N | C | 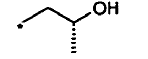 | H | 455,52 | 100 |
| 1.29 | C | N | C | 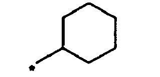 | H | 479,58 | 110 |
| 1.30 | C | N | C | 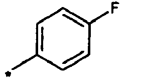 | H | 491,52 | 204 |
| 1.31 | C | N | C | 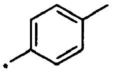 | H | 487,56 | 151 |
| 1.32 | C | N | C | -(CH₂)₃-OH | H | 455,52 | 65 |
| 1.33 | C | N | C | -(CH₂)₅-OH | H | 483,57 | 70 |
| 1.34 | C | N | C | -(CH₂)₄-OH | H | 469,54 | 70 |
| 1.35 | C | N | C | -(CH₂)₂N(CH₃)₂ | H | 455,52 | 98 |
| 1.36 | C | N | C | -(CH₂)₃N(CH₃)₂ | H | 482,59 | 95 |
| 1.37 | C | N | C | 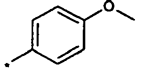 | H | 503,56 | 190 |
| 1.38 | C | N | C | 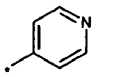 | H | 474,52 | 190 |
| 1.39 | C | N | C | 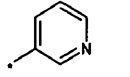 | H | 474,52 | 105 |
| 1.40 | C | N | C | 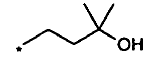 | H | 483,57 | 75 |
| 1.41 | C | N | C | 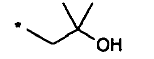 | H | 469,54 | 50 |
| 1.42 | C | N | C | 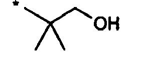 | H | 469,54 | 170 |
| 1.43 | C | N | C | -(CH₂)₂OCH₃ | H | 455,52 | 67 |
| 1.44 | C | N | C | 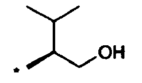 | H | 483,57 | 86 |
| 1.45 | C | N | C | 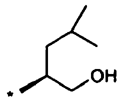 | H | 497,6 | 86 |
| 1.46 | C | N | C | 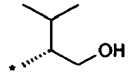 | H | 483,57 | 66 |
| 1.47 | C | N | C | 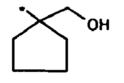 | H | 495,58 | 148 |
| 1.48 | C | N | C | 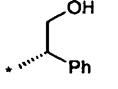 | H | 517,58 | 78 |
| 1.49 | C | N | C | 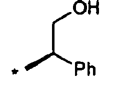 | H | 517,58 | 91 |
| 1.50 | C | N | C | 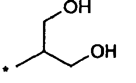 | H | 471,51 | 85 |
| 1.51 | C | N | C | 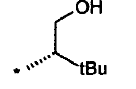 | H | 497,59 | 98 |
| 1.52 | C | N | C | CH₂CF₃ | H | 479,46 | 96 |
| 1.53 | C | N | C | 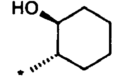 | H | 495,58 | 127 |
| 1.54 | C | N | C | 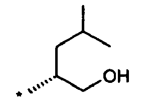 | H | 497,59 | 96 |
| 1.55 | C | N | C | 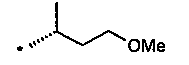 | H | 469,54 | 78 |
| 1.56 | C | N | C | 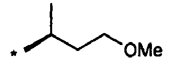 | H | 469,54 | 78 |
| 1.57 | C | N | C | 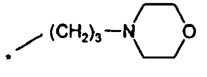 | H | 510,59 | |
| 1.58 | C | N | C | 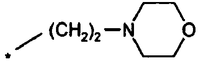 | H | 524,62 | |
| 1.59 | C | N | C | 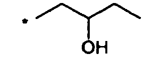 | H | 469,54 | |

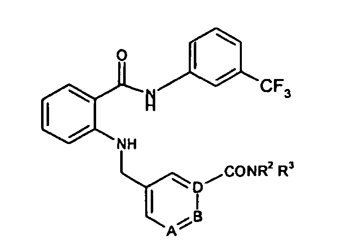

| **Beispiel Nr.** | **A** | **D** | **B** | **R²** | **R³** | **MW** | Smp. [°C] oder MS Molpeak (m/e) |
|---|---|---|---|---|---|---|---|
| 1.60 | C | C | N | -CH(CH₂OH)₂ | H | 488,46 | 97 |
| 1.61 | C | C | N | -(CH₂)₃OH | H | 500,52 | 125 |
| 1.62 | C | C | N | -(CH₂)₂-OMe | H | 472,46 | 67 |
| 1.63 | C | C | N | -(CH₂)₅OH | H | 500,52 | 92 |
| 1.64 | C | C | N | -(CH₂)₄OH | H | 486,49 | 73 |
| 1.65 | C | C | N | 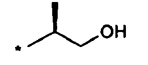 | H | 472,46 | 82 |
| 1.66 | C | C | N | 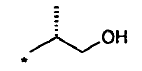 | H | 472,46 | 73 |
| 1.67 | C | C | N | 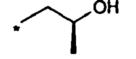 | H | 472,46 | 87 |
| 1.68 | C | C | N | 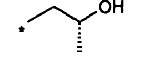 | H | 472,46 | 93 |
| 1.69 | C | C | N | 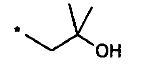 | H | 486,49 | 67 |
| 1.70 | C | C | N | 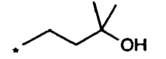 | H | 500,52 | 67 |
| 1.71 | C | C | N | -(CH₂)₂N(CH₃)₂ | H | 485,51 | 82 |
| 1.72 | C | C | N | -(CH₂)₃N(CH₃)₂ | H | 499,53 | 74 |
| 1.73 | C | C | N | 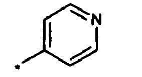 | H | 491,47 | 142 |
| 1.74 | C | C | N | 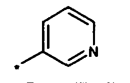 | H | 491,47 | 104 |
| 1.75 | C | C | N | 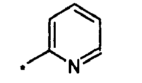 | H | 491,47 | 73 |

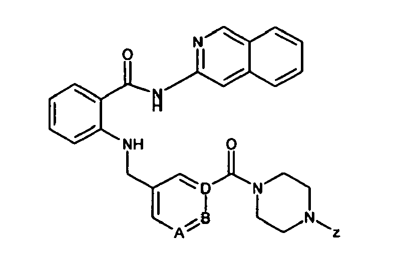

| **Beispiel Nr.** | **A** | **B** | **D** | **Z** | **MW** | **Smp. [°C] / MS Molpeak (m/e)** |
|---|---|---|---|---|---|---|
| 1.76 | C | N | C | -CH₃ | 480, 57 | 99 |

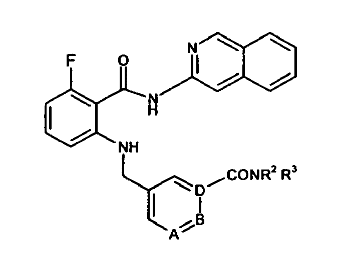

| **Beispiel Nr.** | **A** | **B** | **D** | **R²** | **R³** | **MW** | **Smp. [°C] / MS Molpeak (m/e)** |
|---|---|---|---|---|---|---|---|
| 1.77 | C | N | C | 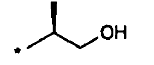 | H | 473,50 | |
| 1.78 | C | N | C | 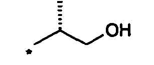 | H | 473,50 | |
| 1.79 | C | N | C | 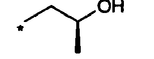 | H | 473,50 | |
| 1.80 | C | N | C | 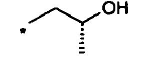 | H | 473,50 | |
| 1.81 | C | N | C | -(CH₂)₃N(CH₃)₂ | H | 486,54 | |

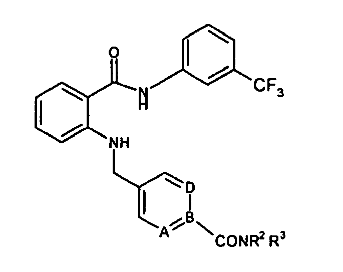

| **Beispiel Nr.** | **A** | **B** | **D** | **R²** | **R³** | **MW** | Smp. [°C] oder MS Molpeak (m/e) |
|---|---|---|---|---|---|---|---|
| 1.82 | C | C | N | -(CH₂)₃OH | H | 500,52 | 80 |
| 1.83 | C | C | N | 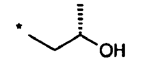 | H | 472,46 | 50 |
| 1.84 | C | C | N | 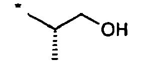 | H | 472,46 | 83 |
| 1.85 | C | C | N | 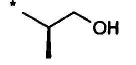 | H | 472,46 | 129 |
| 1.86 | C | C | N | 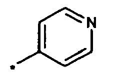 | H | 491,47 | 150 |
| 1.87 | C | C | N | 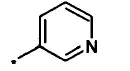 | H | 491,47 | 148 |
| 1.88 | C | C | N | -(CH₂)₅OH | H | 500,52 | 101 |
| 1.89 | C | C | N | -CH(CH₂OH)₂ | H | 488,46 | 144 |
| 1.90 | C | C | N | -(CH₂)₃N(CH₃)₂ | H | 499,53 | 117 |
| 1.91 | C | C | N | -(CH₂)₂-OMe | H | 472,46 | 54 |
| 1.92 | C | C | N | 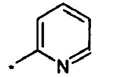 | H | 491,47 | 121 |
| 1.93 | C | C | N | -(CH₂)₂N(CH₃)₂ | H | 485,51 | 139 |
| 1.94 | C | C | N | 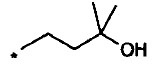 | H | 500,52 | 70 |
| 1.95 | C | C | N | 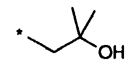 | H | 486,49 | 88 |
| 1.96 | C | C | N | 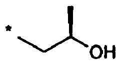 | H | 472,46 | 76 |

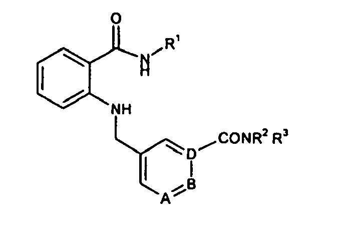

| **Beispiel Nr.** | **A** | **B** | **D** | **R¹** | **R²** | **R³** | **MW** | **Smp. [°C] / MS Molpeak (m/e)** |
|---|---|---|---|---|---|---|---|---|
| 1.97 | C | N | C | 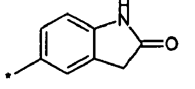 | 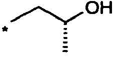 | H | 459,50 | |
| 1.98 | C | N | C | 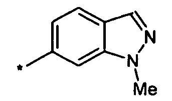 | 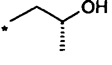 | H | 458,51 | |
| 1.99 | C | N | C | 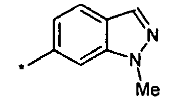 | 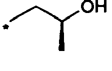 | H | 458,51 | |
| 1.100 | C | N | C | 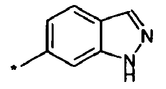 | 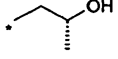 | H | 444,49 | |
| 1.101 | C | N | C | 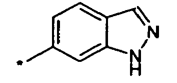 | 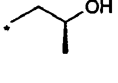 | H | 444,49 | |
| 1.102 | C | N | C | 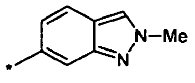 | 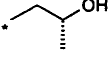 | H | 458,51 | |
| 1.103 | C | N | C | 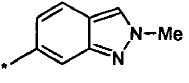 | 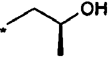 | H | 458,51 | |
| 1.104 | C | N | C | 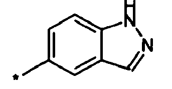 | 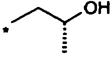 | H | 444,49 | |
| 1.105 | C | N | C | 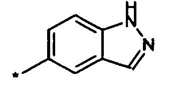 | 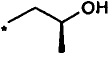 | H | 444,49 | |

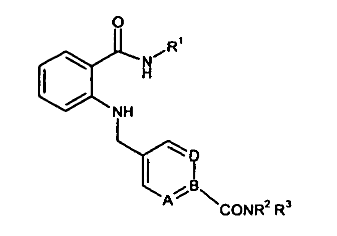

| **Beispiel Nr.** | **A** | **D** | **B** | **R¹** | **R²** | **R³** | **MW** | **Smp. [°C] / MS Molpeak (m/e)** |
|---|---|---|---|---|---|---|---|---|
| 1.106 | C | N | C | 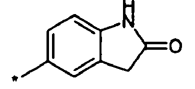 | 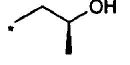 | H | 459,50 | 160,7 |
| 1.107 | C | N | C | 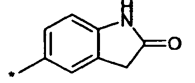 | 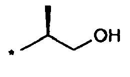 | H | 459,50 | 123,8 |
| 1.108 | C | N | C | 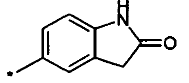 | 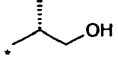 | H | 459,50 | 123 |
| 1.109 | C | N | C | 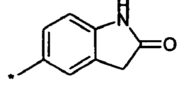 | 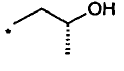 | H | 459,50 | |
| 1.110 | C | N | C | 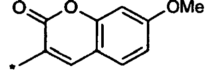 | 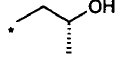 | H | 502,52 | 199,2 |
| 1.111 | C | N | C | 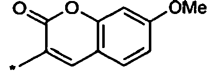 | 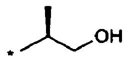 | H | 502,52 | 180,4 |
| 1.112 | C | N | C | 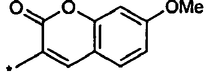 | 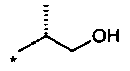 | H | 502,52 | |
| 1.113 | C | N | C | 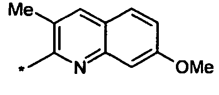 | 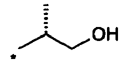 | H | 499,56 | |
| 1.114 | C | N | C | 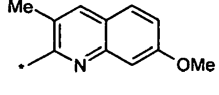 | 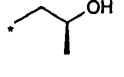 | H | 499, 56 | 174 |
| 1.115 | C | N | C | 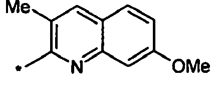 | 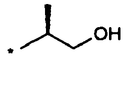 | H | 499,56 | 173,8 |
| 1.116 | C | N | C | 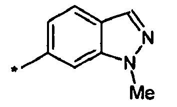 | 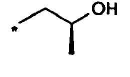 | H | 458,51 | |
| 1.117 | C | N | C | 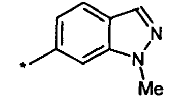 | 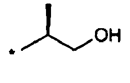 | H | 458,51 | |
| 1.118 | C | N | C | 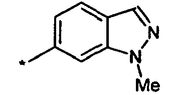 | 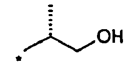 | H | 458,51 | |
| 1.119 | C | N | C | 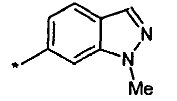 | 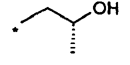 | H | 458,51 | |
| 1.120 | C | N | C | 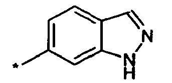 | 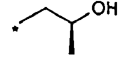 | H | 444,49 | |
| 1.121 | C | N | C | 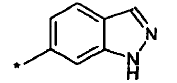 | 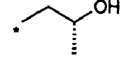 | H | 444,49 | |
| 1.122 | C | N | C | 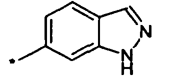 | 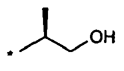 | H | 444,49 | |
| 1.123 | C | N | C | 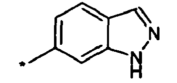 | 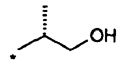 | H | 444,49 | |

### Beispiel 2.0

### Herstellung von 5-{[2-(Isochinolin-3-ylcarbamoyl)-phenylamino]-methyl}-pyridin-2-carbonsäure pyridin-3-ylamid

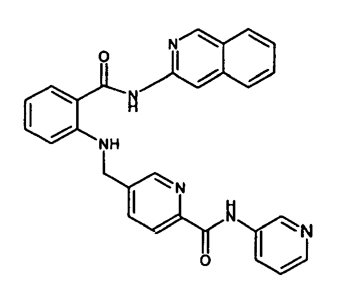

120 mg (0,3 mMol) 5-{[2-(Isochinolin-3-ylcarbamoyl)-phenylamino]-methyl}-pyridin-2-carbonsäure werden unter Argon in 5 ml absolutem Dimethylformamid gelöst, mit 56 mg (0,6 mMol) 3-Aminopyridin, 76 mg (0,75 mMol) N-Methylmorpholin und 136 mg (0,36 mMol) O-(7-Azabenzotriazol-1-yl)-1,1,3,3-tetramethyluroniumhexafluorophosphat (HATU) versetzt und 48 h bei Raumtemperatur gerührt. Anschliessend wird am Vakuum eingeengt und der Rückstand über eine Flashsäule (5 g Isolute flash silica, Fa. Sepostis) mit einem Gradienten von Methylenchlorid : Ethanol = 100 : 0 bis 95 : 5 als Elutionsmittel chromatographiert. Man erhält mg 5-{[2-(Isochinolin-3-ylcarbamoyl)-phenylamino]-methyl}-pyridin-2-carbonsäure pyridin-3-ylamid.

### MS (m/e 474)

In analoger Verfahrensweise werden hergestellt:

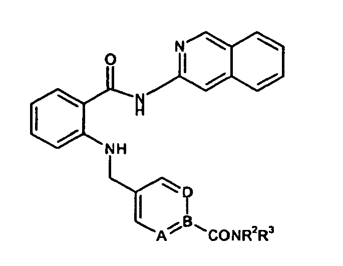

| **Beispiel Nr.** | **A** | **B** | **D** | **R²** | **R³** | **MW** | **Smp. [°C]/ MS Molpeak (m/e)** |
|---|---|---|---|---|---|---|---|
| 2.1 | C | C | N | 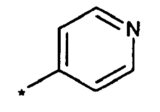 | H | 474,52 | 474 (m/e) |
| 2.2 | C | C | N | 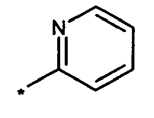 | H | 474,52 | 474 (m/e) |

### Beispiel 3.0

### Herstellung von 5-{[2-(2-Oxo-2,3-dihydro-1H-indol-5-ylcarbamoyl)-phenylamino]-methyl}-pyridin-2-carbonsäureamid

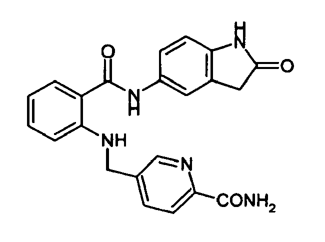

36 mg (0,09 mMol) 2-[(6-Cyano-pyridin-3-ylmethyl)-amino]-N-(2-oxo-2,3-dihydro-1*H*-indol-5-yl)-benzamid werden in 1 ml Dimethylsulfoxid mit 30 mg (0,22 mMol) Kaliumcarbonat und 0,05 ml (0,42 mMol) Wasserstoffperoxid (30 %ig) versetzt und 3,5 h bei Raumtemperatur gerührt. Es wird dann mit Wasser verdünnt und mit Essigester extrahiert. Die organische Phase wird gewaschen, getrocknet, filtriert und eingeengt. Der Rückstand wird mit warmem Methanol ausgerührt. Man erhält 5 mg (11 % der Theorie) 5-{[2-(2-Oxo-2,3-dihydro-1*H-*indol-5-ylcarbamoyl)-phenylamino]-methyl}-pyridin-2-carbonsäureamid.

In analoger Verfahrensweise werden hergestellt:

### Beispiel 3.1

### 4-{[2-(7-Methoxy-3-methyl-chinolin-2-ylcarbamoyl)-phenylamino]-methyl}-pyridin-2-carbonsäureamid

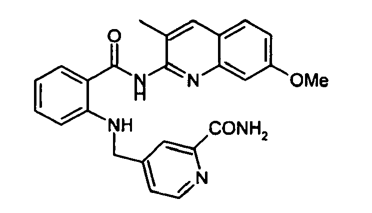

### Beispiel 3.2

### 4-{[2-(7-Methoxy-2-oxo-2H-chromen-3-ylcarbamoyl)-phenylamino]-methyl}-pyridin-2- carbonsäureamid

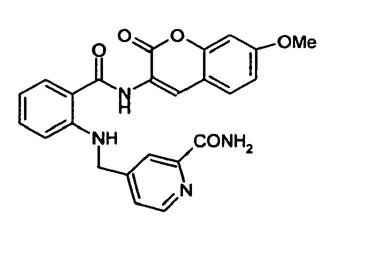

### Beispiel 3.3

### 5-{[2-(7-Methoxy-2-oxo-2H-chromen-3-ylcarbamoyl)-phenylamino]-methyl}-pyridin-2-carbonsäureamid

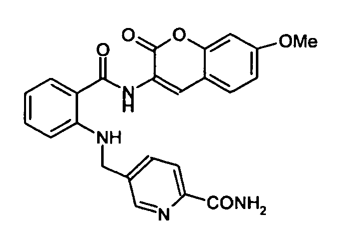

### Beispiel 3.4

### 5-{[2-(7-Methoxy-3-methyl-chinolin-2-ylcarbamoyl)-phenylamino]-methyl}-pyridin-2-carbonsäureamid

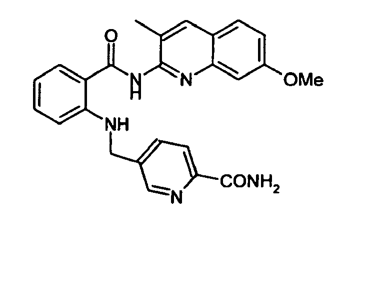

### Beispiel 3.5

### 5-{[2-(2-Oxo-2,3-dihydro-1H-indol-6-ylcarbamoyl)-phenylamino]-methyl}-pyridin-2-carbonsäureamid

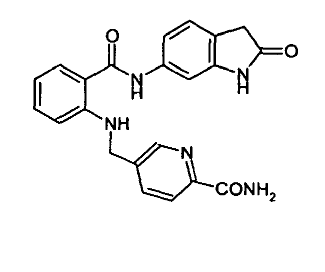

### Beispiel 3.6

### 4-{[2-(2-Methyl-2H-indazol-6-ylcarbamoyl)-phenylamino]-methyl}-pyridin-2-carbonsäureamid

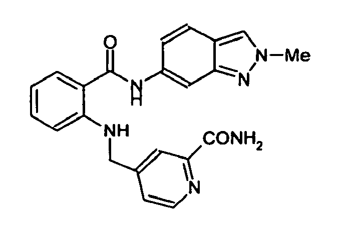

### Beispiel 3.7

### 4-{[2-(1H-Indazol-6-ylcarbamoyl)-phenylamino]-methyl}-pyridin-2-carbonsäureamid

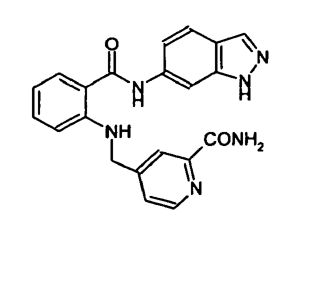

### Beispiel 3.8

### 4-{[2-(1-Methyl-1H-Indazol-6-ylcarbamoyl)-phenylamino]-methyl}-pyridin-2-carbonsäureamid

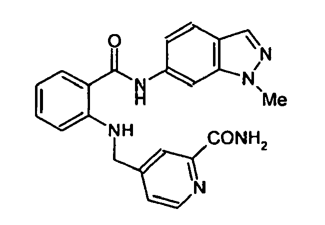

### Beispiel 4.0

### Herstellung von N-(Isochinolin-3-yl)-2-[3-(pyridin-3-yl)-pyridin-4-yl-methylamino]-benzoesäureamid

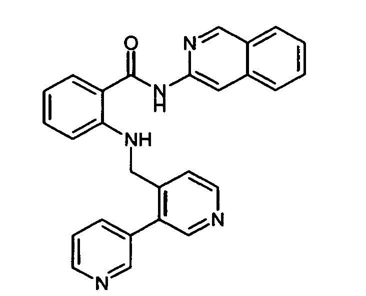

94 mg (0,22 mMol) N-(Isochinolin-3-yl)-2-[3-brompyridin-4-yl-methylamino]-benzoesäureamid werden in 3,7 ml Toluol nacheinander mit 0,73 ml Ethanol, 0,36 ml einer 2 molaren Natriumcarbonatlösung, 6 mg Palladium(o)tetrakistriphenylphosphin und 32 mg Pyridin-3-boronsäure versetzt und 6,5 Stunden auf 120°C Badtemperatur erwärmt. Es wird dann mit Wasser auf 25 ml verdünnt und dreimal mit je 25 ml Essigester extrahiert. Die gesammelte Essigesterphase wird getrocknet, filtriert und eingeengt. Der Rückstand wird über Kieselgel mit Methylenchlorid, Ethanol = 10 : 1 als Elutionsmittel chromatographiert. Man erhält 45 mg (47 % der Theorie) N-(Isochinolin-3-yl)-2-[3-(pyridin-3-yl)-pyridin-4-yl-methylamino]-benzoesäureamid als Harz.

1 H-NMR (d6-DMSO): 10.68 (s, 1H), 9.21 (s, 1H), 8.72 (s, 1 H), 8.64 (d, J=3.8, 1 H), 8.57 (d, J=5.1, 1 H), 8.54 (s, 1 H), 8.48 (s, 1 H), 8.11-7.94 (m, 4H), 7.85 (d, J=7.6, 1H), 7.74 (t, J=7.3, 1 H), 7.59-7.47 (m, 3H), 7.23 (t, J≈7.5, 1 H), 6.63 (t, J≈7.5, 1 H), 6.39 (d, J=8.3, 1 H), 4.45 (d, J=5.0, 2H).
MS (Cl-NH3): 432 (80%, [M+H]⁺)

### Beispiel 4.1

### Herstellung von N-(Isochinolin-3-yl)-2-[3-(thien-3-yl)-pyridin-4-yl-methylamino]-benzoesäureamid

In analoger Verfahrensweise wird auch N-(Isochinolin-3-yl)-2-[3-(thien-3-yl)-pyridin-4-yl-methylamino]-benzoesäureamid hergestellt: 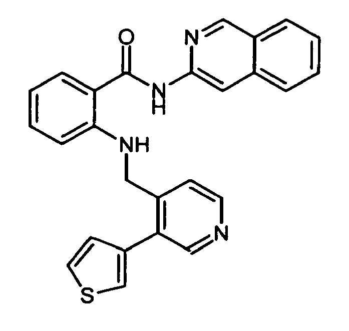 MS (Cl-NH3): 437 (100%, [M+H]⁺)

### Beispiel 4.2

### Herstellung von N-(Isochinolin-3-yl)-2-[2-aminocarbonylpyridin-4-yl-methylamino]-benzoesäureamid

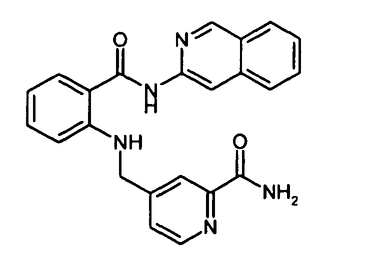

130 mg (0,34 mMol) N-(Isochinolin-3-yl)-2-[2-cyanopyridin-4-yl-methylamino]-benzoesäureamid werden in 2,5 ml Dimethylsulfoxid mit 126 mg Kaliumcarbonat und 0,25 ml Wasserstoffperoxid (30 %ig) versetzt und 1 Stunde bei Raumtemperatur gerührt. Es wird dann mit Wasser versetzt und das ausgefallene Produkt abgesaugt. Der Rückstand wird in einem Gemisch aus Methylenchlorid / Ethanol ausgerührt und abgesaugt. Man erhält 96 mg (71 % der Theorie) an N-(Isochinolin-3-yl)-2-[(2-aminocarbonylpyridin-4-yl)-methylamino]-benzoesäureamid vom Schmelzpunkt: 200°C.

1 H-NMR (d6-DMSO): 10.73 (s, 1 H), 9.22 (s, 1 H), 8.60 (s, 1 H), 8.56 (d, J=4.7, 1 H), 8.25 (br.s, 1 H), 8.10-8.04 (m, 3H), 7.95 (d, J=8.0, 1 H), 7.88 (d, J=6.9, 1 H), 7.74 (t, J≈7.4, 1 H), 7.63-7.57 (m, 3H), 7.25 (t, J=7.0, 1 H), 6.64 (t, J≈7.5, 1 H), 6.54 (d, J=8.4, 1 H), 4.62 (br.d, J=5.5, 2H).
MS (EI): 397 (38%, [M]⁺)

### Beispiel 4.3

### Herstellung von N-(Isochinolin-3-yl)-2-[(2-aminocarbonylpyridin-5₋yl)-methylamino]-benzoesäureamid

In analoger Verfahrensweise wird auch N-(lsochinolin-3-yl)-2-[(2-aminocarbonylpyridin-5-yl)-methylamino]-benzoesäureamid 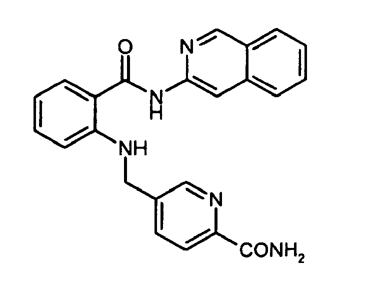 hergestellt.
MS (ESI): 398 (78%, [M+H]⁺)

### Beispiel 4.4

### Herstellung von N-(Isochinolin-3-yl)-2-[(2-methoxycarbonylpyridin-4-yl)-methylamino]-benzoesäureamid

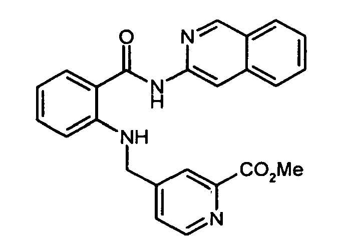

20 mg N-(Isochinolin-3-yl)-2-[2-brompyridin-4-yl-methylamino]-benzoesäureamid (0,05 mmol), 1,6 mg (0,003 mmol) Bis(diphenylphosphin)ferrocen (DPPF), 0,35 mg (0.0015 mmol) Palladium(II)acetat, 14 µl (0,1 mmol) Triethylamin werden in einem Gemisch aus 1 ml Methanol und 1 ml Dimethylformamid suspendiert und 5 Stunden im Autoclaven unter CO-Atmosphäre (3 bar) bei 50°C gerührt. Das Reaktionsgemisch wird über einen Membranfilter filtriert, eingeengt und über Kieselgel mit Hexan:EtOAc=3 :7 als Elutionsmittel chromatographiert. Man erhält 12 mg (58 % der Theorie) N-(Isochinolin-3-yl)-2-[(2-methoxycarbonylpyridin-4-yl)-methylamino]-benzoesäureamid.

1 H-NMR (CDCl3): 9.12 (br.s, 1 H), 8.94 (s, 1 H), 8.67 (s, 1 H), 8.61 (d, J=5.1, 1 H), 8.36 (br.s, 1 H), 8.09 (s, 1 H), 7.87 (d, J=8.5, 1 H), 7.81 (d, J=8.5, 1 H), 7.71 (d, J=7.7, 1 H), 7.64 (t, J≈7.8, 1 H), 7.49-7.44 (m, 2H), 7.24-7.19 (m, 1 H), 6.68 (t, J≈7.8, 1 H), 6.42 (d, J=8.0, 1 H), 4.50 (br.s, 2H), 3.93 (s, 3H).
MS(ESI) : 413 (100 %, [M+H]⁺)

### Beispiel 4.5

### Herstellung von N-(Isochinolin-3-yl)-2-[(2-benzyloxycarbonylpyridin-4-yl)-methylamino]-benzoesäureamid

In analoger Verfahrensweise wird auch N-(Isochinolin-3-yl)-2-[(2-benzyloxycarbonylpyridin-4-yl)-methylamino]-benzoesäureamid hergestellt.

1 H-NMR (CDCl3): 9.00 (s, 1 H), 8.76 (br.s, 1 H), 8.68 (d, J=5.0, 1 H), 8.66 (s, 1 H), 8.39 (t, J≈6.1, 1 H), 8.14 (s, 1 H), 7.91 (d, J=7.9, 1H), 7.85 (d, J=8.0, 1 H), 7.69-7.62 (m, 2H), 7.53-7.46 (m, 4H), 7.38-7.25 (m, 4H), 6.73 (t, J≈7.2, 1 H), 6.48 (d, J=7.8, 1 H), 5.44 (s, 2H), 4.56 (d, J=6.0, 2H).
MS (Cl-NH3): 489 (85%, [M+H]⁺)

### Beispiel 4.6

### Herstellung von N-(Isochinolin-3-yl)-2-[(2-hydroxycarbonylpyridin-4-yl)-methylamino]-benzoesäureamid

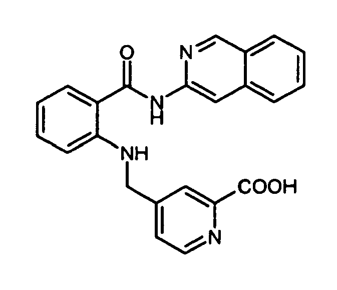

a. 20 mg N-(Isochinolin-3-yl)-2-[(2-methoxycarbonylpyridin-4-yl)-methylamino]-benzoesäureamid (0,05 mmol) werden in einem Gemisch aus 1 ml Tetrahydrofuran und 1 ml Methanol mit 10.2 mg (0,25mmol) Lithiumhydroxyd in Wasser versetzt und 4 Stunden bei 22°C gerührt. Das Reaktionsgemisch wird über einen Membranfilter filtriert, eingeengt und über Kieselgel mit Toluol:Essigsäure:Wasser 10:10:1 als Elutionsmittel chromatographiert. Man erhält 14 mg (69 % der Theorie) an N-(Isochinolin-3-yl)-2-[(2-hydroxy-carbonylpyridin-4-yl)-methylamino]-benzoesäureamid.
b. 433mg N-(Isochinolin-3-yl)-2-[2-brompyridin-4-yl)-methylamino]-benzoesäureamid (1 mmol), 50 mg (0.09 mmol) Bis(diphenylphosphin)ferrocen (DPPF), 10 mg (0.045 mmol) Palladium(II)acetat, 280 µl (2 mmol) Triethylamin werden in einem Gemisch aus 5ml Wasser und 10 ml Dimethylformamid suspendiert und 5 Stunden im Autoclaven unter CO-Atmosphäre (3 bar) bei 50°C gerührt. Das Reaktionsgemisch wird über einen Membranfilter filtriert, eingeengt, in Dichlormethan gelöst, mit Aktivkohle versetzt, erhitzt, filtriert und eingeengt. Der erhaltene Feststoff wird aus Dichlormethan umkristallisiert. Man erhält 283 mg (71 % der Theorie) an N-(Isochinolin-3-yl)-2-[(2-hydroxycarbonylpyridin-4-yl)-methylamino]-benzoesäureamid.

1 H-NMR (d6-DMSO): 10.73 (s, 1H), 9.22 (s, 1 H), 8.63 (d, J=4.9, 1 H), 8.60 (s, 1 H), 8.22 (br.t, J=6.0, 1 H), 8.10 (d, J=8.0, 1 H), 8.04 (s, 1 H), 7.94 (d, J=8.1, 1H), 7.87 (d, J=6.8, 1H), 7.74 (t, J=7.5, 1H), 7.60-7.54 (m, 2H), 7.25 (t, J=7.0, 1 H), 6.65 (t, J=7.6, 1H), 6.54 (d, J=8.4, 1H), 4.62 (br.d, J=5.5, 2H). Ein Proton wird nicht beobachtet oder ist verdeckt.
MS(CI-NH3) : 399 (75 %, [M+H]⁺)
Schmelzpunkt: 185 °C

### Beispiel 4.7

### Herstellung von N-(Isochinolin-3-yl)-2-[(2-morpholinocarbonylpyridin-4-yl)-methylamino]-benzoesäureamid

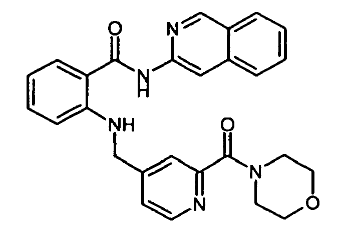

Eine Mischung aus 40 mg N-(lsochinolin-3-yl)-2-[(2-hydroxycarbonylpyridin-4-yl)-methylamino]-benzoesäureamid (0,1 mmol) und 9 µl (0,1 mmol) Morpholin in 1 ml Dimethylformamid wird portionsweise mit 34 mg (0,2 mmol) Carbonyldiimidazol versetzt. Nach 4 Stunden Rühren bei 22 °C wird eingeengt, der Rückstand in 5 ml Dichlormethan gelöst, mit 1 molarer wäßriger Kaliumcarbonatlösung (2 ml) gewaschen, getrocknet (MgSO₄), filtriert und eingeengt. Farbloses Harz (38 mg, 81% der Theorie).

1 H-NMR (CDCl3): 9.02 (s, 1 H), 8.71 (br.s, 1 H), 8.64 (s, 1 H), 8.51 (d, J=5.1, 1 H), 8.36 (t, J≈6.0, 1 H), 8.01 (s, 1 H), 7.93-7.82 (m, 2H), 7.69-7.64 (m, 2H), 7.50 (t, J≈7.8, 1 H), 7.39 (d, J=6.1, 1H), 7.28-7.20 (m, 1H), 6.73 (t, J≈7.8, 1H), 6.52 (d, J=8.1, 1 H), 4.55 (d, J=6.0, 2H), 3.79-3.62 (m, 8H).
MS(EI) : 467 (15 %, [M+H]⁺)

### Beispiel 4.8

### Herstellung von N-(Isochinolin-3-yl)-2-[3-trimethylsilylethinylpyridin-4-yl)-methylamino]-benzoesäureamid

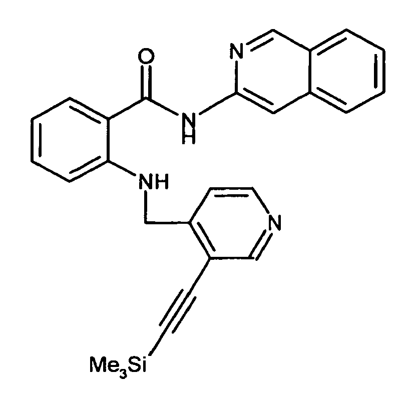

108 mg (0,25 mMol) N-(Isochinolin-3-yl)-2-[3-brompyridin-4-yl)-methylamino]-benzoesäureamid werden in 1ml Dimethylformamid mit 1ml Triethylamin, 5 mg (0,026 mMol) Kupfer-1-jodid, 9 mg (0,008 mMol) Palladiumtetrakistriphenylphosphin und 0,07 ml Trimethylsilylacetylen versetzt und unter Argon und Feuchtigkeitsausschluss 3,5 Stunden auf 70°C Badtemperatur erwärmt. Es wird dann mit 40 ml Waser versetzt und dreimal mit je 25 ml Essigester extrahiert. Die Essigesterphase wird mit Wasser gewaschen, getrocknet, filtriert und eingeengt. Der Rückstand wird über Kieselgel mit Essigester:Hexan=1:1 als Elutionsmittel chromatographiert. Man erhält 38 mg (33,6 % der Theorie) an N-(Isochinolin-3-yl)-2-[3-trimethylsilylethinylpyridin-4-yl)-methylamino]-benzoesäureamid als amorpher Feststoff.

1 H-NMR (d6-DMSO): 10.71 (s, 1 H), 9.22 (s, 1 H), 8.62 (s, 1 H), 8.58 (s, 1 H), 8.49 (d, J=4.9, 1H), 8.21 (br.t, J≈6.1, 1 H), 8.09 (d, J=8.2, 1H), 7.92 (d, J=8.0, 1H), 7.88 (d, J=7.9, 1 H), 7.74 (t, J=8.0, 1H), 7.57 (t, J=7.7, 1 H), 7.40 (d, J=5.1, 1 H), 7.28 (t, J=7.5, 1 H), 6.65 (t, J=7.7, 1 H), 6.54 (d, J=8.1, 1 H), 4.58 (d, J=6.0, 2H), 0.27 (s, 3H).
MS (EI): 450 (105%, [M]⁺)

### Beispiel 4.9

### Herstellung von N-(Isochinolin-3-yl)-2-[2-trimethylsilylethinylpyridin-4-yl)-methylamino]-benzoesäureamid

In analoger Verfahrensweise zu Beispiel 9 wird auch N-(Isochinolin-3-yl)-2-[2-trimethylsilylethinylpyridin-4-yl)-methylamino]-benzoesäureamid 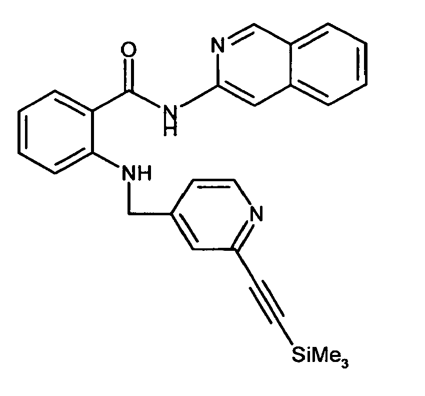 hergestellt.

### Herstellung der Ausgangs- und Zwischenverbindungen

Soweit die Herstellung der Zwischenverbindungen nicht beschrieben wird, sind diese bekannt oder analog zu bekannten Verbindungen oder hier beschriebenen Verfahren herstellbar.

### Beispiel A

### Verfahrensstufe 1

### A-1) Herstellung von 2-Brompyridin-5-carbaldehyd

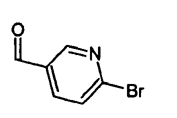

2-Brompyridin-5-carbaldehyd wid nach F.J.Romero-Salguerra et al.THL 40,859 (1999) hergestellt.

### A-2) Herstellung von 2-Brom-isonicotinsäure

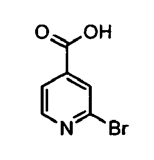

160 g (0,93 mol) 2-Brom-4-methyl-pyridin werden zu 152 g (0,96 mol) Kaliumpermanganat in 4 I Wasser zugetropft. Anschließend wird eine Stunde unter Rückfluss gerührt, bevor noch einmal 152 g (0,96 mol) Kaliumpermanganat zugegeben werden. Nach zwei weiteren Stunden des Nachrührens unter Rückfluss wird heiß über Celite abgesaugt und mit Wasser gewaschen. Die wässrige Phase wird dreimal mit Dichlormethan ausgeschüttelt. Die wässrige Phase wird auf die Hälfte eingeengt und mit konzentrierter Salzsäure auf pH 2 eingestellt. Der ausgefallene Feststoff wird abgesaugt und bei 70° C im Vakuum getrocknet. Es fallen 56,5 g (28 % der Theorie) 2-Brom-isonicotinsäure als weißes Festprodukt an.

### A-3) Herstellung von 2-Brom-4-hydroxymethyl-pyridin

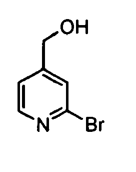

Zu 56,5 g (280 mmol) 2-Brom-isonicotinsäure in 1,2 I Tetrahydrofuran (THF) werden 30,2 ml (295 mmol) Triethylamin zugegeben. Anschließend wird auf - 10° C abgekühlt und tropfenweise mit 38,2 ml (295 mmol) Chlorameisensäureisobutylester versetzt. Nachdem eine Stunde bei -10° C nachgerührt worden ist, wird auf -70° C abgekühlt und tropfenweise mit 590 ml (590 mmol) Lithiumaluminiumhydrid (LiAIH₄)-Lösung (1M in THF) versetzt. Nach einer Stunde des Nachrührens bei -70° C lässt man auf -40° C kommen. Es werden 600 ml 50 %-ige Essigsäure zugegeben. Über Nacht wird bei Raumtemperatur gerührt. Die unlöslichen Bestandteile werden abgesaugt, und das Filtrat wird eingeengt. Der Rückstand wird über Kieselgel mit Hexan und Hexan/Essigester 1:1 gereinigt. Es fallen 28,0 g (55 % der Theorie) 2-Brom-4-hydroxymethyl-pyridin als weißes erstarrendes Öl an.

### A-4) Herstellung von 2-Brom-4-formyl-pyridin

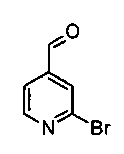

Zu 28,0 g (148,9 mmol) 2-Brom-4-hydroxymethyl-pyridin in 500 ml Dichlormethan werden 149 g (1714 mmol) Braunstein in 6 Stunden zudosiert. Anschließend wird 48 Stunden bei RT nachgerührt. Es wird über Celite abgesaugt und eingeengt. Es fallen 16,4 g (60 % der Theorie) 2-Brom-4-formylpyridin als erstarrendes weißes Öl an.

### Verfahrensstufe 2

### A-5) Herstellung von 2-[(6-Brom-pyridin-3-ylmethyl)-amino]-N-isochinolin-3-yl-benzamid

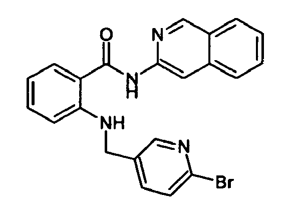

3,46 g (13,17 mMol) 2-Amino-*N*-isochinolin-3-yl-benzamid werden in 50 ml Methanol vorgelegt, mit 1,5 ml Eisessig sowie 2,45 g (13,17 mMol) 2-Brompyridin-5-carbaldehyd versetzt und für 24 h unter Argon und Feuchtigkeitsausschluss bei Raumtemperatur gerührt. Anschliessend wird mit 828 mg (13,17 mMol) Natriumcyanoborhydrid versetzt und weitere 24 h bei Raumtemperatur gerührt. Nach Einengen unter Vakuum wird der Rückstand in verdünnter Natriumhydrogenkarbonatlösung aufgenommen und abgesaugt. Der erhaltene Rückstand wird in wenig Essigester ausgerührt und nochmals abgesaugt. Der dabei erhaltene Rückstand wird über Kieselgel mit Hexan:Essigester=1:1 als Elutionsmittel chromatographiert. Man erhält 3,27 g (57 % der Theorie) 2-[(6-Bromo-pyridin-3-ylmethyl)-amino]-*N*-isochinolin-3-yl-benzamid.

### A-6) Herstellung von N-(Isochinolin-3-yl)-2-[3-brompyridin-4-yl-methylamino]-benzoesäureamid

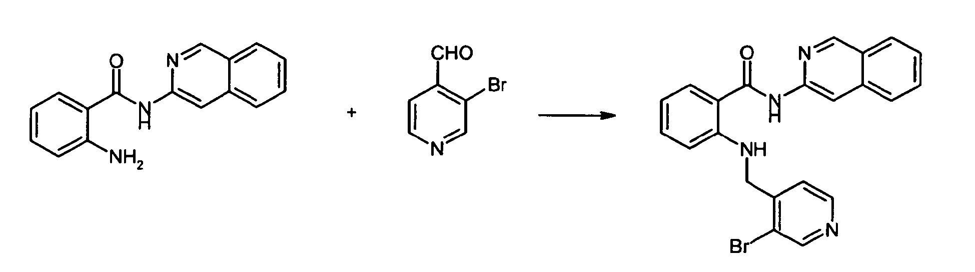

263 mg (1 mMol) N-(Isochinolin-3-yl)-2-aminobenzoesäureamid werden in 6 ml MeOH nacheinander mit 0,06 ml Eisessig, 298 mg (1,6 mMol) 3-Brom-pyridin-4-carbaldehyd (dargestellt nach Tetrahedron 2000, 347) versetzt und 24 Stunden bei Raumtemperatur gerührt. Anschließend werden 100 mg (1,6 mMol) Natriumcyanoborhydrid zugefügt und weitere 24 Stunden gerührt. Man versetzt dann mit 50 ml verdünnter Natriumhydrogencarbonatlösung und saugt das ausgefallene Produkt ab. Der Rückstand wird über Kieselgel mit Methylenchlorid : Ethanol = 95 : 5 als Elutionsmittel chromatographiert. Man erhält N-(Isochinolin-3-yl)-2-[3-brompyridin-4-yl-methylamino]-benzoesäureamid als Harz. 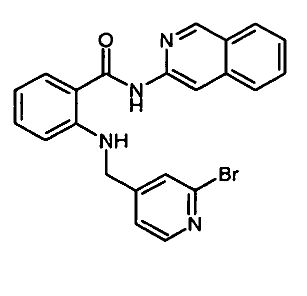 In analoger Verfahrensweise werden hergestellt:

### A-7) 2-[(2-Brom-pyridin-4-ylmethyl)-amino]-N-isochinolin-3-yl-benzamid

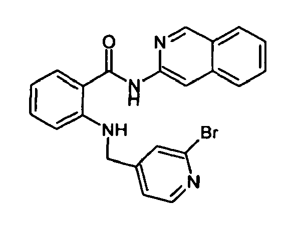

1 H-NMR (CDCl3): 9.00 (s, 1 H), 8.78 (s, 1 H), 8.66 (s, 1 H), 8.35 (t, J≈5.7, 1 H), 8.30 (d, J=5.1, 1 H), 7.92 (d, J=8.1, 1 H), 7.86 (d, J=8.5, 1 H), 7.70-7.65 (m, 2H), 7.53-7.48 (m, 2H), 7.33-7.26 (m, 2H), 6.75 (t, J=7.8, 1H), 6.48 (d, J=8.5, 1 H), 4.48 (d, J=5.9, 2H).
MS (Cl, NH3) : 435 (100 %), 433 (100 %)
Das eingesetzte 3-Brom-pyridin-4-carbaldehyd wird nach Chem. Pharm. Bull. 1970, 38, 2446 hergestellt.

### A-8) 2-[(2-Brom-pyridin-4-ylmethyl)-amino]-N-(2-oxo-2,3-dihydro-1H-indol-6-yl)-benzamid

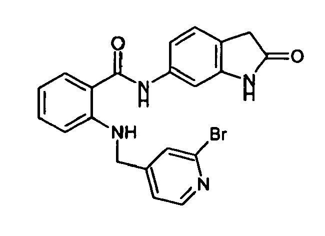

### A-9) 2-[(2-Brom-pyridin-4-ylmethyl)-amino]-N-(2-oxo-2,3-dihydro-1H-indol-5-yl)-benzamid

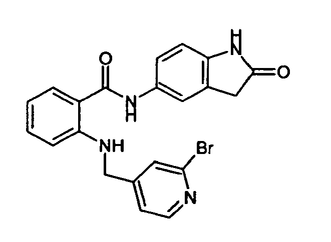

### A-10) 2-[(6-Brom-pyridin-3-ylmethyl)-amino]-N-(2-oxo-2,3-dihydro-1H-indol-6-yl)-benzamid

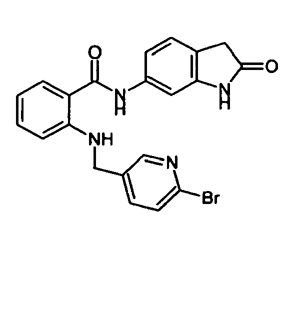

### A-11) 2-[(6-Brom-pyridin-3-ylmethyl)-amino]-N-(2-oxo-2,3-dihydro-1H-indol-5-yl)-benzamid

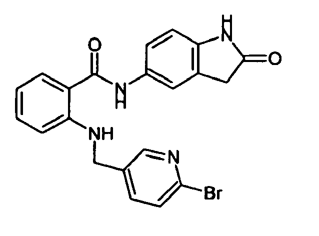

### A-12) 2-[(6-Brom-pyridin-3-ylmethyl)-amino]-N-(7-methoxy-2-oxo-2H-chromen-3-yl)-benzamid

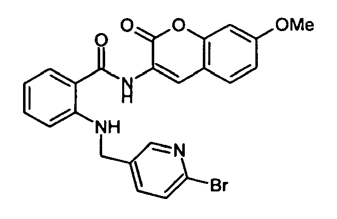

### A-13) 2-[(2-Brom-pyridin-4-ylmethyl)-amino]-N-(7-methoxy-2-oxo-2H-chromen-3-yl)-benzamid

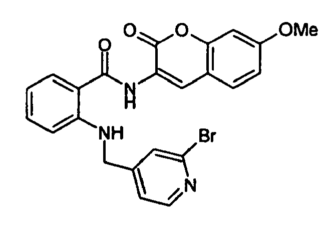

### A-14a) 2-[(2-Brom-pyridin-4-ylmethyl)-amino]-N-(3-trifluoromethylphenyl)-benzamid

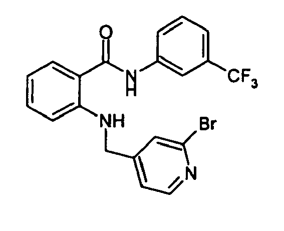

### A-14b) 2-[(6-Brom-pyridin-3-ylmethyl)-amino]-N-(7-methoxy-3-methylchinolin-2-yl)-benzamid

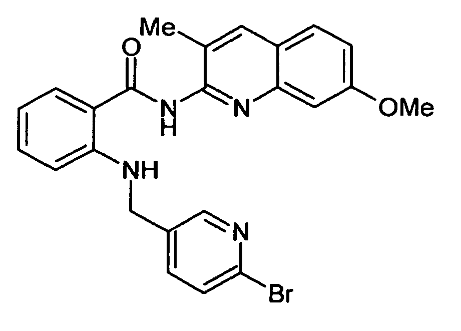

### Verfahrensstufe 3

### A-15) Herstellung von 5-{[2-(Isochinolin-3-ylcarbamoyl)-phenylamino]-methyl}-pyridin-2-carbonsäure

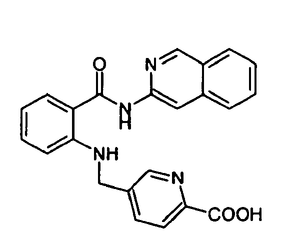

3,27 g (7,55 mMol) 2-[(6-Bromo-pyridin-3-ylmethyl)-amino]-*N*-isochinolin-3-yl-benzamid werden in 75 ml Dimethylformamid mit 2,2 ml Triethylamin, 36 ml Wasser, 362 mg (0,65 mMol) Bisdiphenylphosphinoferrocen und 75 mg (0,33 mMol) Palladium(II)acetat versetzt und im Autoklaven unter Kohlenmonoxid bei einem Druck von 3 Bar und einer Temperatur von 50°C 3 h geschüttelt. Nach Erkalten wird über Kieselgur abgesaugt und eingeengt. Der Rückstand wird in Wasser aufgenommen, mit Eisessig auf pH 5-6 eingestellt, abgesaugt und der Filterkuchen mit Hexan nachgewaschen. Man erhält 3,35 g 5-{[2-(Isochinolin-3-ylcarbamoyl)-phenylamino]-methyl}-pyridin-2-carbonsäure , die ohne weitere Reinigung weiter umgesetzt werden.

In analoger Verfahrensweise werden hergestellt:

### A-16) 4-{[2-(Isochinolin-3-ylcarbamoyl)-phenylamino]-methyl}-pyridin-2-carbonsäure

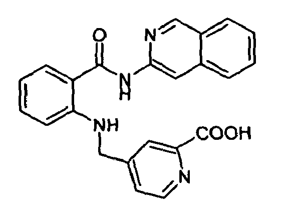

### A-17) 4-{[2-(2-Oxo-2,3-dihydro-1H-indol-6-ylcarbamoyl)-phenylamino]-methyl}-pyridin-2-carbonsäure

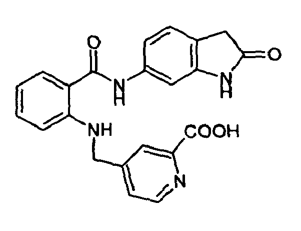

### A-18) 5-{[2-(2-Oxo-2,3-dihydro-1H-indol-6-ylcarbamoyl)-phenylamino]-methyl}-pyridin-2-carbonsäure

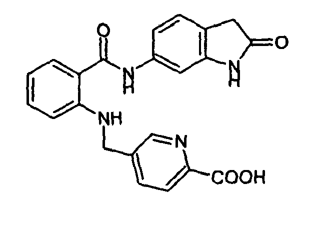

### A-19) 4-{[2-(2-Oxo-2,3-dihydro-1H-indol-5-ylcarbamoyl)-phenylamino]-methyl}-pyridin-2-carbonsäure

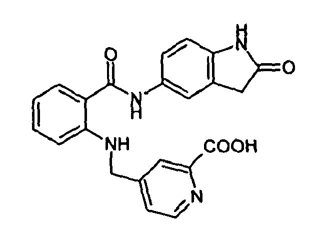

### A-20) 5-{[2-(2-Oxo-2,3-dihydro-1H-indol-5-ylcarbamoyl)-phenylamino]-methyl}-pyridin-2-carbonsäure

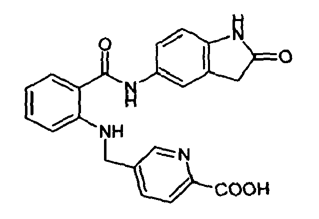

### A-21) 5-{[2-(7-Methoxy-2-oxo-2H-chromen-3-ylcarbamoyl)-phenylamino]-methyl}-pyridin-2-carbonsäure

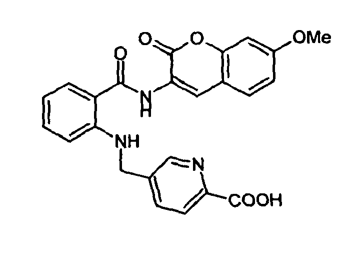

### A-22) 4-{[2-(7-Methoxy-2-oxo-2H-chromen-3-ylcarbamoyl)-phenylamino]-methyl}-pyridin-2-carbonsäure

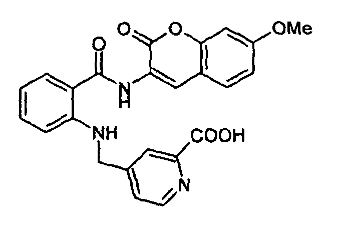

### A-23) 4-{[2-(7-Methoxy-3-methyl-chinolin-2-ylcarbamoyl)-phenylamino]-methyl}-pyridin-2-carbonsäure

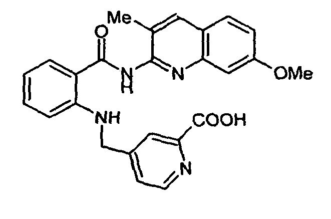

### A-24) 5-{[2-(7-Methoxy-3-methyl-chinolin-2-ylcarbamoyl)-phenylamino]-methyl}-pyridin-2-carbonsäure

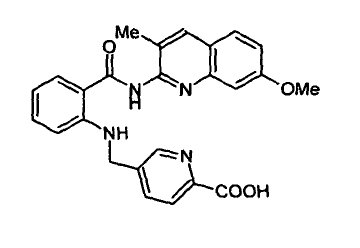

### A-25) 5-{[2-(1-Methyl-1H-indazol-6-ylcarbamoyl)-phenylamino]-methyl}-pyridin-2-carbonsäure

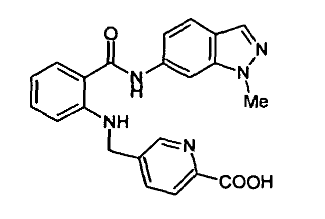

### A-26) 4-{[2-(1-Methyl-1H-indazol-6-ylcarbamoyl)-phenylamino]-methyl}-pyridin-2-carbonsäure

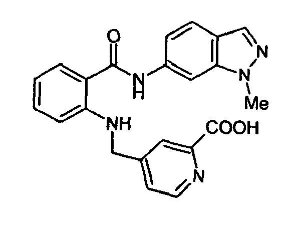

### A-27) 4-{[2-(2-Methyl-2H-indazol-6-ylcarbamoyl)-phenylamino]-methyl}-pyridin-2-carbonsäure

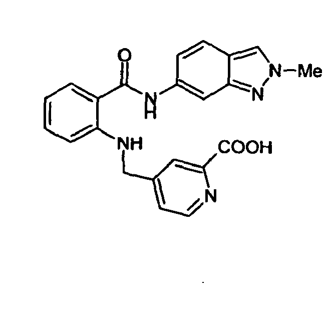

### A-28) 5-{[2-(2-Methyl-2H-indazol-6-ylcarbamoyl)-phenylamino]-methyl}-pyridin-2-carbonsäure

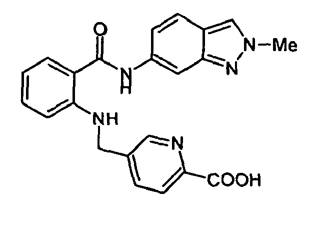

### A-29) 4-{[2-(3-Trifluoromethyl-phenylcarbamoyl)-phenylamino]-methyl}-pyridin-2-carbonsäure

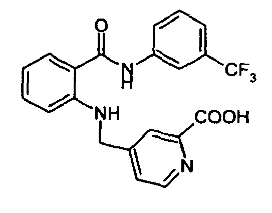

Schmelzpunkt 151 °C

### A-30) 4-{[2-(1H-Indazol-6-ylcarbamoyl)-phenylamino]methyl}-pyridin-2-carbonsäure

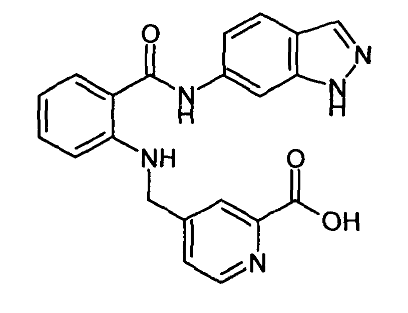

### A-31) 4-{[2-(1H-Indazol-5-ylcarbamoyl)-phenylamino]methyl}-pyridin-2 carbonsäure

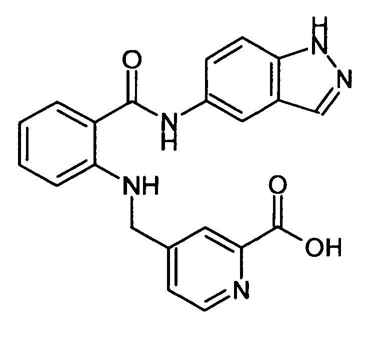

### Beispiel B

### Verfahrensstufe 1

### B-1) Herstellung von 5-Nitro-1,3-dihydro-indol-2-on

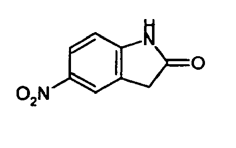

5-Nitro-1,3-dihydro-indol-2-on wird nach R.T. Courts, J.Org.Chem. 48, 3747,(1970) hergestellt.

### B-2) Herstellung von Dinitrophenylessigsäuremethylester.

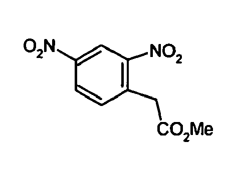

22,6 g (100 mMol) 2,4-Dinitrophenylessigsäure werden in einer Mischung von 200 ml Methanol und 830 ml Toluol gelöst und bei Raumtemperatur mit 83 ml Trimethylsilyldiazomethan (2 molar in Toluol; 166 mMol) versetzt und 3 h bei Raumtemperatur gerührt. Nach Einengen zur Trockene und Trocknen bei 70°C am Vakuum erhält man 24 g (100 % der Theorie) an 2,4-Dinitrophenylessigsäuremethylester.

### B-3) Herstellung von 6-Nitro-1,3-dihydro-indol-2-on

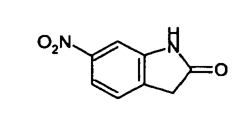

20 g (83 mMol) an 2,4-Dinitrophenylessigsäuremethylester werden in 400 ml Eisessig mit 2,1 g Palladium/ Kohle (10%) unter 20 bar Wasserstoff 1,5 h bei Raumtemperatur hydriert. Nach Abfiltrieren vom Katalysator wird eingeengt und scharf über festem Kaliumhydroxid im Vakuum getrocknet. Der Rückstand wird über Kieselgel mit einem Gradienten aus Methylenchlorid:Ethanol = 97,5 : 2,5 bis 90 : 10 als Elutionsmittel chromatographiert. Nach Umkristallisation aus Essigester erhält man 4 g (30 % der Theorie) 6-Nitro-1,3-dihydro-indol-2-on vom Schmelzpunkt 206 °C.

### Verfahrensstufe 2

### B-4) Herstellung von 5-Amino-1,3-dihydro-indol-2-on

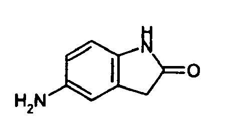

356 mg 5-Nitro-1,3-dihydro-indol-2-on werden in 30 ml Tetrahydrofuran : Ethanol = 1 : 1 mit 400 mg Palladium auf Kohle (10 %) bei Raumtemperatur und Normaldruck 1 h hydriert. Nach Absaugen vom Katalysator über Kieselgur und Einengen erhält man 320 mg (100 % der Theorie) an 5-Amino-1,3-dihydro-indol-2-on.

### B-5) Herstellung von 6-Amino-1,3-dihydro-indol-2-on

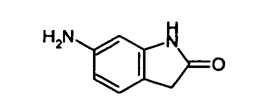

In analoger Weise wird aus der entsprechenden Nitroverbindung 6-Amino-1,3-dihydro-indol-2-on hergestellt.

### Verfahrensstufe 3

### B-6) 2-Nitro-N-(2-oxo-2,3-dihydro-1H-indol-5-yl)-benzamid

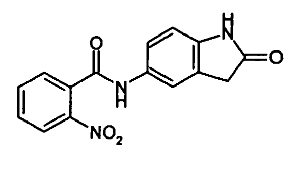

In 1 ml Dimethylacetamid werden 320 mg 5-Amino-1,3-dihydro-indol-2-on gelöst und tropfenweise mit 371 mg (2 mMol) 2-Nitrobenzoylchlorid versetzt, wobei eine leichte Erwärmung eintritt. Nach Rühren über Nacht bei Raumtemperatur wird im Vakuum eingeengt und der Rückstand in Essigester und Wasser aufgenommen. Das Absaugen eines unlöslichen Feststoffs gibt 130 mg (21,9 % der Theorie) 2-Nitro-*N*-(2-oxo-2,3-dihydro-1*H*-indol-5-yl)-benzamid. Nach Ausschütteln wird die organische Phase gewaschen, filtriert und eingeengt und man erhält nochmals 400 mg (67 % der Theorie) 2-Nitro-*N*-(2-oxo-2,3-dihydro-1*H*-indol-5-yl)-benzamid vom Schmelzpunkt 265 °C.

### B-7) Herstellung von 2-Nitro-N-(2-oxo-2,3-dihydro-1H-indol-6-yl)-benzamid

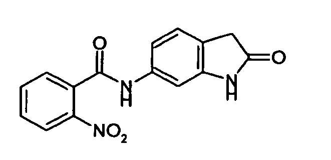

In analoger Verfahrensweise zu I) wird 2-Nitro-*N*-(2-oxo-2,3-dihydro-1*H*-indol-6-yl)-benzamid vom Schmelzpunkt >300°C hergestellt.

### Verfahrensstufe 4

### B-8) Herstellung von 2-Amino-N-(indol-2-on-5-yl)benzoesäureamid

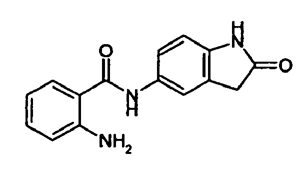

In analoger Verfahrensweise zur Verfahrensstufe 2 wird auch 2-Amino-N-(indol-2-on-5-yl)benzoesäureamid vom Schmelzpunkt 219 °C hergestellt.

### B-9) Herstellung von 2-Amino-N-(indol-2-on-6-yl)benzoesäureamid

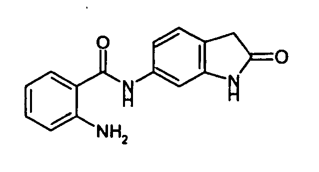

In analoger Verfahrensweise zur Stufe 2 wird auch 2-Amino-N-(indol-2-on-6-yl)benzoesäureamid vom Schmelzpunkt 230 °C hergestellt.

### Beispiel C

### C-1) Herstellung von 2-Amino-N-(7-methoxy-2-oxo-2H-chromen-3-yl)-benzamid

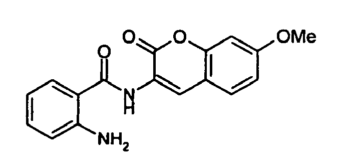

### Verfahrensstufe 1

### C-2) Herstellung von 3-Nitro-7-methoxy-chromen-2-on

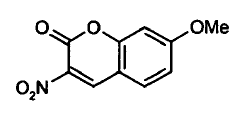

13 g (85,4 mMol) 2-Hydroxy-4-methoxybenzaldehyd werden in 300 ml Toluol mit 9,8 g (102,5 mMol) n-Propylaminhydrochlorid und 11,5 ml (102,5 mMol) Nitroessigsäureethylester 15 h am Wasserabscheider erhitzt. Es werden dann nochmals 3 ml Nitroessigsäureethylester zugegeben und weitere 5 h am Wasserabscheider gekocht. Nach dem Abkühlen wird mit Essigester verdünnt und mit Wasser ausgeschüttelt. Die Essigesterphase wird getrocknet, filtriert und eingeengt. Der Rückstand wird über Kieselgel mit Methylenchlorid als Elutionsmittel chromatographiert. Man erhält 6,14 g (33 % der Theorie) 3-Nitro-7-methoxy-chromen-2-on.

### Verfahrensstufe 2

### C-3) Herstellung von 3-Amino-7-methoxy-chromen-2-on

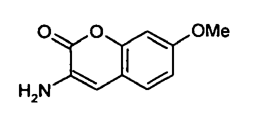

In analoger Weise zu Verfahrensstufe 2 aus Beispiel B wird aus 3-Nitro-7-methoxy-chromen-2-on in Ethanol 3-Amino-7-methoxy-chromen-2-on hergestellt.

### Verfahrensstufe 3

### C-4) Herstellung von 2-Nitro-N-(7-methoxybenzopyran-2-on-3-yl)benzoesäureamid

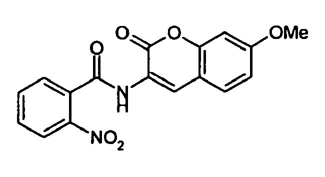

In Analogie zu Verfahrensstufe 3 aus Beispiel B wird aus 2-Nitrobenzoylchlorid und 3-Amino-7-methoxy-chromen-2-on 2-Nitro-*N*-(7-methoxy-2-oxo-2*H-*chromen-3-yl)-benzamid das 2-Nitro-N-(7-methoxybenzopyran-2-on-3-yl)benzoesäureamid hergestellt.

### Verfahrensstufe 4

### C-5) Herstellung von 2-Amino-N-(7-methoxy-2-oxo-2H-chromen-3-yl)-benzamid

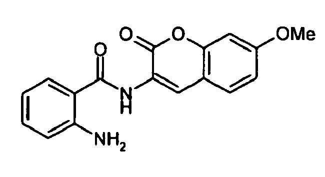

In Analogie zu Verfahrensstufe 2 aus Beispiel B wird aus 2-Nitro-*N*-(7-methoxy-2-oxo-2*H*-chromen-3-yl)-benzamid in Ethanol : Tetrahydrofuran = 5 : 2 das 2-Amino-*N*-(7-methoxy-2-oxo-2*H*-chromen-3-yl)-benzamid hergestellt.

### Beispiel D

### D-1) Herstellung von 2-[(6-Cyano-pyridin-3-ylmethyl)-amino]-N-(2-oxo-2,3-dihydro-1H-indol-5-yl)-benzamid

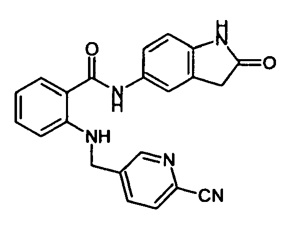

219 mg (0,5 mMol) 2-[(6-Bromo-pyridin-3-ylmethyl)-amino]-*N*-(2-oxo-2,3-dihydro-1*H*-indol-5-yl)-benzamid werden in 7 ml Dimethylacetamid mit 59 mg (0,5 mMol) Zink(II)cyanid, 12 mg (0,013 mMol) Tris(dibenzylidenaceton)-dipalladium, 10 mg (0,018 mMol) Bis(diphenylphosphino)ferrocen und 4 mg (0,06 mMol) Zinkpulver gegeben und unter Argon und Feuchtigkeitsausschluss 7,5 h bei 150°C Badtemperatur gerührt. Nach Abkühlen wird mit Wasser verdünnt, mit Essigester ausgeschüttelt und die organische Phase getrocknet, filtriert und eingeengt. Der Rückstand wird über Kieselgel mit einem Gradienten von Methylenchlorid : Ethanol = 97,5 : 2,5 bis 90 : 10 als Elutionsmittel chromatographiert. Man erhält 65 mg (30 % der Theorie) 2-[(6-Cyano-pyridin-3-ylmethyl)-amino]-*N*-(2-oxo-2,3-dihydro-1*H*-indol-5-yl)-benzamid.

In analoger Verfahrensweise werden hergestellt:

### D-2) 2-[(6-Cyano-pyridin-3-ylmethyl)-amino]-N-(7-methoxy-2-oxo-2H-chromen-3-yl)-benzamid

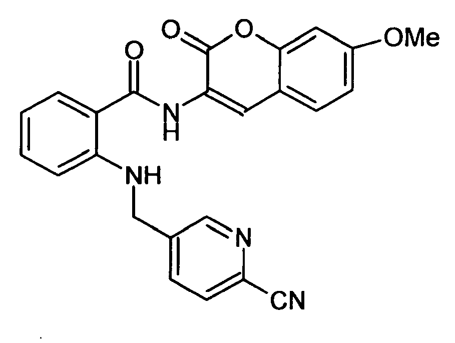

### Beispiel E

### E-1) N-(Isochinolin-3-yl)-2-[2-cyanopyridin-4-yl-methylamino]-benzoesäureamid

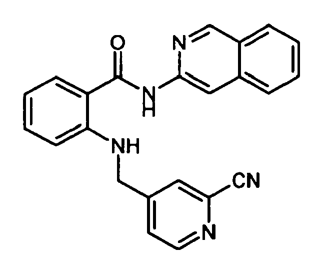

920 mg (2,5 mMol) N-(Isochinolin-3-yl)-2-(4-pyridylmethyl)-aminobenzoesäureamid-N-oxid werden in einem Glasdruckgefäss nacheinander mit 20 ml Dimethylformamid nacheinander mit 760 mg (7,5 mMol) Triethylamin und 1,24 g (12,5 mMol) Trimethylsilylcyanid versetzt und dann für 10 Stunden auf 110°C Badtemperatur erwärmt. Es wird dann mit Wasser auf ca 200 ml verdünnt und dreimal mit je 50 ml Essigester ausgeschüttelt. Die gesammelte organische Phase wird mit 50 ml Wasser gewaschen, getrocknet, filtriert und eingeengt. Der Rückstand wird zunächst über Kieselgel mit Essigester:Hexan=1:1 und anschliessend nochmals über Kieselgel mit Dichlormethan:Ethanol=100:2 als Elutionsmittel chromatographiert. Man erhält 132 mg (14% der Theorie) an N-(Isochinolin-3-yl)-2-(4-2-cyanopyridylmethyl)amino-benzoesäureamid als Harz

Soweit die Herstellung der Zwischenverbindungen nicht beschrieben wird, sind diese bekannt oder analog zu bekannten Verbindungen oder hier beschriebenen Verfahren herstellbar.

### Beispiel F

### 1. Verfahrensstufe

### F-1) Herstellung von 2-[(2-Bromo-pyridin-4-ylmethyl)-amino]-benzoesäure-methylester

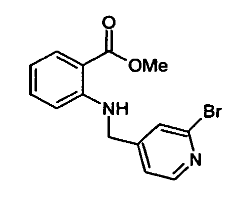

6,04 g (40 mmol) Anthranilsäuremethylester in 600 ml Methanol werden mit 3,2 ml Essigsäure und 7,4 g (40 mmol) 2-Brompyridin-4-carbaldehyd versetzt und bei 40° C über Nacht gerührt. Hierauf werden 3,8 g (60 mmol) Natriumcyanoborhydrid zugefügt und bei 40° C über Nacht gerührt. Es werden nochmals 3,8 g (60 mmol) Natriumcyanoborhydrid zugegeben und übers Wochenende bei 40 °C gerührt. Es wird mit Wasser versetzt und weitgehend eingeengt. Die wässrige Phase wird mit Essigester extrahiert, die vereinigten organischen Phasen werden getrocknet, filtriert und eingeengt. Das Rohprodukt wird über Kieselgel mit einem Gradienten aus Hexan und Hexan/ Essigester 1 : 3 und Hexan/ Essigester 1 : 1 als Elutionsmittel chromatographiert. Man erhält 10,0 g (78% der Theorie) an 2-[(2-Bromo-pyridin-4-ylmethyl)-amino]-benzoesäuremethylester als farbloses Öl.

### 2. Verfahrensstufe

### F-2) Herstellung von 2-[(2-Cyano-pyridin-4-ylmethyl)-amino]-benzoesäuremethylester

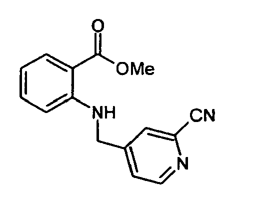

1,28 g (4,0 mmol) 2-[(2-Bromo-pyridin-4-ylmethyl)-amino]-benzoesäuremethylester in 140 ml Dimethylacetamid werden mit 0,532 g (4,56 mmol) Zink(II)cyanid, 0,072 g (0,08 mmol) Tris-(dibenzylidenaceton)-dipalladium, 0,088 g (0,16 mmol) Bis-(diphenylphosphino)-ferrocen und 0,029 g (0,46 mmol) Zinkpulver versetzt. Es wird 6 Stunden bei 150° C gerührt. Nach dem Abkühlen wird das Reaktionsgemisch in Wasser gegossen. Es wird dreimal mit Essigester extrahiert; die vereinigten organischen Phasen werden über Natriumsulfat getrocknet und eingeengt. Das Reaktionsprodukt wird über Kieselgel mit einem Gradienten aus Hexan:Essigester=100:0 bis 50:50 als Elutionsmittel chromatographiert. Man erhält 0,887 g (83% d.Th.) 2-[(2-Cyanopyridin-4-ylmethyl)-amino]-benzoesäuremethylester in Form eines gelben Feststoffes.

### 3. Verfahrensstufe

### F-3) 2-[(2-Cyano-pyridin-4-ylmethyl)-amino]-N-(7-methoxy-3-methylchinolin-2-yl)-benzamid

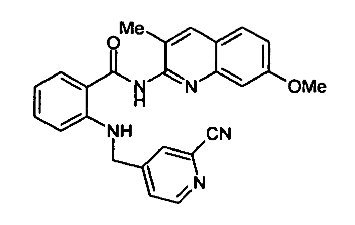

Bei 0° C werden 0,25 ml Trimethylaluminium (2 M in Toluol) zu 0,094 g (0,5 mmol) 7-Methoxy-3-methyl-chinolin-2-ylamine in 4 ml Toluol zugetropft. Nach 10 Minuten des Nachrührens bei 0° C werden 0,133 g (0,5 mmol) 2-[(2-Cyanopyridin-4-ylmethyl)-amino]-benzoesäuremethylester in 2 ml Toluol zugetropft. Anschließend wird 2 Stunden unter Rückfluss und über Nacht bei RT gerührt. Der Niederschlag wird abgesaugt und in gesättigter Natriumhydrogencarbonatlösung suspendiert. Anschließend wird Ethylendiamintetraessigsäure zugegeben. Es wird mit Essigester ausgeschüttelt, über Natriumsulfat getrocknet und eingeengt. Säulenchromatographische Reinigung an Kieselgel mit einem Gradienten Hexan : Aceton = 100 : 0 bis 50 : 50 als Elutionsmittel ergibt 0.113 g (54 % der Theorie) an 2-[(2-Cyano-pyridin-4-ylmethyl)-amino]-*N*-(7-methoxy-3-methylchinolin-2-yl)-benzamid als gelben Schaum.

### Beispiel G

### 1. Verfahrensstufe

### G-1) Herstellung von 2-[(2-Bromo-pyridin-4-ylmethyl)-amino]-benzoesäure

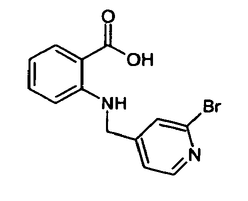

10,0 g (31,2 mmol) 2-[(2-Bromo-pyridin-4-ylmethyl)-amino]-benzoesäuremethylester werden in 290 ml Ethanol gelöst und mit 31,2 ml 2 M Natronlauge versetzt. Nachdem über Nacht bei Raumtemperatur gerührt worden ist, wird das Ethanol abgezogen, und die wässrige Phase wird mit Essigester ausgeschüttelt. Die wässrige Phase wird mit konzentrierter Salzsäure angesäuert. Der gebildete Niederschlag wird abgesaugt und getrocknet. Es fallen 5,93 g (62 %) 2-[(2-Bromo-pyridin-4-ylmethyl)-amino]-benzoesäure in Form eines weißen Feststoffes an.

### 2. Verfahrensstufe

### G-2) Herstellung von 2-[(2-Bromo-pyridin-4-ylmethyl)-amino]-N-(2-methyl-2H-indazol-6-yl)-benzamid

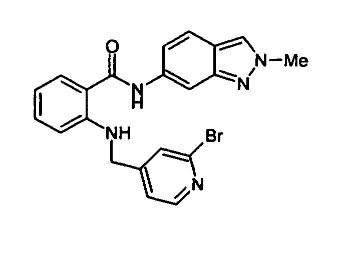

0,500 g (1,6 mmol) 2-[(2-Bromo-pyridin-4-ylmethyl)-amino]-benzoesäure, 0,471 g (3,2 mmol) 2-Methyl-2*H*-indazol-6-ylamin, 0,4 ml (3,68 mmol) N-Methylmorpholin und 0,729 g (1,92 mmol) O-(7-Azabenzotriazol-1-yl)-1,1,3,3-tetramethyluroniumhexafluorophosphat (HATU) in 25 ml Dimethylformamid werden 16 Stunden bei Raumtemperatur gerührt. Das Dimethylformamid wird im Ölpumpenvakuum abgezogen. Der verbleibende Rückstand wird in gesättigter Natriumhydrogencarbonatlösung aufgenommen. Es wird dreimal mit Essigester extrahiert, und die vereinigten organischen Phasen werden getrocknet, filtriert und eingeengt. Der Rückstand wird über Kieselgel mit einem Gradienten aus Hexan : Aceton = 100 : 0 bis 50 : 50 als Elutionsmittel chromatographiert. Man erhält 0,669 g (96 % der Theorie) 2-[(2-Bromo-pyridin-4-ylmethyl)-amino]-*N*-(2-methyl-2*H*-indazol-6-yl)-benzamid in Form eines beigen Schaums.

In analoger Verfahrensweise werden hergestellt:

### G-3) 2-[(2-Bromo-pyridin-4-ylmethyl)-amino]-N-(1-methyl-1H-indazol-6-yl)-benzamid

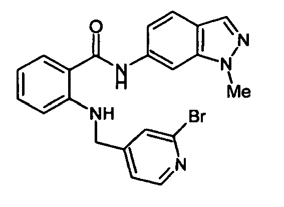

### G-4) 2-[(2-Bromo-pyridin-4-ylmethyl)-amino]-N-(1H-indazol-6-yl)-benzamid

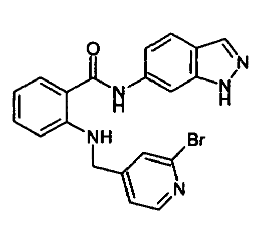

### G-5) 2-[(2-Bromo-pyridin-4-ylmethyl)-amino]-N-(1H-indazol-5-yl)-benzamid

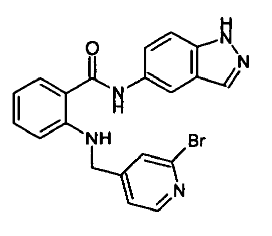

### G-6) 2-[(2-Bromo-pyridin-4-ylmethyl)-amino]-N-(2-oxo-2,3-dihydro-1H-indol-6-yl)-benzamid

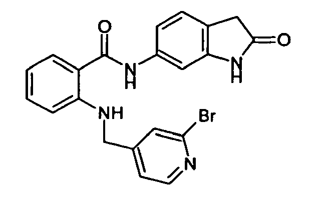

### G-7) 2-[(2-Bromo-pyridin-4-ylmethyl)-amino]-N-(2-oxo-2,3-dihydro-1H-indol-5-yl)-benzamid

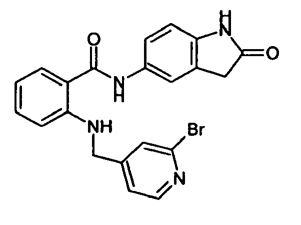

Die nachfolgenden Anwendungsbeispiele erläutern die biologische Wirkung und Verwendung der erfindungsgemäßen Verbindungen ohne diese auf die Beispiele zu beschränken.

### Für die Versuche benötigte Lösungen

Stammlösungen
Stammlösung A: 3 mM ATP in Wasser pH 7,0 (-70 °C)
Stammlösung B: g-33 P-ATP 1mCi/ 100µl
Stammlösung C: poly-(Glu4Tyr) 10mg/ ml in Wasser

Lösung für Verdünnungen
- Substratlösemittel:: 10mM DTT, 10 mM Manganchlorid, 100 mM Magnesiumchlorid
- Enzymlösung:: 120 mM Tris/ HCl, pH 7,5, 10 µM Natriumvanadiumoxid

### Anwendungsbeispiel 1

### Hemmung der KDR- und FLT-1 Kinaseaktivität in Gegenwart der erfindungsgemäßen Verbindungen

In einer spitz zulaufenden Mikrotiterplatte (ohne Proteinbindung) werden 10 µl Substratmix (10µl Vol ATP Stammlösung A + 25µCi g-33P-ATP (ca. 2,5 µl der Stammlösung B) + 30µl poly-(Glu4Tyr) Stammlösung C + 1,21ml Substratlösemittel), 10 µl Hemmstofflösung (Substanzen entsprechend den Verdünnungen, als Kontrolle 3% DMSO in Substratlösemittel) und 10 µl Enzymlösung (11,25µg Enzymstammlösung (KDR oder FLT-1 Kinase) werden bei 4°C in 1,25ml Enzymlösung verdünnt) gegeben. Es wird gründlich durchgemischt und 10 Minuten bei Raumtemperatur inkubiert. Anschließend gibt man 10µl Stop-Lösung (250mM EDTA, pH 7,0) zu, mischt und überträgt 10 µl der Lösung auf einen P 81 Phosphozellulosefilter. Anschließend wird mehrfach in 0,1 M Phosphorsäure gewaschen. Das Filterpapier wird getrocknet, mit Meltilex beschichtet und im Microbetazähler gemessen. Die IC50-Werte bestimmen sich aus der Inhibitorkonzentration, die notwendig ist, um den Phosphateinbau auf 50% des ungehemmten Einbaus nach Abzug des Leerwertes (EDTA gestoppte Reaktion) zu hemmen.

Die Ergebnisse der Kinase-Inhibition IC50 in nM sind in der nachfolgenden Tabelle dargestellt:

| **Beispiel Nr.** | VEGFR II (KDR) **[nM]** |
|---|---|
| 1.32 | 40 |

### Anwendungsbeispiel 2

### Cytochrom P450 - Inhibition

Die Cytochrom P450 - Inhibition wurde entsprechend der Veröffentlichung von Crespi et al. (Anal. Biochem., 248, 188-190 (1997)) unter Verwendung von Baculovirus/ lnsektenzellen-exprimierten, humanen Cytochrom P 450 Isoenzymen (1A2, 2C9, 2C19, 3A4) durchgeführt.

Die Ergebnisse sind in der folgenden Tabelle dargestellt.
Hemmung der Cytochrom P450 Isoenzyme (IC50, µM)

| Cytochrom P450 Isoenzym | 1A2 | 2C9 | 2C19 | 3A4 |
|---|---|---|---|---|
| Beispiel 2.54 der WO 00/27819 | 5,2 | 0,2 | 0,05 | 3,6 |
| Beispiel 1.32 | 30 | 2,9 | 4,9 | 25 |

Aus dem Ergebnis ist deutlich die überlegene Eigenschaften der erfindungsgemäßen Verbindungen gegenüber den bekannten Verbindungen zu erkennen, d. h. die erfindungsgemäßen Verbindungen zeigen eine wesentlich geringere Hemmung des detoxifizierenden P450-Systems als die bekannten Verbindungen, was zu deutlich weniger Wechselwirkungen mit anderen Wirkstoffen führt.

## Patentansprüche

1. Verbindungen der allgemeinen Formel I 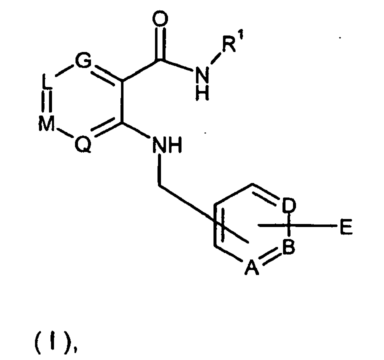 in der
A, B und D unabhängig voneinander für ein Stickstoff- oder Kohlenstoff-Atom stehen, wobei mindestens ein Stickstoff-Atom im Ring enthalten ist,
E für gegebenenfalls ein- oder mehrfach, gleich oder verschieden mit Halogen, Cyano, C₁-C₆-Alkyl, C₁-C₆-Alkoxy, Halo-C₁-C₆-Alkyl oder mit der Gruppe -OR⁵, -SR⁴, -SOR⁴ oder -SO₂R⁴ substituiertes Aryl oder Hetaryl steht, oder für die Gruppe -COOR⁸, -CONR²R³, -SR⁴, -SOR⁴, -SO₂R⁴, -SCN, -PO(OR¹²)(OR¹³), -CH=CH-COR⁹ oder -C ≡ C-R⁹ steht,
G für ein Stickstoffatom oder für die Gruppe -C-X steht,
L für ein Stickstoffatom oder für die Gruppe -C-X steht,
M für ein Stickstoffatom oder für die Gruppe -C-X steht,
Q für ein Stickstoffatom oder für die Gruppe -C-X steht, wobei im Ring maximal ein Stickstoff-Atom steht,
X für Wasserstoff, Halogen oder für unsubstituiertes oder gegebenenfalls ein- oder mehrfach mit Halogen substituiertes C₁-C₆-Alkyl, C₁-C₆-Alkyloxy oder C₁-C₆-Carboxyalkyl steht,
R¹ für gegebenenfalls ein- oder mehrfach, gleich oder verschieden mit Halogen, Hydroxy, C₁-C₆-Alkyloxy, Aralkyloxy, C₁-C₆-Alkyl und/ oder mit der Gruppe -NR²R³ substituiertes verzweigtes oder unverzweigtes C₁-C₁₂-Alkyl oder C₂-C₁₂-Alkenyl steht; oder für gegebenenfalls ein- oder mehrfach, gleich oder verschieden mit Halogen, Hydroxy, C₁-C₆-Alkyloxy, C₁-C₆-Alkyl und/ oder mit der Gruppe-NR²R³ substituiertes C₃-C₁₀-Cycloalkyl oder C₃-C₁₀-Cycloalkenyl steht; oder für gegebenenfalls ein- oder mehrfach, gleich oder verschieden mit Halogen, Cyano, Hydroxy, C₁-C₆-Alkyloxy, C₂-C₆-Alkenyl, Aryl-C₁-C₆-alkyloxy, Aralkyloxy, C₁-C₆-Alkyl, Halo-C₁-C₆-alkyl oder mit der Gruppe =O, -SO₂R⁴, OR⁵, -R⁵ oder -PO(OR¹²)(OR¹³) substituiertes Aryl oder Hetaryl steht,
R² und R³ unabhängig voneinander für Wasserstoff oder für gegebenenfalls ein- oder mehrfach, gleich oder verschieden mit Halogen, Cyano, C₁-C₆-Alkyl, Phenyl, Hydroxy-C₁-C₆-Alkyl, Halo-C₁-C₆-Alkyl oder mit der Gruppe -NR⁶R⁷, -OR⁵ , C₁-C₆-Alkyl-OR⁵, -SR⁴, -SOR⁴ oder -SO₂R⁴ substituiertes C₁-C₆-Alkyl, C₃-C₆-Cycloalkyl, C₃-C₆-Cycloalkenyl, Aryl oder Hetaryl steht, oder
R² und R³ gemeinsam mit dem Stickstoff-Atom einen C₃-C₈-Ring bilden, der gegebenenfalls ein weiteres Stickstoff-, Schwefel- oder Sauerstoffatom im Ring enthalten kann, oder die Gruppe -N(R¹⁰) enthalten kann, und der gegebenenfalls ein oder mehrfach, gleich oder verschieden mit Halogen, Cyano, C₁-C₆-Alkyl, Halo-C₁-C₆-Alkyl, Aryl oder mit der Gruppe -OR⁵, -SR⁴, -SOR⁴ oder -SO₂R⁴ substituiert sein kann,
R⁴ für Hydroxy, C₁-C₆-Alkyl, Aryl, Hetaroaryl oder für die Gruppe -NR²R³ steht,
R⁵ für Wasserstoff, C₁-C₁₂-Alkyl, Halo-C₁-C₆-Alkyl, C₃-C₆-Cycloalkyl oder Halo-C₃-C₆-Cycloalkyl steht, oder für C₁-C₁₂-Alkyl steht, welches ein- oder mehrfach mit Sauerstoff unterbrochen ist, oder für die Gruppe -(CH₂)₂NR²R³, -CH₂CN, oder -CH₂CF₃ steht,
R⁶ und R⁷ unabhängig voneinander für Wasserstoff oder C₁-C₆-Alkyl stehen, oder
R⁶ und R⁷ gemeinsam einen 5 bis 7 gliedrigen Ring bilden, der ein Sauerstoff- oder Schwefel-Atom oder die Gruppe -N(R¹⁰)-enthalten kann,
R⁸ für Wasserstoff oder für gegebenenfalls mit Halogen ein- oder mehrfach substituiertes C₁-C₆-Alkyl, C₁-C₆-Alkoxy, Benzyl, Aryl oder Hetaryl steht,
R⁹ für Wasserstoff, C₁-C₆-Alkyl, Tri-C₁₋₆-alkylsilyl, Aryl, Hetaryl oder für die Gruppe -COR¹¹ steht,
R¹⁰ für Wasserstoff, C₁-C₆-Alkyl oder Aryl steht,
R¹¹ für Wasserstoff, C₁-C₆-Alkyl oder für die Gruppe -NR²R³ steht, und
R¹² und R¹³ unabhängig voneinander für Wasserstoff oder C₁-C₆-Alkyl stehen, bedeuten,
wobei Cycloalkyl sind monocyclische Alkylringe wie Cyclopropyl, Cyclobutyl, Cyclopentyl, Cyclohexyl, oder Cycloheptyl, Cyclooctyl, Cyclononyl oder Cyclodecyl, aber auch bicyclische Ringe oder tricyclische Ringe wie Adamantanyl; und wobei der Aryl und der Heteroarylrest in R⁴, R⁸, R⁹ und R¹⁰ können jeweils 1-, 2-, oder 3-fach gleich oder verschieden substituiert sein mit Hydroxy, Halogen, C₁₋₄-Alkoxy, mit C₁-₄-Alkyl, ein oder mehrfach mit Halogen substituiertes C₁-₄-Alkyl; sowie deren tautomeren Formen, E- oder Z-Isomeren, oder falls ein chirales Zentrum vorhanden ist, auch die Racemate und Enantiomeren und Salze.

2. Verbindungen der allgemeinen Formel I, gemäß Anspruch 1, in der
A, B und D unabhängig voneinander für ein Stickstoff- oder Kohlenstoff-Atom stehen, wobei mindestens ein Stickstoff-Atom im Ring enthalten ist,
E für gegebenenfalls ein- oder mehrfach, gleich oder verschieden mit Halogen, Cyano, C₁-C₆-Alkyl, C₁-C₆-Alkoxy, Halo-C₁-C₆-Alkyl oder mit der Gruppe -OR⁵, -SR⁴, -SOR⁴ oder -SO₂R⁴ substituiertes Aryl oder Hetaryl steht, oder für die Gruppe -COOR⁸, -CONR²R³, -SR⁴, -SOR⁴, -SO₂R⁴, -SCN, -PO(OR¹²)(OR¹³), -CH=CH-COR⁹ oder -C ≡ C-R⁹ steht,
G für ein Stickstoffatom oder für die Gruppe -C-X steht,
L für ein Stickstoffatom oder für die Gruppe -C-X steht,
M für ein Stickstoffatom oder für die Gruppe -C-X steht,
Q für ein Stickstoffatom oder für die Gruppe -C-X steht, wobei im Ring maximal ein Stickstoff-Atom steht,
X für Wasserstoff, Halogen oder für unsubstituiertes oder gegebenenfalls ein- oder mehrfach mit Halogen substituiertes C₁-C₆-Alkyl, C₁-C₆-Alkyloxy oder C₁-C₆-Carboxyalkyl steht,
R¹ für gegebenenfalls ein- oder mehrfach, gleich oder verschieden mit Halogen, Cyano, Hydroxy, C₁-C₆-Alkyloxy, C₂-C₆-Alkenyl, Aryl-C₁-C₆-alkyloxy, Aralkyloxy, C₁-C₆-Alkyl, Halo-C₁-C₆-alkyl oder mit der Gruppe =O, -SO₂R⁴ , OR⁵_{,} -R⁵ oder -PO(OR¹²)(OR¹³) substituiertes Aryl oder Hetaryl steht,
R² und R³ unabhängig voneinander für Wasserstoff oder für gegebenenfalls ein- oder mehrfach, gleich oder verschieden mit Halogen, Cyano, C₁-C₆-Alkyl, Phenyl, Hydroxy-C₁-C₆-Alkyl, Halo-C₁-C₆-Alkyl oder mit der Gruppe -NR⁶R⁷, -OR⁵ , C₁-C₆-Alkyl-OR⁵, -SR⁴, -SOR⁴ oder -SO₂R⁴ substituiertes C₁-C₆-Alkyl, C₃-C₆-Cycloalkyl, C₃-C₆-Cycloalkenyl, Aryl oder Hetaryl steht, oder
R² und R³ gemeinsam mit dem Stickstoff-Atom einen C₃-C₈-Ring bilden, der gegebenenfalls ein weiteres Stickstoff-, Schwefel- oder Sauerstoffatom im Ring enthalten kann, oder die Gruppe-N(R¹⁰) enthalten kann, und der gegebenenfalls ein oder mehrfach, gleich oder verschieden mit Halogen, Cyano, C₁-C₆-Alkyl, Halo-C₁-C₆-Alkyl, Aryl oder mit der Gruppe -OR⁵ , -SR⁴,-SOR⁴ oder -SO₂R⁴ substituiert sein kann,
R⁴ für Hydroxy, C₁-C₆-Alkyl, Aryl, Hetaroaryl oder für die Gruppe-NR²R³ steht,
R⁵ für Wasserstoff, C₁-C₁₂-Alkyl, Halo-C₁-C₆-Alkyl, C₃-C₆-Cycloalkyl oder Halo-C₃-C₆-Cycloalkyl steht, oder für C₁-C₁₂-Alkyl steht, welches ein- oder mehrfach mit Sauerstoff unterbrochen ist, oder für die Gruppe -(CH₂)₂NR²R³, -CH₂CN, oder -CH₂CF₃ steht,
R⁶ und R⁷ unabhängig voneinander für Wasserstoff oder C₁-C₆-Alkyl stehen,
oder
R⁶ und R⁷ gemeinsam einen 5 bis 7 gliedrigen Ring bilden, der ein Sauerstoff- oder Schwefel-Atom oder die Gruppe -N(R¹⁰)-enthalten kann,
R⁸ für Wasserstoff oder für gegebenenfalls mit Halogen ein- oder mehrfach substituiertes C₁-C₆-Alkyl, C₁-C₆-Alkoxy, Benzyl, Aryl oder Hetaryl steht,
R⁹ für Wasserstoff, C₁-C₆-Alkyl, Tri-C₁-C₆-alkylsilyl, Aryl, Hetaryl oder für die Gruppe -COR¹¹ steht,
R¹⁰ für Wasserstoff, C₁-C₆-Alkyl oder Aryl steht,
R¹¹ für Wasserstoff, C₁-C₆-Alkyl oder für die Gruppe -NR²R³ steht, und
R¹² und R¹³ unabhängig voneinander für Wasserstoff oder C₁-C₆-Alkyl stehen, bedeuten, sowie deren tautomeren Formen, E-oder Z-Isomeren, oder falls ein chirales Zentrum vorhanden ist, auch die Racemate und Enantiomeren und Salze.

3. Verbindungen der allgemeinen Formel I, gemäß den Ansprüchen 1 und 2, in der
A, B und D unabhängig voneinander für ein Stickstoff- oder Kohlenstoff-Atom stehen, wobei mindestens ein Stickstoff-Atom im Ring enthalten ist,
E für gegebenenfalls ein- oder mehrfach, gleich oder verschieden mit Halogen, Cyano, C₁-C₆Alkyl, C₁-C₆Alkoxy, Halo-C₁-C₆Alkyl oder mit der Gruppe -OR⁵ , -SR⁴, -SOR⁴ oder -SO₂R⁴ substituiertes Aryl oder Hetaryl steht, oder für die Gruppe -COOR⁸, -CONR²R³, -SR⁴, -SOR⁴, -SO₂R⁴, -SCN, -PO(OR¹²)(OR¹³), -CH=CH-COR⁹ oder -C ≡ C-R⁹ steht,
G für ein Stickstoffatom oder für die Gruppe -C-X steht,
L für ein Stickstoffatom oder für die Gruppe -C-X steht,
M für ein Stickstoffatom oder für die Gruppe -C-X steht,
Q für ein Stickstoffatom oder für die Gruppe -C-X steht, wobei im Ring maximal ein Stickstoff-Atom steht,
X für Wasserstoff oder Halogen steht,
R¹ für gegebenenfalls ein- oder mehrfach, gleich oder verschieden mit Halogen, Hydroxy, C₁-C₆-Alkyloxy, Aralkyloxy, C₁-C₆-Alkyl, Halo-C₁-C₆-alkyl oder mit der Gruppe -SO₂R⁴, OR⁵, -R⁵ oder -PO(OR¹²)(OR¹³) substituiertes Aryl oder Hetaryl steht,
R² und R³ unabhängig voneinander für Wasserstoff oder für gegebenenfalls ein- oder mehrfach, gleich oder verschieden mit Halogen, Cyano, C₁-C₆-Alkyl, Phenyl, Hydroxy-C₁-C₆-Alkyl, Halo-C₁-C₆-Alkyl oder mit der Gruppe -NR⁶R⁷, -OR⁵, C₁-C₆-Alkyl-OR⁵, -SR⁴, -SOR⁴ oder -SO₂R⁴ substituiertes C₁-C₆-Alkyl, C₃-C₆-Cycloalkyl, C₃-C₆-Cycloalkenyl, Aryl oder Hetaryl steht, oder
R² und R³ gemeinsam mit dem Stickstoff-Atom einen C₃-C₈-Ring bilden, der gegebenenfalls ein weiteres Stickstoff-, Schwefel- oder Sauerstoffatom im Ring enthalten kann, oder die Gruppe -N(R¹⁰) enthalten kann, und der gegebenenfalls ein oder mehrfach, gleich oder verschieden mit Halogen, Cyano, C₁-C₆-Alkyl, Halo-C₁-C₆-Alkyl, Aryl oder mit der Gruppe -OR⁵,-SR⁴, -SOR⁴ oder -SO₂R⁴ substituiert sein kann,
R⁴ für Hydroxy oder für die Gruppe -NR²R³ steht,
R⁵ für Wasserstoff, C₁-C₁₂-Alkyl oder für C₁-C₁₂-Alkyl steht, welches ein- oder mehrfach mit Sauerstoff unterbrochen ist, oder für die Gruppe -(CH₂)₂NR²R³, -CH₂CN, oder -CH₂CF₃ steht,
R⁶ und R⁷ unabhängig voneinander für Wasserstoff oder C₁-C₆-Alkyl stehen,
oder
R⁶ und R⁷ gemeinsam einen 5 bis 7 gliedrigen Ring bilden, der ein Sauerstoff- oder Schwefel-Atom oder die Gruppe -N(R¹⁰)-enthalten kann,
R⁸ für Wasserstoff oder für gegebenenfalls mit Halogen ein- oder mehrfach substituiertes C₁-C₆-Alkyl, C₁-C₆-Alkoxy, Benzyl, Aryl oder Hetaryl steht,
R⁹ für Wasserstoff, C₁-C₆-Alkyl, Tri-C₁-C₆-alkylsilyl, Aryl, Hetaryl oder für die Gruppe -COR¹¹ steht,
R¹⁰ für Wasserstoff, C₁-C₆-Alkyl oder Aryl steht,
R¹¹ für Wasserstoff, C₁-C₆-Alkyl oder für die Gruppe -NR²R³ steht, und
R¹² und R¹³ unabhängig voneinander für Wasserstoff oder C₁-C₆-Alkyl stehen, bedeuten, sowie deren tautomeren Formen, E-oder Z-Isomeren, oder falls ein chirales Zentrum vorhanden ist, auch die Racemate und Enantiomeren und Salze.

4. Verbindungen der allgemeinen Formel I, gemäß den Ansprüchen 1 bis 3, in der
A, B und D für ein Stickstoff- oder Kohlenstoff-Atom stehen, wobei mindestens ein Stickstoff-Atom im Ring enthalten ist,
E für gegebenenfalls ein- oder mehrfach, gleich oder verschieden mit Halogen, Cyano, C₁-C₆-Alkyl, C₁-C₆-Alkoxy, Halo-C₁-C₆-Alkyl oder mit der Gruppe -OR⁵ , -SR⁴, -SOR⁴ oder -SO₂R⁴ substituiertes Hetaryl steht, oder für die Gruppe -COOR⁸, -CONR²R³, -SR⁴, -SOR⁴, -SO₂R⁴, -SCN, -PO(OR¹²)(OR¹³), -CH=CH-COR⁹ oder -C ≡ C-R⁹ steht,
G für die Gruppe -C-X steht,
L für die Gruppe -C-X steht,
M für die Gruppe -C-X steht,
Q für ein Stickstoffatom oder für die Gruppe -C-X steht,
X für Wasserstoff oder Halogen steht,
R¹ für gegebenenfalls ein- oder mehrfach, gleich oder verschieden mit Halogen, Hydroxy, C₁-C₆-Alkyloxy, Aralkyloxy, C₁-C₆-Alkyl, Halo-C₁-C₆-alkyl oder mit der Gruppe -SO₂R⁴, OR⁵, -R⁵ oder -PO(OR¹²)(OR¹³) substituiertes Phenyl, Thiophen, Furan, Oxazol, Thiazol, Imidazol, Pyrazol, Pyridin, Pyrimidin, Triazin, Chinolin, Isochinolin oder substituiert an der Gruppe 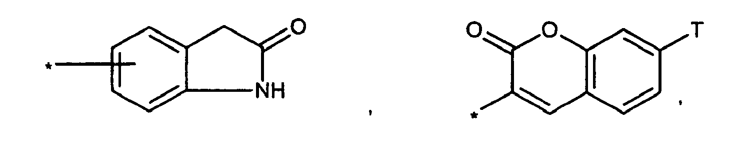 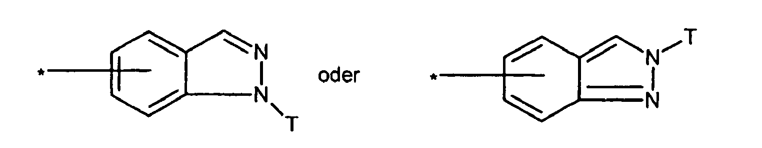 steht, in der T für Wasserstoff, C₁-C₆-Alkyl oder C₁-C₆-Alkoxy steht,
R² und R³ unabhängig voneinander für Wasserstoff oder für gegebenenfalls ein- oder mehrfach, gleich oder verschieden mit Halogen, Cyano, C₁-C₆-Alkyl, Phenyl, Hydroxy-C₁-C₆-Alkyl, Halo-C₁-C₆-Alkyl oder mit der Gruppe -NR⁶R⁷, -OR⁵ , C₁-C₆-Alkyl-OR⁵, -SR⁴, -SOR⁴ oder -SO₂R⁴ substituiertes C₁-C₆-Alkyl, C₃-C₆-Cycloalkyl, C₃-C₆-Cycloalkenyl, Aryl oder Hetaryl steht, oder
R² und R³ gemeinsam mit dem Stickstoff-Atom einen C₃-C₈-Ring bilden, der gegebenenfalls ein weiteres Stickstoff-, Schwefel- oder Sauerstoffatom im Ring enthalten kann, oder die Gruppe-N(R¹⁰) enthalten kann, und der gegebenenfalls ein oder mehrfach, gleich oder verschieden mit Halogen, Cyano, C₁-C₆-Alkyl, Halo-C₁-C₆-Alkyl, Aryl oder mit der Gruppe -OR⁵, -SR⁴,-SOR⁴ oder -SO₂R⁴ substituiert sein kann,
R⁴ für Hydroxy oder für die Gruppe -NR²R³ steht,
R⁵ für Wasserstoff, C₁-C₁₂-Alkyl oder für C₁-C₁₂-Alkyl steht, welches ein- oder mehrfach mit Sauerstoff unterbrochen ist, oder für die Gruppe -(CH₂)₂NR²R³, -CH₂CN , oder -CH₂CF₃ steht,
R⁶ und R⁷ unabhängig voneinander für Wasserstoff oder C₁-C₆-Alkyl stehen,
oder
R⁶ und R⁷ gemeinsam einen 5 bis 7 gliedrigen Ring bilden, der ein Sauerstoff- oder Schwefel-Atom enthalten kann,
R⁸ für Wasserstoff oder für gegebenenfalls mit Halogen ein- oder mehrfach substituiertes C₁-C₆-Alkyl, C₁-C₆-Alkoxy, Benzyl, Aryl oder Hetaryl steht, und
R⁹ für Wasserstoff, C₁-C₆-Alkyl oder Tri-C₁-C₆-alkylsilyl steht, und
R¹² und R¹³ unabhängig voneinander für Wasserstoff oder C₁-C₆-Alkyl stehen, bedeuten, sowie deren tautomeren Formen, E-oder Z-Isomeren, oder falls ein chirales Zentrum vorhanden ist, auch die Racemate und Enantiomeren und Salze.

5. Verbindungen der allgemeinen Formel I, gemäß den Ansprüchen 1 bis 4, in der
A, B und D unabhängig voneinander für ein Stickstoff- oder Kohlenstoff-Atom stehen, wobei mindestens ein Stickstoff-Atom im Ring enthalten ist,
E für Thienyl, Pyridyl oder für die Gruppe -COOR⁸, -CONR²R³ oder -C ≡ C-R⁹ steht,
G für die Gruppe -C-X steht,
L für die Gruppe -C-X steht,
M für die Gruppe -C-X steht,
Q für ein Stickstoffatom oder für die Gruppe -C-X steht,
X für Wasserstoff oder Halogen steht,
R¹ für gegebenenfalls ein- oder mehrfach, gleich oder verschieden mit Halogen, Hydroxy, C₁-C₆-Alkyloxy, Aralkyloxy, C₁-C₆-Alkyl, Halo-C₁-C₆-alkyl oder mit der Gruppe -SO₂R⁴, OR⁵, -R⁵ oder -PO(OR¹²)(OR¹³) substituiertes Phenyl, Thiophen, Furan, Oxazol, Thiazol, Imidazol, Pyrazol, Pyridin, Pyrimidin, Triazin, Chinolin oder Isochinolin oder substituiert an der Gruppe 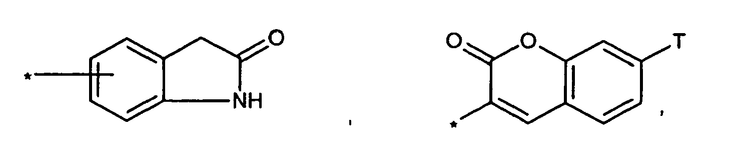 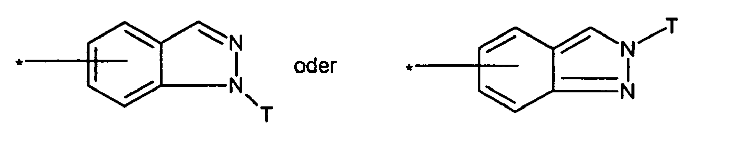 steht, in der T für Wasserstoff, C₁-C₆-Alkyl oder C₁-C₆-Alkoxy steht,
R² und R³ unabhängig voneinander für Wasserstoff oder für gegebenenfalls ein- oder mehrfach, gleich oder verschieden mit Halogen, C₁-C₆-Alkyl, Phenyl oder mit der Gruppe-NR⁶R⁷, -OR⁵ oder C₁-C₆-Alkyl-OR⁵, substituiertes C₁-C₆-Alkyl, C₃-C₆-Cycloalkyl, Phenyl oder Pyridyl steht, oder
R² und R³ gemeinsam mit dem Stickstoff-Atom einen C₃-C₈-Ring bilden, der gegebenenfalls ein weiteres Stickstoff- oder Sauerstoffatom im Ring enthalten kann, und der gegebenenfalls ein oder mehrfach, gleich oder verschieden mit C₁-C₆-Alkyl substituiert sein kann,
R⁴ für Hydroxy oder für die Gruppe -NR²R³ steht,
R⁵, R⁶ und R⁷unabhängig voneinander für Wasserstoff oder C₁-C₆-Alkyl stehen,
oder
R⁶ und R⁷ gemeinsam einen 5 bis 7 gliedrigen Ring bilden, der ein Sauerstoff- oder Schwefel-Atom enthalten kann,
R⁸ für Wasserstoff, C₁-C₆-Alkyl oder Benzyl steht, und
R⁹ für Wasserstoff, C₁-C₆-Alkyl oder Tri-C₁-C₆-alkylsilyl steht, und
R¹² und R¹³ unabhängig voneinander für Wasserstoff oder C₁-C₆-Alkyl stehen, bedeuten, sowie deren tautomeren Formen, E-oder Z-Isomeren, oder falls ein chirales Zentrum vorhanden ist, auch die Racemate und Enantiomeren und Salze.

6. Arzneimittel, enthaltend mindestens eine Verbindung gemäß den Ansprüchen 1 bis 5.

7. Arzneimittel gemäß Anspruch 6, zur Verwendung bei Psoriasis, Kaposis Sarkom, Restenose, wie z. B. Stent-induzierte Restenose, Endometriose, Crohns disease, Hodgkins disease, Leukämie, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotischen Erkrankungen, wie Leberzirrhose, mesangialzellproliferative Erkrankungen, Artheriosklerose, Verletzungen des Nervengewebes, Hemmung der Reocclusion von Gefäßen nach Ballonkatheterbehandlung, Gefäßprothetik oder Einsetzen von mechanischen Vorrichtungen zum Offenhalten von Gefäßen, wie z. B. Stents, und als Immunsuppressiva, und zur Unterstützung der narbenfreien Wundheilung, und bei Altersflecken und bei Kontaktdermatitis.

8. Arzneimittel gemäß Anspruch 6, zur Verwendung als VEGFR Kinase 3 - Inhibitor bei der Lymphangiogenese und bei hyper- und dysplastischen Veränderungen des Lymphgefäßsystems.

9. Verbindungen gemäß den Ansprüchen 1 bis 5 und Arzneimittel, gemäß den Ansprüchen 6 bis 8, mit geeigneten Formulierungs und Trägerstoffen.

10. Verwendung der Verbindungen der Formel I, gemäß den Ansprüchen 1 bis 5, zur Herstellung eines Arzneimittels als Inhibitoren der Tyrosinkinase KDR und FLT.

11. Verwendung der Verbindungen der allgemeinen Formel I, gemäß den Ansprüchen 1 bis 5, zur Herstellung eines Arzneimittels in Form eines pharmazeutischen Präparats für die enteral, parenterale und orale Applikation.

12. Verwendung der Verbindungen gemäß den Ansprüchen 1 bis 5 zur Herstellung eines Arzneimittles für die Behandlung von Psoriasis, Kaposis Sarkom, Restenose, wie z. B. Stent-induzierte Restenose, Endometriose, Crohns disease, Hodgkins disease, Leukämie, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen, Artheriosklerose, Verletzungen des Nervengewebes und zur Hemmung der Reocclusion von Gefäßen nach Ballonkatheterbehandlung, bei der Gefäßprothetik oder nach dem Einsetzen von mechanischen Vorrichtungen zum Offenhalten von Gefäßen, wie z. B. Stents, und als Immunsuppressiva, und zur Unterstützung der narbenfreien Wundheilung, und bei Altersflecken und bei Kontaktdermatitis.

## Claims

1. Compounds of general formula I 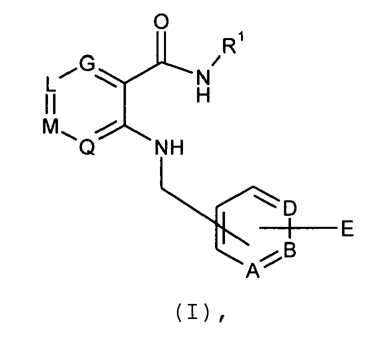 in which
A, B and D, independently of one another, stand for a nitrogen or carbon atom, whereby at least one nitrogen atom is contained in the ring,
E stands for aryl or hetaryl that is optionally substituted in one or more places in the same way or differently with halogen, cyano, C₁-C₆-alkyl, C₁-C₆-alkoxy, halo-C₁-C₆-alkyl or with the group -OR⁵, -SR⁴, -SOR⁴ or -SO₂R⁴, or for the group -COOR⁸, -CONR²R³, -SR⁴, -SOR⁴, -SO₂R⁴, -SCN, -PO(OR¹²)(OR¹³), -CH=CH-COR⁹ or -C ≡ C-R⁹,
G stands for a nitrogen atom or for the group -C-X,
L stands for a nitrogen atom or for the group -C-X,
M stands for a nitrogen atom or for the group -C-X,
Q stands for a nitrogen atom or for the group -C-X, whereby at most one nitrogen atom is in the ring,
X stands for hydrogen, halogen or for C₁-C₆-alkyl, C₁-C₆-alkyloxy or C₁-C₆-carboxyalkyl that is unsubstituted or optionally substituted in one or more places with halogen,
R¹ stands for branched or unbranched C₁-C₁₂-alkyl or C₂-C₁₂-alkenyl that is optionally substituted in one or more places in the same way or differently with halogen, hydroxy, C₁-C₆-alkyloxy, aralkyloxy, C₁-C₆-alkyl and/or with the group -NR²R³; or for C₃-C₁₀-cycloalkyl or C₃-C₁₀-cycloalkenyl that is optionally substituted in one or more places in the same way or differently with halogen, hydroxy, C₁-C₆-alkyloxy, C₁-C₆-alkyl and/or with the group -NR²R³; or for aryl or hetaryl that is optionally substituted in one or more places in the same way or differently with halogen, cyano, hydroxy, C₁-C₆-alkyloxy, C₂-C₆-alkenyl, aryl-C₁-C₆-alkyloxy, aralkyloxy, C₁-C₆-alkyl, halo-C₁-C₆-alkyl or with the group =O, -SO₂R⁴, OR⁵, -R⁵ or -PO(OR¹²)(OR¹³),
R² and R³, independently of one another, stand for hydrogen or for C₁-C₆-alkyl, C₃-C₆-cycloalkyl, C₃-C₆-cycloalkenyl, aryl or hetaryl that is optionally substituted in one or more places in the same way or differently with halogen, cyano, C₁-C₆-alkyl, phenyl, hydroxy-C₁-C₆-alkyl, halo-C₁-C₆-alkyl, or with the group -NR⁶R⁷, -OR⁵, C₁-C₆-alkyl-OR⁵, -SR⁴, -SOR⁴ or -SO₂R⁴, or
R² and R³, together with the nitrogen atom, form a C₃-C₈-ring, which optionally can contain another nitrogen, sulfur or oxygen atom in the ring, or can contain the group -N(R¹⁰), and which optionally can be substituted in one or more places in the same way or differently with halogen, cyano, C₁-C₆-alkyl, halo-C₁-C₆-alkyl, aryl or with the group -OR⁵, -SR⁴, -SOR⁴ or -SO₂R⁴,
R⁴ stands for hydroxy, C₁-C₆-alkyl, aryl, heteroaryl or for the group -NR²R³,
R⁵ stands for hydrogen, C₁-C₁₂-alkyl, halo-C₁-C₆-alkyl, C₃-C₆-cycloalkyl or halo-C₃-C₆-cycloalkyl, or for C₁-C₁₂-alkyl, which is interrupted in one or more places with oxygen or stands for the group -(CH₂)₂NR²R³, -CH₂CN or -CH₂CF₃,
R⁶ and R⁷, independently of one another, stand for hydrogen or C₁-C₆-alkyl, or
R⁶ and R⁷ together form a 5- to 7-membered ring that can contain an oxygen or sulfur atom or the group -N(R¹⁰)-,
R⁸ stands for hydrogen or for C₁-C₆-alkyl, C₁-C₆-alkoxy, benzyl, aryl or hetaryl that is optionally substituted with halogen in one or more places,
R⁹ stands for hydrogen, C₁-C₆-alkyl, tri-C₁₋₆-alkylsilyl, aryl, hetaryl, or for the group -COR¹¹,
R¹⁰ stands for hydrogen, C₁-C₆-alkyl or aryl,
R¹¹ stands for hydrogen, C₁-C₆-alkyl or for the group -NR²R³, and
R¹² and R¹³, independently of one another, stand for hydrogen or C₁-C₆-alkyl, as well as isomers, enantiomers and salts thereof.

2. Compounds of general formula I, according to claim 1, in which
A, B, and D, independently of one another, stand for a nitrogen or carbon atom, whereby at least one nitrogen atom is contained in the ring,
E stands for aryl or hetaryl that is optionally substituted in one or more places in the same way or differently with halogen, cyano, C₁-C₆-alkyl, C₁-C₆-alkoxy, halo-C₁-C₆-alkyl or with the group -OR⁵, -SR⁴, -SOR⁴ or -SO₂R⁴, or for the group -COOR⁸, -CONR²R³, -SR⁴, -SOR⁴, -SO₂R⁴, -SCN, -PO(OR¹²)(OR¹³), -CH=CH-COR⁹ or -C ≡ C-R⁹,
G stands for a nitrogen atom or for the group -C-X,
L stands for a nitrogen atom or for the group -C-X,
M stands for a nitrogen atom or for the group -C-X,
Q stands for a nitrogen atom or for the group -C-X,
whereby at most one nitrogen atom is in the ring,
X stands for hydrogen, halogen or for C₁-C₆-alkyl, C₁-C₆-alkyloxy or C₁-C₆-carboxyalkyl that is unsubstituted or that is optionally substituted in one or more places with halogen,
R¹ stands for aryl or hetaryl that is optionally substituted in one or more places in the same way or differently with halogen, cyano, hydroxy, C₁-C₆-alkyloxy, C₂-C₆-alkenyl, aryl-C₁-C₆-alkyloxy, aralkyloxy, C₁-C₆-alkyl, halo-C₁-C₆-alkyl or with the group =O, -SO₂R⁴, OR⁵, -R⁵ or -PO(OR¹²)(OR¹³),
R² and R³, independently of one another, stand for hydrogen or for C₁-C₆-alkyl, C₃-C₆-cycloalkyl, C₃-C₆-cycloalkenyl, aryl or hetaryl that is optionally substituted in one or more places in the same way or differently with halogen, cyano, C₁-C₆-alkyl, phenyl, hydroxy-C₁-C₆-alkyl, halo-C₁-C₆-alkyl or with the group -NR⁶R⁷, -OR⁵, C₁-C₆-alkyl-OR⁵, -SR⁴, -SOR⁴ or -SO₂R⁴, or
R² and R³ together with the nitrogen atom form a C₃-C₈ ring, which optionally can contain another nitrogen, sulfur or oxygen atom in the ring, or can contain the group -N(R¹⁰), and which optionally can be substituted in one or more places in the same way or differently with halogen, cyano, C₁-C₆-alkyl, halo-C₁-C₆-alkyl, aryl or with the group -OR⁵, -SR⁴, -SOR⁴ or -SO₂R⁴,
R⁴ stands for hydroxy, C₁-C₆-alkyl, aryl, heteroaryl or for the group -NR²R³,
R⁵ stands for hydrogen, C₁-C₁₂-alkyl, halo-C₁-C₆-alkyl, C₃-C₆-cycloalkyl or halo-C₃-C₆-cycloalkyl, or for C₁-C₁₂-alkyl, which is interrupted in one or more places with oxygen, or stands for the group -(CH₂)₂NR²R³, -CH₂CN or -CH₂CF₃,
R⁶ and R⁷, independently of one another, stand for hydrogen or C₁-C₆-alkyl,
or
R⁶ and R⁷ together form a 5- to 7-membered ring, which can contain an oxygen or sulfur atom or the group -N(R¹⁰)-,
R⁸ stands for hydrogen or for C₁-C₆-alkyl, C₁-C₆-alkoxy, benzyl, aryl or hetaryl that is optionally substituted with halogen in one or more places,
R⁹ stands for hydrogen, C₁-C₆-alkyl, tri-C₁-C₆-alkylsilyl, aryl, hetaryl or for the group -COR¹¹,
R¹⁰ stands for hydrogen, C₁-C₆-alkyl or aryl,
R¹¹ stands for hydrogen, C₁-C₆-alkyl or for the group -NR²R³, and
R¹² and R¹³, independently of one another, stand for hydrogen or C₁-C₆-alkyl, as well as isomers, enantiomers and salts thereof.

3. Compounds of general formula I, according to claims 1 and 2, in which
A, B and D, independently of one another, stand for a nitrogen or carbon atom, whereby at least one nitrogen atom is contained in the ring,
E stands for aryl or hetaryl that is optionally substituted in one or more places in the same way or differently with halogen, cyano, C₁-C₆alkyl, C₁-C₆alkoxy, halo-C₁-C₆alkyl or with the group -OR⁵, -SR⁴, -SOR⁴ or -SO₂R⁴, or for the group -COOR⁸, -CONR²R³, -SR⁴, -SOR⁴, -SO₂R⁴, -SCN, -PO(OR¹²)(OR¹³), -CH=CH-COR⁹ or -C ≡ C-R⁹,
G stands for a nitrogen atom or for the group -C-X,
L stands for a nitrogen atom or for the group -C-X,
M stands for a nitrogen atom or for the group -C-X,
Q stands for a nitrogen atom or for the group -C-X, whereby at most one nitrogen atom is in the ring,
X stands for hydrogen or halogen,
R¹ stands for aryl or hetaryl that is optionally substituted in one or more places in the same way or differently with halogen, hydroxy, C₁-C₆-alkyloxy, aralkyloxy, C₁-C₆-alkyl, halo-C₁-C₆-alkyl or with the group ₋SO₂R⁴, OR⁵, -R⁵ or -PO(OR¹²)(OR¹³),
R² and R³, independently of one another, stand for hydrogen or for C₁-C₆-alkyl, C₃-C₆-cycloalkyl, C₃-C₆-cycloalkenyl, aryl or hetaryl that is optionally substituted in one or more places in the same way or differently with halogen, cyano, C₁-C₆-alkyl, phenyl, hydroxy-C₁-C₆-alkyl, halo-C₁-C₆-alkyl or with the group -NR⁶R⁷, -OR⁵, C₁-C₆-alkyl-OR⁵, -SR⁴, -SOR⁴ or -SO₂R⁴, or
R² and R³ together with the nitrogen atom form a C₃-C₈-ring, which optionally can contain another nitrogen, sulfur or oxygen atom in the ring, or can contain the group -N(R¹⁰), and which optionally can be substituted in one or more places in the same way or differently with halogen, cyano, C₁-C₆-alkyl, halo-C₁-C₆-alkyl, aryl or with the group -OR⁵, -SR⁴, -SOR⁴ or -SO₂R⁴,
R⁴ stands for hydroxy or for the group -NR²R³,
R⁵ stands for hydrogen, C₁-C₁₂-alkyl or for C₁-C₁₂-alkyl, which is interrupted in one or more places with oxygen or stands for the group -(CH₂)₂NR²R³, -CH₂CN or -CH₂CF₃,
R⁶ and R⁷, independently of one another, stand for hydrogen or C₁-C₆-alkyl, or
R⁶ and R⁷ together form a 5- to 7-membered ring, which can contain an oxygen or sulfur atom or the group -N(R¹⁰)-,
R⁸ stands for hydrogen or for C₁-C₆-alkyl, C₁-C₆-alkoxy, benzyl, aryl or hetaryl that is optionally substituted with halogen in one or more places,
R⁹ stands for hydrogen, C₁-C₆-alkyl, tri-C₁-C₆-alkylsilyl, aryl, hetaryl or for the group -COR¹¹,
R¹⁰ stands for hydrogen, C₁-C₆-alkyl or aryl,
R¹¹ stands for hydrogen, C₁-C₆-alkyl or for the group -NR²R³, and
R¹² and R¹³, independently of one another, stand for hydrogen or C₁-C₆-alkyl, as well as isomers, enantiomers and salts thereof.

4. Compounds of general formula I, according to claims 1 to 3, in which
A, B and D stand for a nitrogen or carbon atom, whereby at least one nitrogen atom is contained in the ring,
E stands for hetaryl that is optionally substituted in one or more places in the same way or differently with halogen, cyano, C₁₋₆-alkyl, C₁-C₆-alkoxy, halo-C₁-C₆-alkyl or with the group -OR⁵, -SR⁴, -SOR⁴ or -SO₂R⁴, or for the group -COOR⁸, -CONR²R³, -SR⁴, -SOR⁴, -SO₂R⁴, -SCN, -PO(OR¹²)(OR¹³), -CH=CH-COR⁹ or -C ≡ C-R⁹,
G stands for the group -C-X,
L stands for the group -C-X,
M stands for the group -C-X,
Q stands for a nitrogen atom or for the group -C-X,
X stands for hydrogen or halogen,
R¹ stands for phenyl, thiophene, furan, oxazole, thiazole, imidazole, pyrazole, pyridine, pyrimidine, triazine, quinoline, or isoquinoline that is optionally substituted in one or more places in the same way or differently with halogen, hydroxy, C₁-C₆-alkyloxy, aralkyloxy, C₁-C₆-alkyl, halo-C₁-C₆-alkyl or with the group -SO₂R⁴, OR⁵, -R⁵ or -PO(OR¹²)(OR¹³) or is substituted on the group 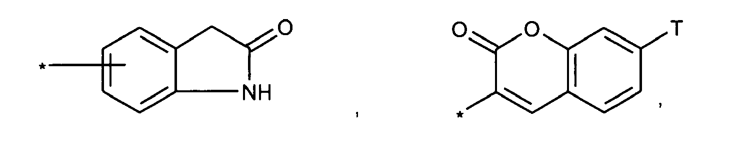 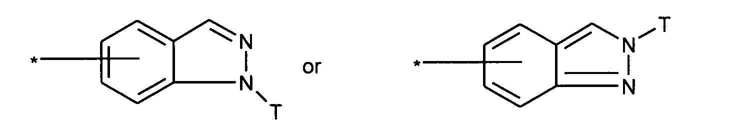 in which T stands for hydrogen, C₁-C₆-alkyl or C₁-C₆-alkoxy,
R² and R³, independently of one another, stand for hydrogen or for C₁-C₆-alkyl, C₃-C₆-cycloalkyl, C₃-C₆-cycloalkenyl, aryl or hetaryl that is optionally substituted in one or more places in the same way or differently with halogen, cyano, C₁-C₆-alkyl, phenyl, hydroxy-C₁-C₆-alkyl, halo-C₁-C₆-alkyl or with the group -NR⁶R⁷, -OR⁵, C₁-C₆-alkyl-OR⁵, -SR⁴, -SOR⁴ or -SO₂R⁴, or
R² and R³, together with the nitrogen atom, form a C₃-C₈-ring, which optionally can contain another nitrogen, sulfur or oxygen atom in the ring, or can contain the group -N(R¹⁰), and which optionally can be substituted in one or more places in the same way or differently with halogen, cyano, C₁-C₆-alkyl, halo-C₁-C₆-alkyl, aryl or with the group -OR⁵, -SR⁴, -SOR⁴ or -SO₂R⁴,
R⁴ stands for hydroxy or for the group -NR²R³,
R⁵ stands for hydrogen, C₁-C₁₂-alkyl or for C₁-C₁₂-alkyl, which is interrupted in one or more places with oxygen, or stands for the group -(CH₂)₂NR²R³, -CH₂CN, or -CH₂CF₃,
R⁶ and R⁷, independently of one another, stand for hydrogen or C₁-C₆-alkyl, or
R⁶ and R⁷ together form a 5- to 7-membered ring that can contain an oxygen or sulfur atom,
R⁸ stands for hydrogen or for C₁-C₆-alkyl, C₁-C₆-alkoxy, benzyl, aryl or hetaryl that is optionally substituted in one or more places with halogen, and
R⁹ stands for hydrogen, C₁-C₆-alkyl or tri-C₁-C₆-alkylsilyl, and
R¹² and R¹³, independently of one another, stand for hydrogen or C₁-C₆-alkyl, as well as isomers, enantiomers and salts thereof.

5. Compounds of general formula I, according to claims 1 to 4, in which
A, B and D, independently of one another, stand for a nitrogen or carbon atom, whereby at least one nitrogen atom is contained in the ring,
E stands for thienyl, pyridyl or for the group -COOR⁸, -CONR²R³, or -C ≡ C-R⁹,
G stands for the group -C-X,
L stands for the group -C-X,
M stands for the group -C-X,
Q stands for a nitrogen atom or for the group -C-X,
X stands for hydrogen or halogen,
R¹ stands for phenyl, thiophene, furan, oxazole, thiazole, imidazole, pyrazole, pyridine, pyrimidine, triazine, quinoline or isoquinoline that is optionally substituted in one or more places in the same way or differently with halogen, hydroxy, C₁-C₆-alkyloxy, aralkyloxy, C₁-C₆-alkyl, halo-C₁-C₆-alkyl or with the group -SO₂R⁴, OR⁵, -R⁵ or -PO(OR¹²)(OR¹³) or substituted on the group 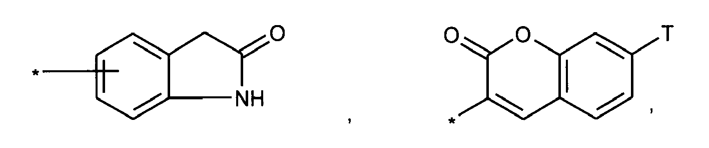 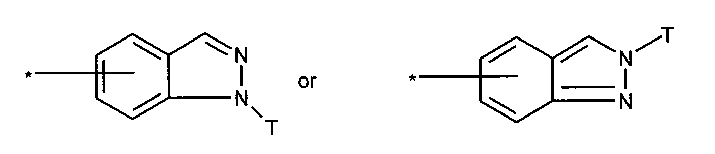 in which T stands for hydrogen, C₁-C₆-alkyl or C₁-C₆-alkoxy,
R² and R³, independently of one another, stand for hydrogen or for C₁-C₆-alkyl, C₃-C₆-cycloalkyl, phenyl or pyridyl that is optionally substituted in one or more places in the same way or differently with halogen, C₁-C₆-alkyl, phenyl or with the group -NR⁶R⁷, -OR⁵ or C₁-C₆-alkyl-OR⁵, or
R² and R³ together with the nitrogen atom form a C₃-C₈-ring, which optionally can contain another nitrogen or oxygen atom in the ring, and which optionally can be substituted in one or more places in the same way or differently with C₁-C₆-alkyl,
R⁴ stands for hydroxy or for the group -NR²R³,
R⁵, R⁶ and R⁷, independently of one another, stand for hydrogen or C₁-C₆-alkyl,
or
R⁶ and R⁷ together form a 5- to 7-membered ring, which can contain an oxygen or sulfur atom,
R⁸ stands for hydrogen, C₁-C₆-alkyl or benzyl, and
R⁹ stands for hydrogen, C₁-C₆-alkyl or tri-C₁-C₆-alkylsilyl, and
R¹² and R¹³, independently of one another, stand for hydrogen or C₁-C₆-alkyl, as well as isomers and salts thereof.

6. Pharmaceutical agents that contain at least one compound according to claims 1 to 5.

7. Pharmaceutical agents according to claim 6 for use in the case of psoriasis, Kaposi's sarcoma, restenosis, such as, e.g., stent-induced restenosis, endometriosis, Crohn's disease, Hodgkin's disease, leukemia; arthritis, such as rheumatoid arthritis, hemangioma, angiofibroma; eye diseases, such as diabetic retinopathy, neovascular glaucoma; renal diseases, such as glomerulonephritis, diabetic nephropathy, malignant nephrosclerosis, thrombic microangio-pathic syndrome, transplant rejections and glomerulopathy; fibrotic diseases, such as cirrhosis of the liver, mesangial cell proliferative diseases, arteriosclerosis, injuries to nerve tissue, inhibition of the reocclusion of vessels after balloon catheter treatment, vascular prosthetics or use of mechanical devices to keep vessels open, such as, e.g., stents, and as immunosuppressive agents, and for supporting scar-free healing, in senile keratosis and in contact dermatitis.

8. Pharmaceutical agents according to claim 6 for use as VEGFR kinase 3-inhibitors in lymphangiogenesis and in hyper- and dysplastic changes of the lymphatic system.

9. Compounds according to claims 1 to 5 and pharmaceutical agents, according to claims 6 to 8, with suitable formulation substances and vehicles.

10. Use of the compounds of formula I, according to claims 1 to 5, as inhibitors of the tyrosine kinases KDR and FLT.

11. Use of the compounds of general formula I, according to claims 1 to 5, in the form of a pharmaceutical preparation for enteral, parenteral and oral administration.

12. Use of the compounds according to claims 1 to 5 in the case of psoriasis, Kaposi's sarcoma, restenosis, such as, e.g., stent-induced restenosis, endometriosis, Crohn's disease, Hodgkin's disease, leukemia; arthritis, such as rheumatoid arthritis, hemangioma, angiofibroma; eye diseases, such as diabetic retinopathy, neovascular glaucoma; renal diseases, such as glomerulonephritis, diabetic nephropathy, malignant nephrosclerosis, thrombic microangio-pathic syndrome, transplant rejections and glomerulopathy; fibrotic diseases, such as cirrhosis of the liver, mesangial cell proliferative diseases, arteriosclerosis, injuries to nerve tissue, and for inhibiting the reocclusion of vessels after balloon catheter treatment, in vascular prosthetics or after mechanical devices are used to keep vessels open, such as, e.g., stents, and as immunosuppressive agents, and for supporting scar-free healing, and in senile keratosis and in contact dermatitis.

## Revendications

1. Composés de formule générale I 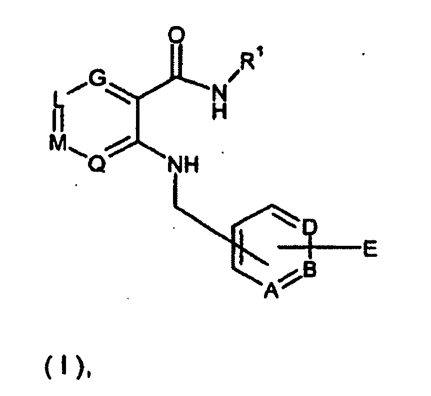 dans laquelle
A, B et D représentent chacun indépendamment un atome de carbone ou d'azote, au moins un atome d'azote étant contenu dans le cycle,
E représente un radical aryle ou hétéroaryle, éventuellement une ou plusieurs fois substitué, de façon identique ou différente, par halogène, cyano, alkyle en C₁-C₆, alcoxy en C₁-C₆, halogénoalkyle en C₁-C₆ ou par le groupe -OR⁵, -SR⁴, -SOR⁴ ou -SO₂R⁴, ou représente le groupe -COOR⁸, -CONR²R³, -SR⁴, -SOR⁴, -SO₂R⁴, -SCN, -PO(OR¹²)(OR¹³), -CH=CH-COR⁹ ou -C≡C-R⁹,
G représente un atome d'azote ou le groupe -C-X,
L représente un atome d'azote ou le groupe -C-X,
M représente un atome d'azote ou le groupe -C-X,
Q représente un atome d'azote ou le groupe -C-X, au maximum un atome d'azote étant présent dans le cycle,
X représente un atome d'hydrogène ou d'halogène ou un groupe alkyle en C₁-C₆, alkyloxy en C₁-C₆ ou carboxyalkyle en C₁-C₆, non substitué ou éventuellement une ou plusieurs fois substitué par halogène,
R¹ représente un radical alkyle en C₁-C₁₂ ou alcényle en C₂-C₁₂, ramifié ou non ramifié, éventuellement une ou plusieurs fois substitué, de façon identique ou différente, par halogène, hydroxy, alkyloxy en C₁-C₆, aralkyloxy, alkyle en C₁-C₆ et/ou par le groupe -NR²R³ ; ou représente un radical cycloalkyle en C₃-C₁₀ ou cycloalcényle en C₃-C₁₀, éventuellement une ou plusieurs fois substitué, de façon identique ou différente, par halogène, hydroxy, alkyloxy en C₁-C₆, alkyle en C₁-C₆ et/ou par le groupe -NR²R³ ; ou représente un radical aryle ou hétéroaryle, éventuellement une ou plusieurs fois substitué, de façon identique ou différente, par halogène, cyano, hydroxy, alkyloxy en C₁-C₆, alcényle en C₂-C₆, aryl-alkyl(C₁-C₆)oxy, aralkyloxy, alkyle en C₁-C₆, halogénoalkyle(C₁-C₆) ou par le groupe =O, -SO₂R⁴, OR⁵, -R⁵ ou -PO(OR¹²)(OR¹³),
R² et R³ représentent, indépendamment l'un de l'autre, un atome d'hydrogène ou un radical alkyle en C₁-C₆, cycloalkyle en C₃-C₆, cycloalcényle en C₃-C₆, aryle ou hétéroaryle, éventuellement une ou plusieurs fois substitué, de façon identique ou différente, par halogène, cyano, alkyle en C₁-C₆, phényle, hydroxy-alkyle(C₁-C₆), halogéno-alkyle(C₁-C₆) ou par le groupe -NR⁶R⁷, -OR⁵, alkyl (C₁-C₆)-OR⁵, -SR⁴, -SOR⁴ ou -SO₂R⁴, ou
R² et R³ forment ensemble avec l'atome d'azote un cycle en C₃-C₈ qui éventuellement peut contenir un autre atome d'azote, de soufre ou d'oxygène dans le cycle, ou peut contenir le groupe -N(R¹⁰), et qui peut être éventuellement une ou plusieurs fois substitué, de façon identique ou différente, par halogène, cyano, alkyle en C₁-C₆, halogéno-alkyle(C₁-C₆), aryle ou par le groupe -OR⁵, -SR⁴, -SOR⁴ ou -SO₂R⁴,
R⁴ représente un groupe hydroxy, alkyle en C₁-C₆, aryle, hétéroaryle ou le groupe -NR²R³,
R⁵ représente un atome d'hydrogène, un groupe alkyle en C₁-C₁₂, halogénoalkyle en C₁-C₆, cycloalkyle en C₃-C₆ ou halogéno-cycloalkyle en C₃-C₆, ou représente un groupe alkyle en C₁-C₁₂ qui est interrompu une ou plusieurs fois par un atome d'oxygène, ou représente le groupe -(CH₂)₂NR²R³, -CH₂CN ou -CH₂CF₃,
R⁶ et R⁷ représentent, indépendamment l'un de l'autre, un atome d'hydrogène ou un groupe alkyle en C₁-C₆, ou
R⁶ et R⁷ forment ensemble un cycle à 5-7 chaînons, qui peut contenir un atome d'oxygène ou de soufre ou le groupe -N(R¹⁰)-,
R⁸ représente un atome d'hydrogène ou un groupe alkyle en C₁-C₆, alcoxy en C₁-C₆, benzyle, aryle ou hétéroaryle, éventuellement une ou plusieurs fois substitué par halogène,
R⁹ représente un atome d'hydrogène, un groupe alkyle en C₁-C₆, trialkyl (C₁-C₆) -silyle, aryle, hétéroaryle ou représente le groupe -COR¹¹,
R¹⁰ représente un atome d'hydrogène, un groupe alkyle en C₁-C₆ ou aryle,
R¹¹ représente un atome d'hydrogène, un groupe alkyle en C₁-C₆ ou représente le groupe -NR²R³, et
R¹² et R¹³ représentent, indépendamment l'un de l'autre, un atome d'hydrogène ou un groupe alkyle en C₁-C₆,
les groupes cycloalkyle étant des cycles alkyle monocycliques tels que cyclopropyle, cyclobutyle, cyclopentyle, cyclohexyle, ou cycloheptyle, cylo-octyle, cyclononyle ou cyclodécyle, mais également des cycles bicycliques ou tricycliques tels qu'adamantyle ; et le radical aryle et le radical hétéroaryle dans R⁴, R⁸, R⁹ et R¹⁰ pouvant être substitués chacun 1, 2 ou 3 fois, de façon identique ou différente, par hydroxy, halogène, alcoxy en C₁-C₄, par alkyle en C₁-C₄, alkyle en C₁-C₄ une ou plusieurs fois substitué par halogène ; ainsi que leurs formes tautomères, isomères E ou Z, ou, lorsqu'un centre chiral est présent, également les racémates et leurs énantiomères et sels.

2. Composés de formule générale I selon la revendication 1, dans lesquels
A, B et D représentent chacun indépendamment un atome de carbone ou d'azote, au moins un atome d'azote étant contenu dans le cycle,
E représente un radical aryle ou hétéroaryle, éventuellement une ou plusieurs fois substitué, de façon identique ou différente, par halogène, cyano, alkyle en C₁-C₆, alcoxy en C₁-C₆, halogénoalkyle en C₁-C₆ ou par le groupe -OR⁵, -SR⁴, -SOR⁴ ou -SO₂R⁴, ou représente le groupe -COOR⁸, -CONR²R³, -SR⁴, -SOR⁴, -SO₂R⁴, -SCN, -PO(OR¹²)(OR¹³), -CH=CH-COR⁹ ou -C≡C-R⁹,
G représente un atome d'azote ou le groupe -C-X,
L représente un atome d'azote ou le groupe -C-X,
M représente un atome d'azote ou le groupe -C-X,
Q représente un atome d'azote ou le groupe -C-X, au maximum un atome d'azote étant présent dans le cycle,
X représente un atome d'hydrogène ou d'halogène ou un groupe alkyle en C₁-C₆, alkyloxy en C₁-C₆ ou carboxyalkyle en C₁-C₆, non substitué ou éventuellement une ou plusieurs fois substitué par halogène,
R¹ représente un radical aryle ou hétéroaryle, éventuellement une ou plusieurs fois substitué, de façon identique ou différente, par halogène, cyano, hydroxy, alkyloxy en C₁-C₆, alcényle en C₂-C₆, aryl-alkyl(C₁-C₆)oxy, aralkyloxy, alkyle en C₁-C₆, halogénoalkyle(C₁-C₆) ou par le groupe =O, -SO₂R⁴, OR⁵, -R⁵ ou -PO(OR¹²)(OR¹³),
R² et R³ représentent, indépendamment l'un de l'autre, un atome d'hydrogène ou un radical alkyle en C₁-C₆, cycloalkyle en C₃-C₆, cycloalcényle en C₃-C₆, aryle ou hétéroaryle, éventuellement une ou plusieurs fois substitué, de façon identique ou différente, par halogène, cyano, alkyle en C₁-C₆, phényle, hydroxy-alkyle(C₁-C₆), halogéno-alkyle(C₁-C₆) ou par le groupe-NR⁶R⁷, -OR⁵, alkyl (C₁-C₆)-OR⁵, -SR⁴, -SOR⁴ ou -SO₂R⁴, ou
R² et R³ forment ensemble avec l'atome d'azote un cycle en C₃-C₈ qui éventuellement peut contenir un autre atome d'azote, de soufre ou d'oxygène dans le cycle, ou peut contenir le groupe -N(R¹⁰), et qui peut être éventuellement une ou plusieurs fois substitué, de façon identique ou différente, par halogène, cyano, alkyle en C₁-C₆, halogéno-alkyle(C₁-C₆), aryle ou par le groupe -OR⁵, -SR⁴, -SOR⁴ ou -SO₂R⁴,
R⁴ représente un groupe hydroxy, alkyle en C₁-C₆, aryle, hétéroaryle ou le groupe -NR²R³,
R⁵ représente un atome d'hydrogène, un groupe alkyle en C₁-C₁₂, halogénoalkyle en C₁-C₆, cycloalkyle en C₃-C₆ ou halogéno-cycloalkyle en C₃-C₆, ou représente un groupe alkyle en C₁-C₁₂ qui est interrompu une ou plusieurs fois par un atome d'oxygène, ou représente le groupe -(CH₂)₂NR²R³, -CH₂CN ou -CH₂CF₃,
R⁶ et R⁷ représentent, indépendamment l'un de l'autre, un atome d'hydrogène ou un groupe alkyle en C₁-C₆, ou
R⁶ et R⁷ forment ensemble un cycle à 5-7 chaînons, qui peut contenir un atome d'oxygène ou de soufre ou le groupe -N(R¹⁰)-,
R⁸ représente un atome d'hydrogène ou un groupe alkyle en C₁-C₆, alcoxy en C₁-C₆, benzyle, aryle ou hétéroaryle, éventuellement une ou plusieurs fois substitué par halogène,
R⁹ représente un atome d'hydrogène, un groupe alkyle en C₁-C₆, trialkyl(C₁-C₆)-silyle, aryle, hétéroaryle ou représente le groupe -COR¹¹,
R¹⁰ représente un atome d'hydrogène, un groupe alkyle en C₁-C₆ ou aryle,
R¹¹ représente un atome d'hydrogène, un groupe alkyle en C₁-C₆ ou représente le groupe -NR²R³, et
R¹² et R¹³ représentent, indépendamment l'un de l'autre, un atome d'hydrogène ou un groupe alkyle en C₁-C₆,
ainsi que leurs formes tautomères, isomères E ou Z, ou, lorsqu'un centre chiral est présent, également les racémates et leurs énantiomères et sels.

3. Composés de formule générale I selon les revendications 1 et 2, dans lesquels
A, B et D représentent chacun indépendamment un atome de carbone ou d'azote, au moins un atome d'azote étant contenu dans le cycle,
E représente un radical aryle ou hétéroaryle, éventuellement une ou plusieurs fois substitué, de façon identique ou différente, par halogène, cyano, alkyle en C₁-C₆, alcoxy en C₁-C₆, halogénoalkyle en C₁-C₆ ou par le groupe -OR⁵, -SR⁴, -SOR⁴ ou -SO₂R⁴, ou représente le groupe -COOR⁸, -CONR²R³, -SR⁴, -SOR⁴, -SO₂R⁴, -SCN, -PO(OR¹²)(OR¹³), -CH=CH-COR⁹ ou -C≡C-R⁹,
G représente un atome d'azote ou le groupe -C-X,
L représente un atome d'azote ou le groupe -C-X,
M représente un atome d'azote ou le groupe -C-X,
Q représente un atome d'azote ou le groupe -C-X, au maximum un atome d'azote étant présent dans le cycle,
X représente un atome d'hydrogène ou d'halogène,
R¹ représente un radical aryle ou hétéroaryle, éventuellement une ou plusieurs fois substitué, de façon identique ou différente, par halogène, hydroxy, alkyloxy en C₁-C₆, aralkyloxy, alkyle en C₁-C₆, halogénoalkyle(C₁-C₆) ou par le groupe -SO₂R⁴, OR⁵, -R⁵ ou -PO(OR¹²)(OR¹³),
R² et R³ représentent, indépendamment l'un de l'autre, un atome d'hydrogène ou un radical alkyle en C₁-C₆, cycloalkyle en C₃-C₆, cycloalcényle en C₃-C₆, aryle ou hétéroaryle, éventuellement une ou plusieurs fois substitué, de façon identique ou différente, par halogène, cyano, alkyle en C₁-C₆, phényle, hydroxy-alkyle(C₁-C₆), halogéno-alkyle(C₁-C₆) ou par le groupe -NR⁶R⁷, -OR⁵, alkyl(C₁-C₆)-OR⁵, -SR⁴, -SOR⁴ ou -SO₂R⁴, ou
R² et R³ forment ensemble avec l'atome d'azote un cycle en C₃-C₈ qui éventuellement peut contenir un autre atome d'azote, de soufre ou d'oxygène dans le cycle, ou peut contenir le groupe -N(R¹⁰), et qui peut être éventuellement une ou plusieurs fois substitué, de façon identique ou différente, par halogène, cyano, alkyle en C₁-C₆, halogéno-alkyle(C₁-C₆), aryle ou par le groupe -OR⁵, -SR⁴, -SOR⁴ ou -SO₂R⁴,
R⁴ représente un groupe hydroxy ou le groupe -NR²R³,
R⁵ représente un atome d'hydrogène, un groupe alkyle en C₁-C₁₂ ou représente un groupe alkyle en C₁-C₁₂ qui est interrompu une ou plusieurs fois par un atome d'oxygène, ou représente le groupe-(CH₂)₂NR²R³, -CH₂CN ou -CH₂CF₃,
R⁶ et R⁷ représentent, indépendamment l'un de l'autre, un atome d'hydrogène ou un groupe alkyle en C₁-C₆, ou
R⁶ et R⁷ forment ensemble un cycle à 5-7 chaînons, qui peut contenir un atome d'oxygène ou de soufre ou le groupe -N(R¹⁰)-,
R⁸ représente un atome d'hydrogène ou un groupe alkyle en C₁-C₆, alcoxy en C₁-C₆, benzyle, aryle ou hétéroaryle, éventuellement une ou plusieurs fois substitué par halogène,
R⁹ représente un atome d'hydrogène, un groupe alkyle en C₁-C₆, trialkyl(C₁-C₆)-silyle, aryle, hétéroaryle ou représente le groupe -COR¹¹,
R¹⁰ représente un atome d'hydrogène, un groupe alkyle en C₁-C₆ ou aryle,
R¹¹ représente un atome d'hydrogène, un groupe alkyle en C₁-C₆ ou représente le groupe -NR²R³, et
R¹² et R¹³ représentent, indépendamment l'un de l'autre, un atome d'hydrogène ou un groupe alkyle en C₁-C₆,
ainsi que leurs formes tautomères, isomères E ou Z, ou, lorsqu'un centre chiral est présent, également les racémates et leurs énantiomères et sels.

4. Composés de formule générale I selon les revendications 1 à 3, dans lesquels
A, B et D représentent un atome de carbone ou d'azote, au moins un atome d'azote étant contenu dans le cycle,
E représente un radical hétéroaryle éventuellement une ou plusieurs fois substitué, de façon identique ou différente, par halogène, cyano, alkyle en C₁-C₆, alcoxy en C₁-C₆, halogénoalkyle en C₁-C₆ ou par le groupe -OR⁵, -SR⁴, -SOR⁴ ou -SO₂R⁴, ou représente le groupe -COOR⁸, -CONR²R³, -SR⁴, -SOR⁴, -SO₂R⁴, -SCN, -PO(OR¹²)(OR¹³), -CH=CH-COR⁹ ou -C≡C-R⁹,
G représente le groupe -C-X,
L représente le groupe -C-X,
M représente le groupe -C-X,
Q représente un atome d'azote ou le groupe -C-X,
X représente un atome d'hydrogène ou d'halogène,
R¹ représente un radical phényle, thiophène, furanne, oxazole, thiazole, imidazole, pyrazole, pyridine, pyrimidine, triazine, quinoléine, isoquinoléine, éventuellement une ou plusieurs fois substitué, de façon identique ou différente, par halogène, hydroxy, alkyloxy en C₁-C₆, aralkyloxy, alkyle en C₁-C₆, halogénoalkyle(C₁-C₆) ou par le groupe -SO₂R⁴, OR⁵, -R⁵ ou -PO(OR¹²)(OR¹³), ou est présent comme substituant sur le groupe 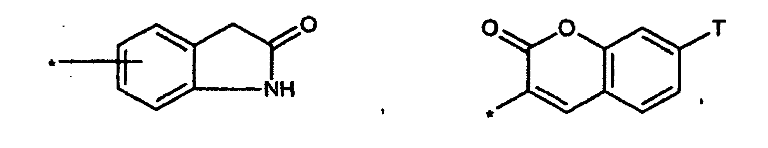 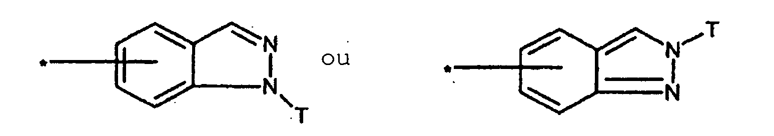 dans lequel T représente un atome d'hydrogène, un groupe alkyle en C₁-C₆ ou alcoxy en C₁-C₆,
R² et R³ représentent, indépendamment l'un de l'autre, un atome d'hydrogène ou un radical alkyle en C₁-C₆, cycloalkyle en C₃-C₆, cycloalcényle en C₃-C₆, aryle ou hétéroaryle, éventuellement une ou plusieurs fois substitué, de façon identique ou différente, par halogène, cyano, alkyle en C₁-C₆, phényle, hydroxy-alkyle(C₁-C₆), halogéno-alkyle(C₁-C₆) ou par le groupe -NR⁶R⁷, -OR⁵, alkyl(C₁-C₆)-OR⁵, -SR⁴, -SOR⁴ ou -SO₂R⁴, ou
R² et R³ forment ensemble avec l'atome d'azote un cycle en C₃-C₈ qui éventuellement peut contenir un autre atome d'azote, de soufre ou d'oxygène dans le cycle, ou peut contenir le groupe -N(R¹⁰), et qui peut être éventuellement une ou plusieurs fois substitué, de façon identique ou différente, par halogène, cyano, alkyle en C₁-C₆, halogéno-alkyle(C₁-C₆), aryle ou par le groupe -OR⁵, -SR⁴, -SOR⁴ ou -SO₂R⁴,
R⁴ représente un groupe hydroxy ou le groupe -NR²R³,
R⁵ représente un atome d'hydrogène, un groupe alkyle en C₁-C₁₂ ou représente un groupe alkyle en C₁-C₁₂ qui est interrompu une ou plusieurs fois par un atome d'oxygène, ou représente le groupe-(CH₂)₂NR²R³, -CH₂CN ou -CH₂CF₃,
R⁶ et R⁷ représentent, indépendamment l'un de l'autre, un atome d'hydrogène ou un groupe alkyle en C₁-C₆, ou
R⁶ et R⁷ forment ensemble un cycle à 5-7 chaînons, qui peut contenir un atome d'oxygène ou de soufre,
R⁸ représente un atome d'hydrogène ou un groupe alkyle en C₁-C₆, alcoxy en C₁-C₆, benzyle, aryle ou hétéroaryle, éventuellement une ou plusieurs fois substitué par halogène,
R⁹ représente un atome d'hydrogène, un groupe alkyle en C₁-C₆ ou trialkyl(C₁-C₆)-silyle, et
R¹² et R¹³ représentent, indépendamment l'un de l'autre, un atome d'hydrogène ou un groupe alkyle en C₁-C₆,
ainsi que leurs formes tautomères, isomères E ou Z, ou, lorsqu'un centre chiral est présent, également les racémates et leurs énantiomères et sels.

5. Composés de formule générale I selon les revendications 1 à 4, dans lesquels
A, B et D représentent chacun indépendamment un atome de carbone ou d'azote, au moins un atome d'azote étant contenu dans le cycle,
E représente le radical thiényle, pyridyle ou le groupe -COOR⁸, -CONR²R³ ou-C≡C-R⁹,
G représente le groupe -C-X,
L représente le groupe -C-X,
M représente le groupe -C-X,
Q représente un atome d'azote ou le groupe -C-X,
X représente un atome d'hydrogène ou d'halogène,
R¹ représente un radical phényle, thiophène, furanne, oxazole, thiazole, imidazole, pyrazole, pyridine, pyrimidine, triazine, quinoléine ou isoquinoléine, éventuellement une ou plusieurs fois substitué, de façon identique ou différente, par halogène, hydroxy, alkyloxy en C₁-C₆, aralkyloxy, alkyle en C₁-C₆, halogénoalkyle(C₁-C₆) ou par le groupe -SO₂R⁴, OR⁵, -R⁵ ou -PO(OR¹²)(OR¹³), ou est présent comme substituant sur le groupe 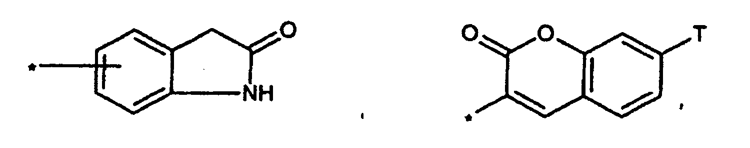 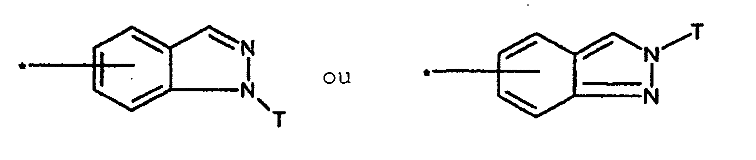 dans lequel T représente un atome d'hydrogène, un groupe alkyle en C₁-C₆ ou alcoxy en C₁-C₆,
R² et R³ représentent, indépendamment l'un de l'autre, un atome d'hydrogène ou un radical alkyle en C₁-C₆, cycloalkyle en C₃-C₆, phényle ou pyridyle, éventuellement une ou plusieurs fois substitué, de façon identique ou différente, par halogène, alkyle en C₁-C₆, phényle ou par le groupe -NR⁶R⁷, -OR⁵ ou alkyl(C₁-C₆)-OR⁵, ou
R² et R³ forment ensemble avec l'atome d'azote un cycle en C₃-C₈ qui éventuellement peut contenir un autre atome d'azote ou d'oxygène dans le cycle et qui peut être éventuellement une ou plusieurs fois substitué, de façon identique ou différente, par alkyle en C₁-C₆,
R⁴ représente un groupe hydroxy ou le groupe -NR²R³,
R⁵, R⁶ et R⁷ représentent, chacun indépendamment, un atome d'hydrogène ou un groupe alkyle en C₁-C₆,
ou
R⁶ et R⁷ forment ensemble un cycle à 5-7 chaînons, qui peut contenir un atome d'oxygène ou de soufre,
R⁸ représente un atome d'hydrogène, un groupe alkyle en C₁-C₆ ou benzyle, et
R⁹ représente un atome d'hydrogène, un groupe alkyle en C₁-C₆ ou trialkyl(C₁-C₆)-silyle, et
R¹² et R¹³ représentent, indépendamment l'un de l'autre, un atome d'hydrogène ou un groupe alkyle en C₁-C₆,
ainsi que leurs formes tautomères, isomères E ou Z, ou, lorsqu'un centre chiral est présent, également les racémates et leurs énantiomères et sels.

6. Médicament, contenant un composé selon les revendications 1 à 5.

7. Médicament selon la revendication 6, pour utilisation dans le psoriasis, le sarcome de Kaposi, la resténose, comme par exemple la resténose induite par stent, l'endométriose, la maladie de Crohn, la maladie de Hodgkin, la leucémie, l'arthrite, telle que la polyarthrite rhumatoïde, l'hémangiome, l'angiofibrome, des maladies des yeux telles que la rétinopathie diabétique, le glaucome néovasculaire, des maladies rénales telles que la glomérulonéphrite, la néphropathie diabétique, la néphrosclérose maligne, les syndromes microangiopathiques thrombotiques, des rejets de greffes et la glomérulopathie, des maladies fibreuses telles que la cirrhose, des maladies prolifératives des cellules mésangiales, l'artériosclérose, des lésions du tissu nerveux, l'inhibition de la ré-occlusion de vaisseaux après traitement par cathéter à ballonnet, la pose de prothèses vasculaires ou l'insertion de dispositifs mécaniques destinés à maintenir ouverts des vaisseaux, comme par exemple des stents, et comme immunosuppresseurs, et pour aider la cicatrisation sans marques, et dans les taches de sénescence et dans la dermatite de contact.

8. Médicament selon la revendication 6, pour utilisation en tant qu'inhibiteur de la VEGFR kinase 3 dans la lymphangiogenèse et dans les modifications hyperplasiques et dysplasiques du système vasculaire lymphatique.

9. Composés selon les revendications 1 à 5 et médicaments selon les revendications 6 à 8, avec des substances appropriées pour la formulation et des véhicules.

10. Utilisation des composés de formule I, selon les revendications 1 à 5, pour la fabrication d'un médicament, en tant qu'inhibiteurs de la tyrosine kinase KDR et FLT.

11. Utilisation des composés de formule générale I, selon les revendications 1 à 5, pour la fabrication d'un médicament sous forme d'une préparation pharmaceutique destinée à l'administration entérale, parentérale et orale.

12. Utilisation des composés selon les revendications 1 à 5, pour la fabrication d'un médicament destiné au traitement du psoriasis, du sarcome de Kaposi, de la resténose, comme par exemple la resténose induite par stent, de l'endométriose, de la maladie de Crohn, de la maladie de Hodgkin, de la leucémie, de l'arthrite, telle que la polyarthrite rhumatoïde, de l'hémangiome, de l'angiofibrome, de maladies des yeux telles que la rétinopathie diabétique, le glaucome néovasculaire, de maladies rénales telles que la glomérulonéphrite, la néphropathie diabétique, la néphrosclérose maligne, de syndromes microangiopathiques thrombotiques, de rejets de greffes et de la glomérulopathie, de maladies fibreuses telles que la cirrhose, de maladies prolifératives des cellules mésangiales, de l'artériosclérose, de lésions du tissu nerveux et à l'inhibition de la ré-occlusion de vaisseaux après traitement par cathéter à ballonnet, dans la pose de prothèses vasculaires ou l'insertion de dispositifs mécaniques destinés à maintenir ouverts des vaisseaux, comme par exemple des stents, et comme immunosuppresseurs, et pour aider la cicatrisation sans marques, et dans les taches de sénescence et dans la dermatite de contact.
